# FASCICULE DE BREVET EUROPEEN

(11) **EP 1 792 903 B1**
(45) Date de publication et mention de la délivrance du brevet: **14.10.2009**
(21) Numéro de dépôt: 06123701.2
(22) Date de dépôt: 08.11.2006
(51) Int. Cl.: C07D 471/04, A61K 8/49, A61Q 5/10

(54) **Composition pour la teinture des fibres kératiniques comprenant au moins un dérivé 3-amino pyrazolopyridine cationique**
Zusammensetzung zum Färben von Keratinhaltigen Fasern enthaltend mindenstens ein kationisches Derivat von 3-Amino-Prazolopyridin
Composition for the dyeing of keratin fibres comprising at least one cationic 3-amino-pyrazolopyridine derivative

(30) Priorité: 09.11.2005 FR 0553402
(43) Date de publication de la demande: 06.06.2007
(73) Titulaire: L'ORÉAL, 75008 Paris (FR)
(72) Inventeur: Fadli, Aziz, 77500, CHELLES (FR)
(74) Mandataire: Fevrier, Murielle Françoise E.

(56) Documents cités:
- WO-A-01/35917
- FR-A- 2 767 475
- FR-A- 2 822 689
- FR-A- 2 822 690
- FR-A- 2 822 691
- FR-A- 2 822 692

## Description

L'invention a pour objet une composition pour la teinture des fibres kératiniques, et en particulier des fibres kératiniques humaines telles que les cheveux, comprenant au moins un dérivé 3-amino pyrazolo-[1,5-a]-pyridine cationique particulier ou un de ses sels d'addition et un procédé mettant en oeuvre ce dérivé. Elle a de même pour objet les dérivés 3-amino pyrazolo-[1,5-a]-pyridines cationiques ou un de ses sels d'addition.

Il est connu de teindre les fibres kératiniques, et en particulier les fibres kératiniques humaines telles que les cheveux, avec des compositions tinctoriales comprenant des précurseurs de colorant d'oxydation, en particulier des ortho ou para-phénylènediamines, des ortho ou para-aminophénols, des composés hétérocycliques appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques tels que par exemple des dérivés de pyrazolo[1,5-b]-1,2,4-triazoles, des dérivés de pyrazolo[3,2-c]-1,2,4-triazoles, des dérivés de pyrazolo[1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

L'utilisation de base d'oxydation telle que les dérivés de para-phénylènediamine et de para-aminophénol permettent d'obtenir une gamme de couleurs assez large à pH basique sans toutefois atteindre des nuances de bonne chromaticité tout en conférant aux cheveux d'excellentes propriétés d'intensité de couleur, de variété de nuances, d'uniformité de la couleur et de la ténacité aux agents extérieurs.

L'utilisation de ces bases à pH neutre est de plus inefficace pour atteindre une gamme de nuances variées, en particuliers pour les nuances chaudes. -

Il a été déjà proposé dans la demande de brevet EP 1 233 743 des compositions de teintures contenant en tant que base d'oxydation des 3-amino pyrazolo-[1,5-a]-pyridine. Parmi les 3-amino pyrazolopyridines citées dans ce document sont cités des composés cationiques particuliers différents de ceux décrits dans la présente invention. Les compositions de teinture décrites dans ce document ne permettent pas d'atteindre de bonnes propriétés de chromaticité, de résistance aux agents extérieurs telle que le lavage et la lumière. De plus, l'étendue de la gamme des nuances est limitée.

Le but de la présente invention est de fournir de nouvelles bases d'oxydation pour la teinture des fibres kératiniques qui ne présentent pas les inconvénients des bases d'oxydation existantes. En particulier, le but de l'invention est de fournir de nouvelles bases d'oxydation qui permettent d'obtenir une coloration aux nuances variées, puissante, chromatique, esthétique, peu sélective et résistant bien aux diverses agressions que peuvent subir les cheveux tels que les shampooings, la lumière, la sueur et les déformations permanentes.

Ce but est atteint avec la présente invention qui a pour objet une composition de teinture des fibres kératiniques comprenant, dans un milieu approprié à la teinture, à titre de base de coloration d'oxydation, un dérivé 3-amino pyrazolo-[1,5-a]-pyridine de formule (I) suivante ainsi que ses sels et solvates: 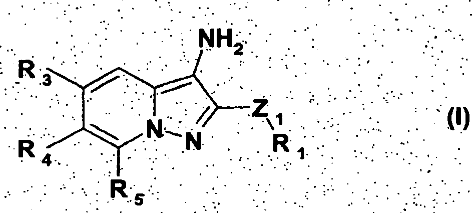 dans laquelle
- Z₁ représente radical -O-, un radical -NR₆- avec R₆ qui représente un atome d'hydrogène, un radical alkyle, ou R₆ forme avec R₁ un hétérocycle cationique et/ou substitué par un radical cationique
- R₁ représente
   - un hydrogène
   - un radical alkyle en C₁-C₁₀ substitué ou interrompu par un radical cationique,
- R₃, R₄ et R₆, identiques ou différents, représentent
   - un atome d'hydrogène,
   - un radical alkyle en C₁-C₄
   - un cycle carboné saturé, insaturé ou aromatique, éventuellement substitué.
   - R₄ et R₅, forment ensemble un cycle saturé ou insaturé à 5 ou 3 chaînons,
- X représente un anion ou groupe d'anions permettant d'assurer l'électronégativité du dérivé de formule (I),
avec la condition qu'au moins un des groupements Z₁, R₁, représente un radical cationique.

La présente invention permet en particulier d'obtenir une coloration des fibres kératiniques tenace, résistante à la lumière et au lavage.

Un autre objet de l'invention est un procédé de teinture des fibres kératiniques mettant en oeuvre la composition de la présente invention, ainsi que l'utilisation de cette composition pour la teinture des fibres kératiniques.

L'invention a aussi pour objet de nouveaux dérivés pyrazolopyridines ainsi que les dérivés nitro ou nitroso correspondants.

Dans le cadre de l'invention, on entend par radical alkyle, les radicaux alkyle linéaires ou ramifiés, pouvant être substitués ou non substitués. Ils peuvent être substitués par n'importe quel substituant classique dans le domaine de la coloration et qui ne changent pas les propriétés de base d'oxydation des composés de formule (I).

A titre d'exemples de substituants de ces radicaux alkyles, on peut citer les substituants halogéno ; hydroxy ; alcoxy ;amino ; thio, alkylthio, alkylamino en C₁-C₆ ; dialkylamino en C₁-C₆ dans lequel les radicaux alkyles peuvent former avec l'atome d'azote auquel ils sont rattachés un hétérocyclique de 5 à 8 chaînons, saturé, insaturé, aromatique ou non, cationique ou non, et pouvant renfermer un ou plusieurs hétéroatomes ou groupements choisis parmi N, O, S, SO₂, CO, l'hétérocycle pouvant être substitué ; alkyl(C₁-C₆)carbonyle ; -O-CO-R ; -CO-O-R ; NR-CO-R' ou -CO-NRR' dans lesquels R et R' sont tels que définis précédemment; un radical ammonium quaternaire tel que défini précédemment.

Ceci s'applique aux radicaux alkyle présents dans n'importe lequel des radicaux définis dans la formule (I), notamment pour les radicaux alcoxy (alkyl-O-) ou alkylthio.

A titre d'exemples de substituants des cycles ou hétérocycles, on peut citer les radicaux alkyles, les radicaux alkyles substitués, les radicaux hydroxy, alcoxy, amino, alkylamino, dialkylamino.

Par radical cationique présent dans le composé de formule (I), on entend dans le cadre de l'invention tout radical linéaire ou ramifié comportant un ammonium quaternaire, cet ammonium quaternaire étant du type -N⁺R₁₇R₁₈R₁₉, R₁₇, R₁₈, R₁₉ identiques ou différents représentant un radical alkyle en C₁-C₆ pouvant être substitué par un hydroxy.

Par cycle ou hétérocycle cationique, on entend un cycle contenant un ammonium quaternaire.

A titre d'exemple de radicaux du type -N⁺R₁₇R₁₈R₁₉ , on peut citer les radicaux triméthylammonium, triéthylammonium, diméthyl-éthyl ammonium, diéthylméthylammonium, diisopropylméthylammonium, diéthyl-propyl ammonium, hydroxyéthyl diéthyl ammonium, di beta hydroxyethylmethylammonium, tri beta hydroxyethylammonium.

A titre d'exemple d'hétérocycle cationique, on peut citer les imidazoliums, les pyridiniums, les pipéraziniums, les pyrrolidiniums, les morpholiniums , les pyrimidiniums, les thiazoliums, les benzimidazoliums, les benzothiazoliums, les oxazoliums, les benzotriazoliums, les pyrazoliums, les triazoliums, les benzoxazoliums.

Les composés de formule (I) peuvent être éventuellement salifiés par des acides minéraux forts tels que par exemple HCl, HBr, Hl, H₂SO₄, H₃PO₄, ou des acides organiques tels que, par exemple, l'acide acétique, lactique, tartrique, citrique ou succinique, benzènesulfonique, para-toluènesulfonique, formique, méthanesulfonique.

Ils peuvent aussi être sous forme de solvates par exemple un hydrate ou un solvate d'alcool linéaire ou ramifié tel que l'éthanol ou l'isopropanol.

Dans le cadre de l'invention, on entend par dérivé de formule (I) toutes formes mésormères ou isomères.

A titre d'exemples de dérivés de formule (I), on peut citer les composés suivants dans lesquels X⁻ est tel que défini précédemment :

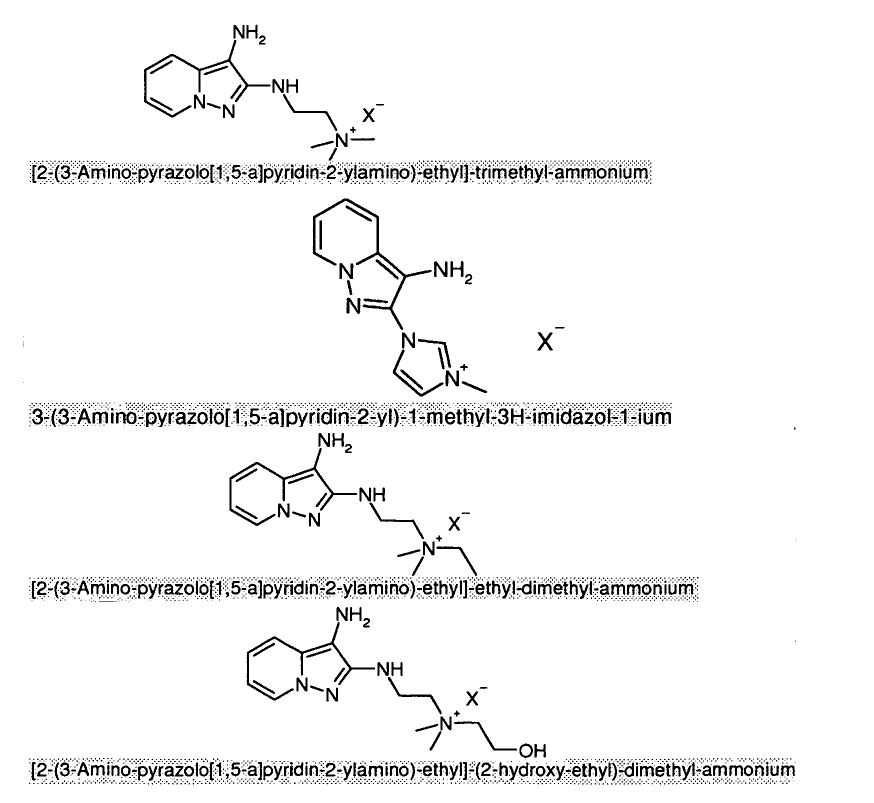

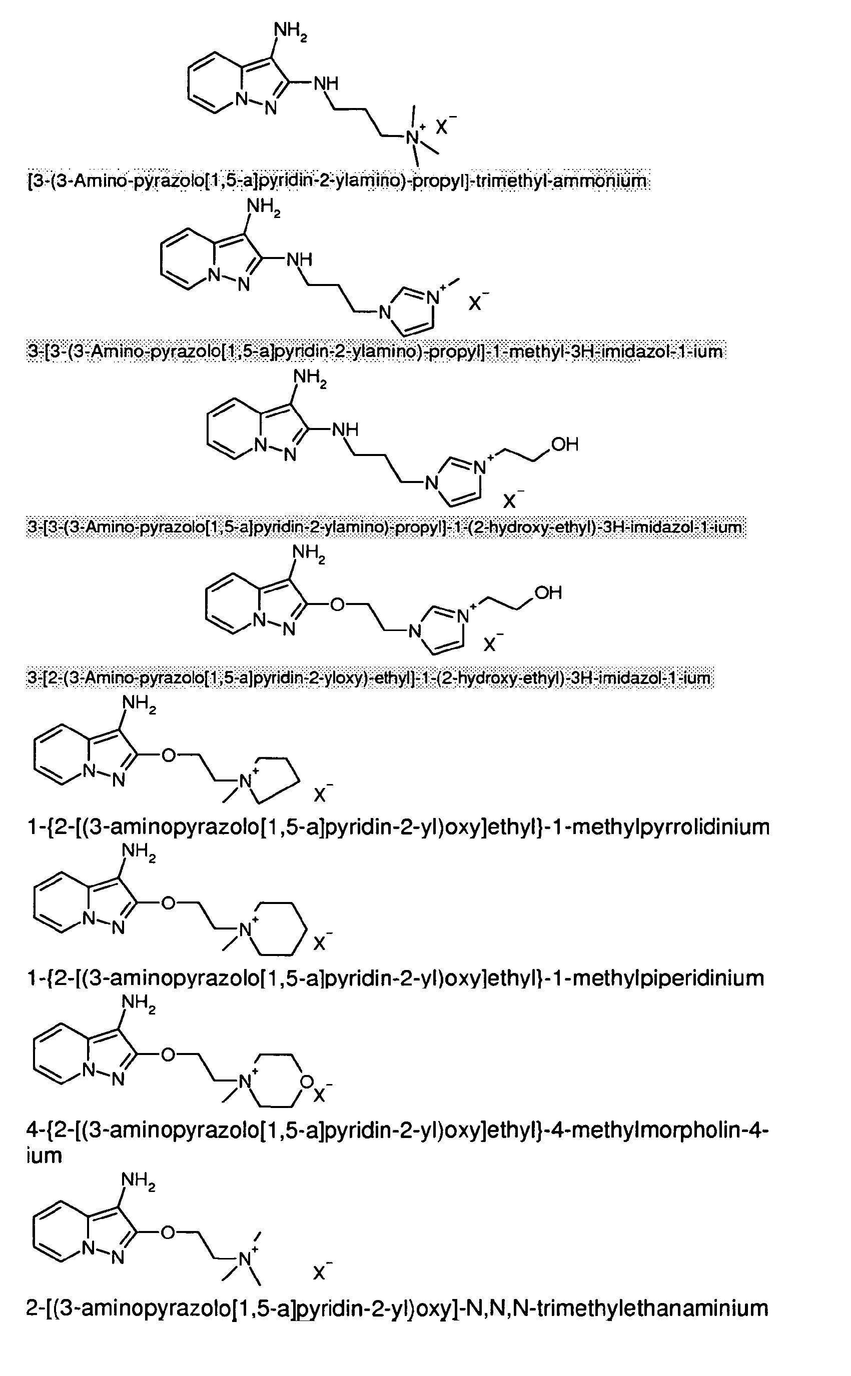

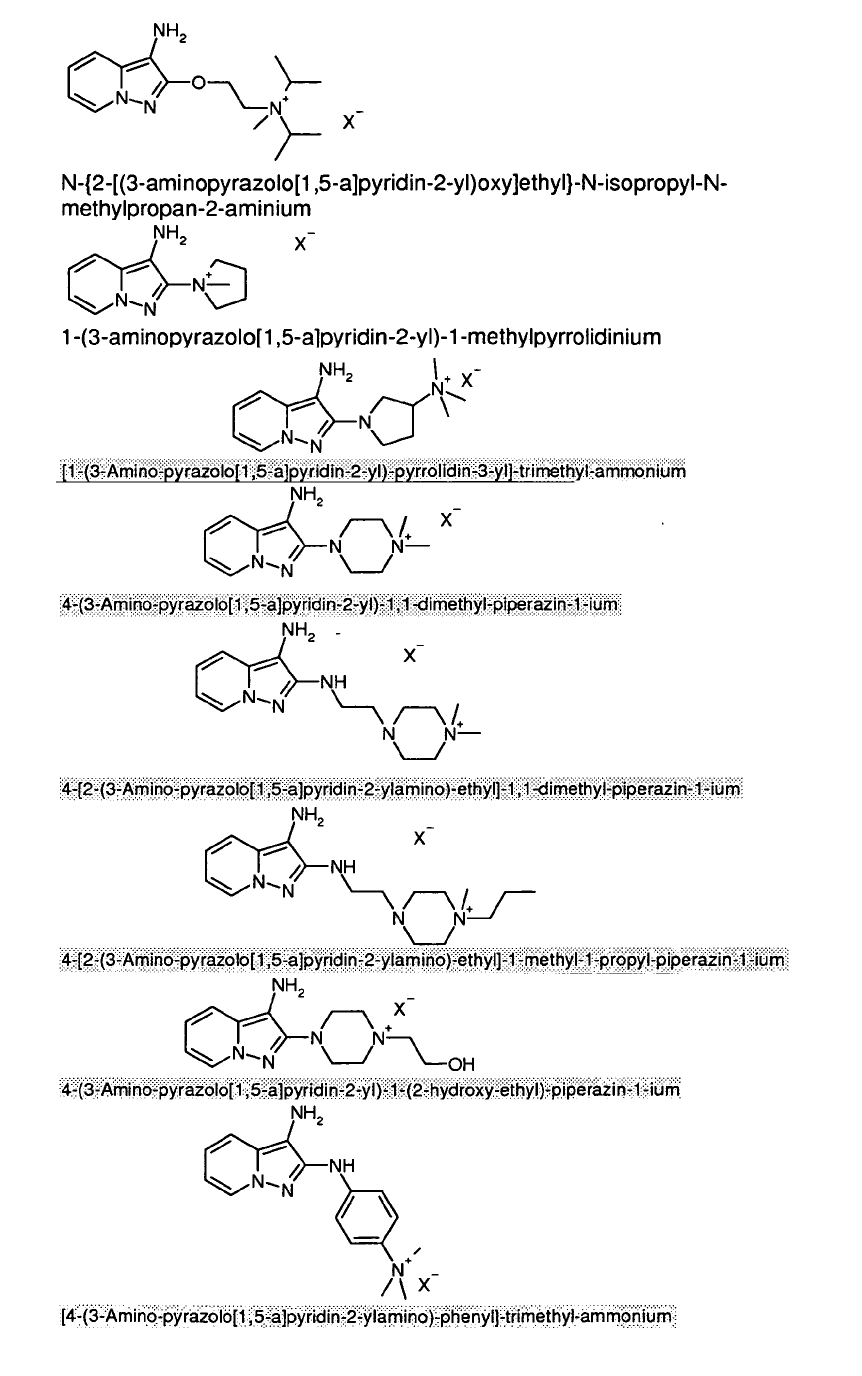

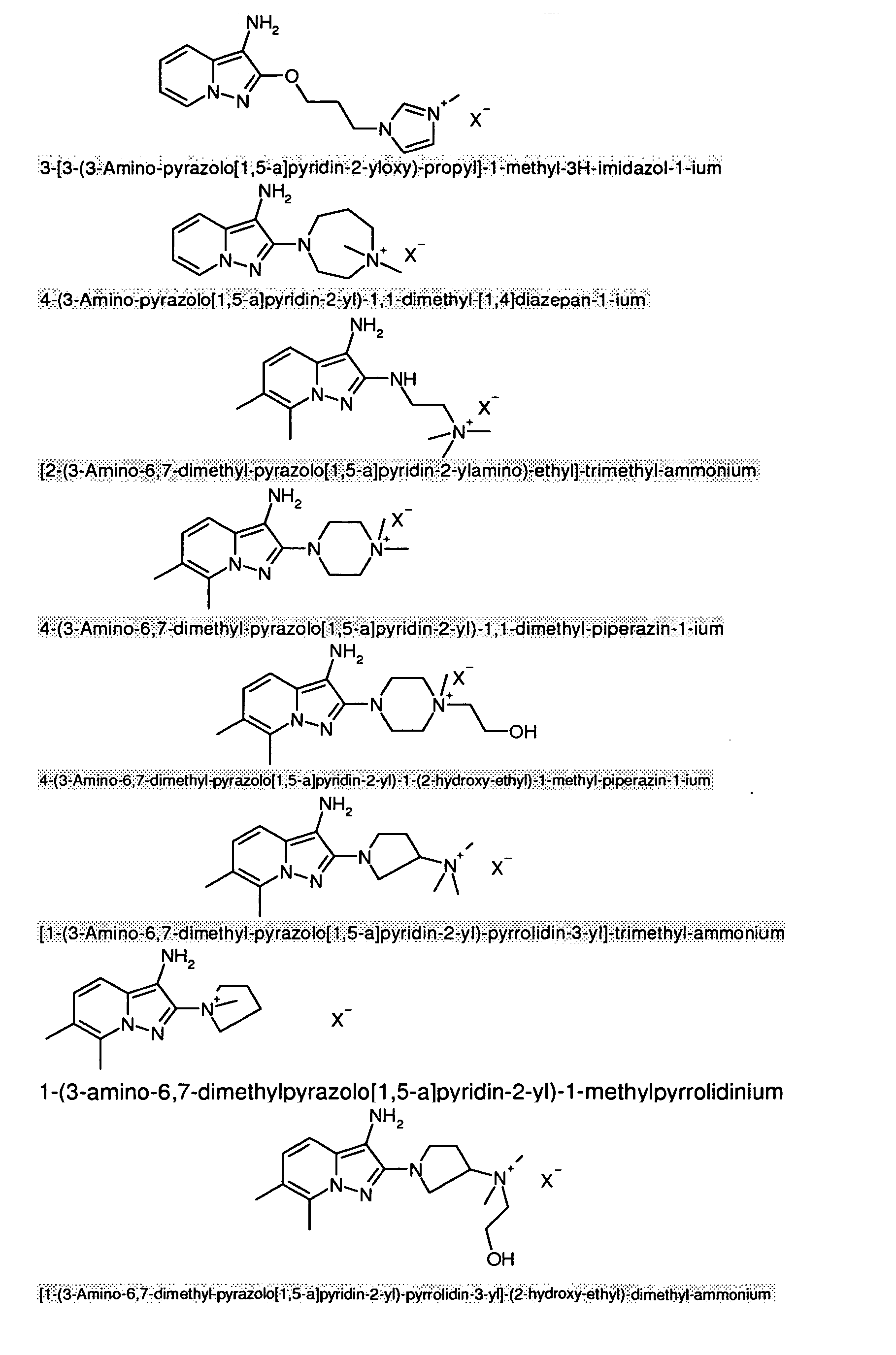

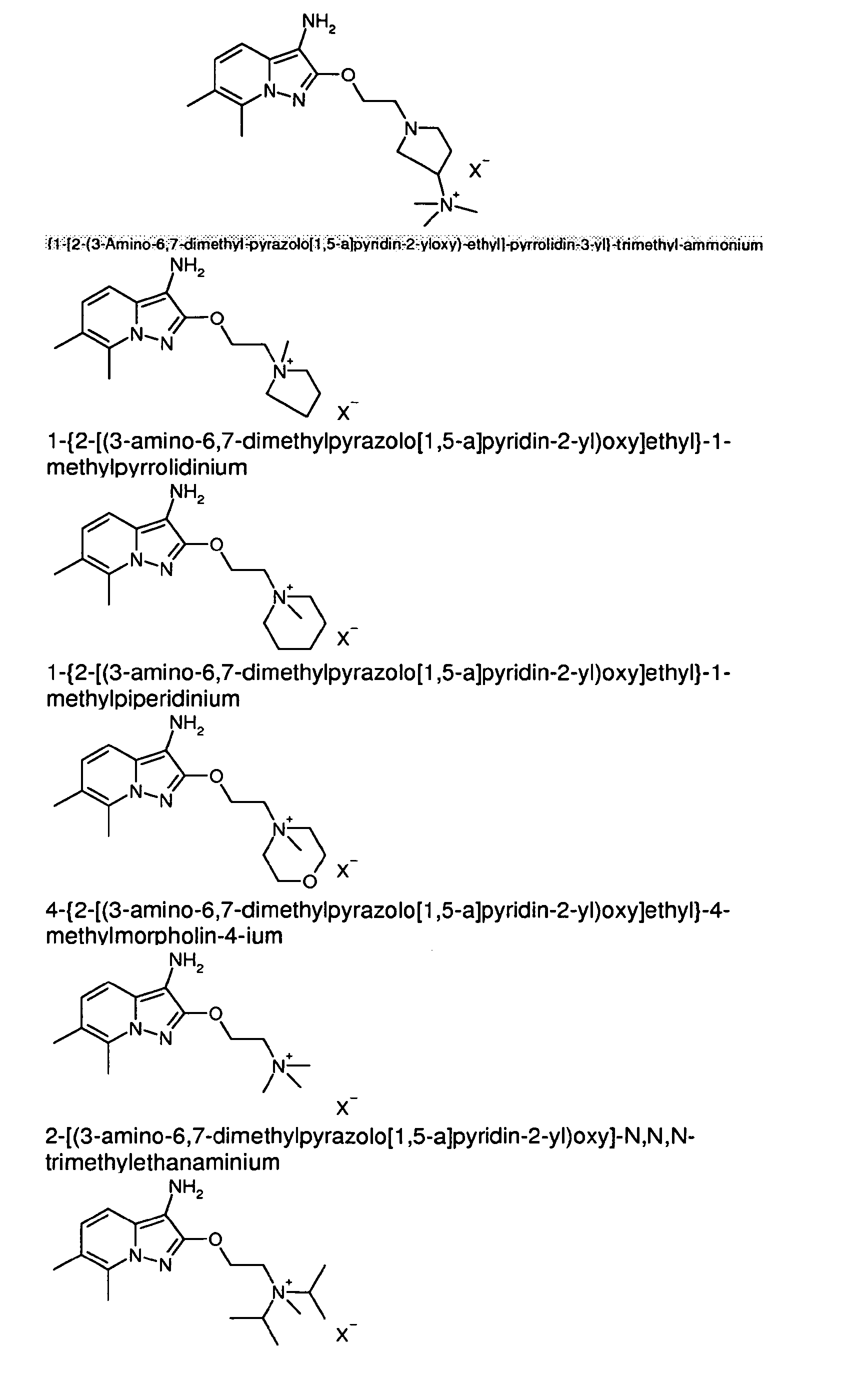

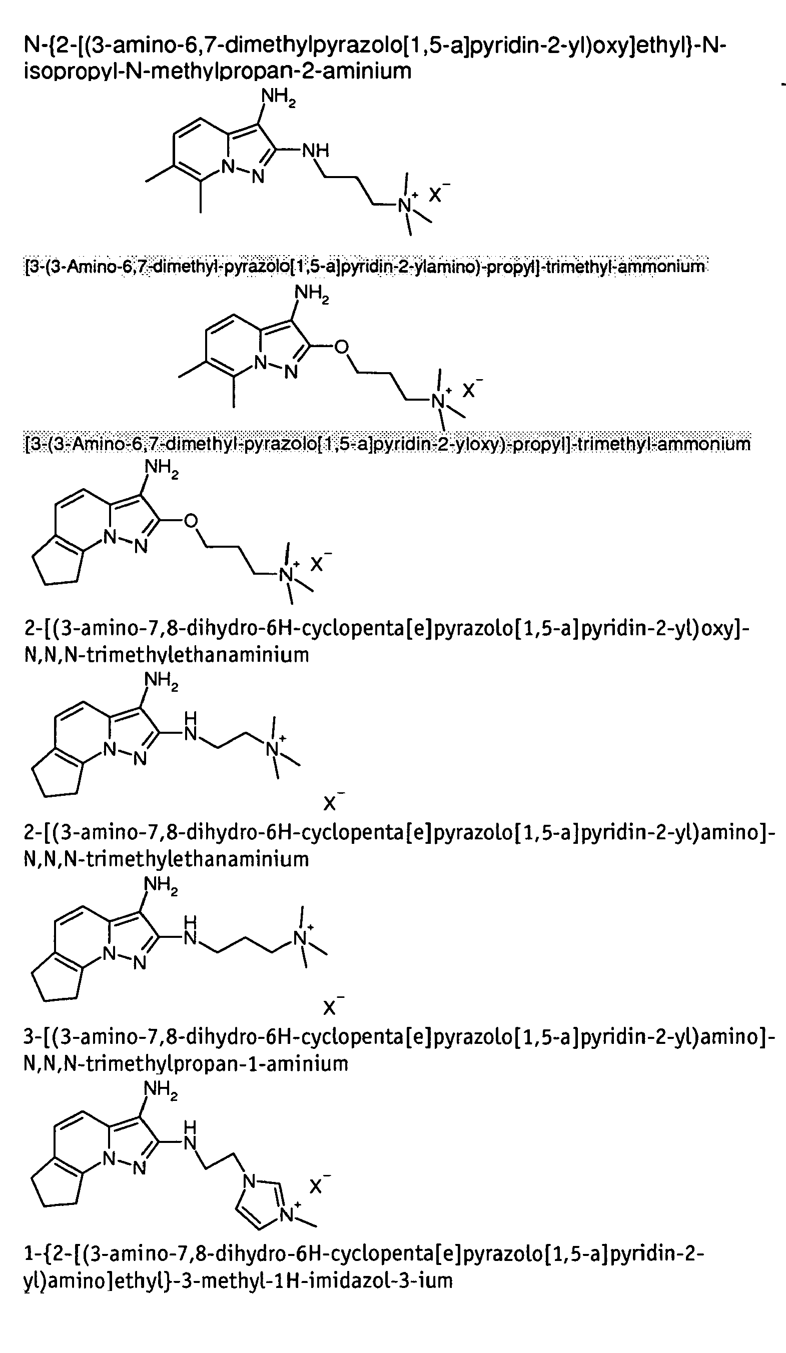

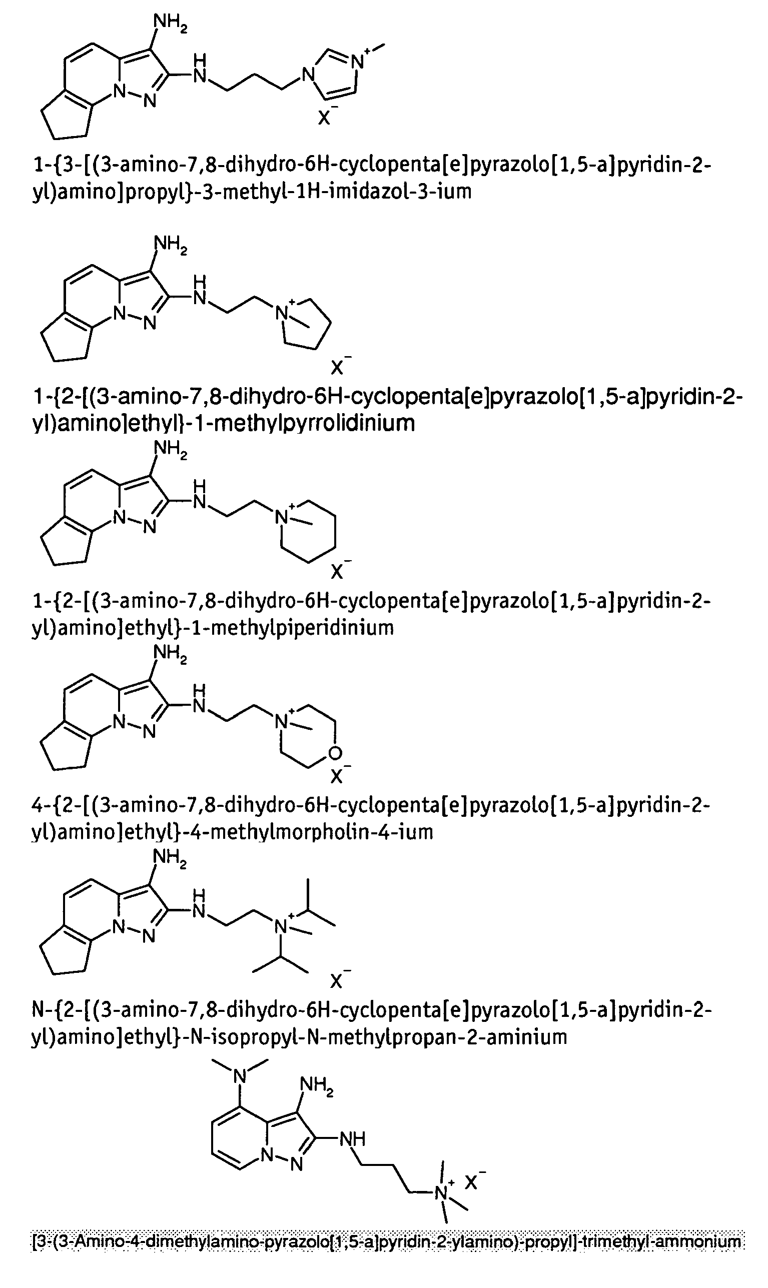

Lorsque R₆ forme avec R_{1,} un hétérocycle, cet hétérocycle est de préférence un hétérocycle cationique ou un hétérocycle substitué par un radical cationique. A titre d'exemple, on peut citer les imidazoles substitués par un radical ammonium quaternaire ou les imidazoliums, les pipérazines substitués par un radical ammonium quaternaire ou les pipéraziniums, les pyrrolidines substitués par un radical ammonium quaternaire ou les pyrrolidiniums, les diazépanes substitués par un radical ammonium quaternaire ou les diazépaniums.

Selon un mode de réalisation différent, R₁ représente un atome d'hydrogène, un radical alkyle pouvant être interrompu ou substitué par un radical cationique, tel qu'alkylammonium, hydroxyalkylammonium, imidazolium, piperazinium, pyrrolidinium, diazépanium, imidazolyle substitué par un radical cationique, piperazinyle substitué par un radical cationique, pyrrolidinyle substitué par un radical cationique, pyridinyle substitué par un radical cationique.

Les radicaux R₃, R₄ et R₅ indépendamment représentent un atome d'hydrogène, un radical alkyle en C₁-C₄. A titre d'exemple, on peut citer les radicaux méthyle, éthyle, hydroxy éthyle, amino éthyle, propyle, butyle. Selon un mode de réalisation particulier, R₃, R₄ et R₅ représentent indépendamment un atome d'hydrogène, un radical alkyle en C₁-C₄.

Selon un mode de réalisation particulier, R₄ et R₅ forment ensembles un cycle saturé ou insaturé à 5 ou 8 chaînons, notamment un cyclopentane ou cyclohexane, éventuellement substitué.

Selon un mode de réalisation particulier de cette formule, Z₁ représente - NH- ou NR₆ avec R₆ qui forme avec R₁ un hétérocycle cationique ou substitué par un radical cationique.

A titre de composés préférés, on peut citer
2-[(3-aminopyrazolo[1,5-a]pyridin-2-yl)amino]-N,N,N-trimethylethanaminium chlorure hydrochlorure
3-[(3-aminopyrazolo[1,5-a]pyridin-2-yl)amino]-N,N,N-trimethylpropan-1-aminium chlorure hydrochlorure
2-[(3-aminopyrazolo[1,5-a]pyridin-2-yl)amino]-N,N-diethyl-N-methylethanaminium chlorure hydrochlorure
N-{2-[(3-aminopyrazolo[1,5-a]pyridin-2-yl)amino]ethyl}-N-isopropyl-N-methylpropan-2-aminium chlorure hydrochlorure
1-{2-[(3-aminopyrazolo[1,5-a]pyridin-2-yl)amino]ethyl}-3-methyl-1H-imidazol-3-ium chlorure hydrochlorure
1-{2-[(3-aminopyrazolo[1,5-a]pyridin-2-yl)amino]ethyl}3-metyl-1H-imidazol-3-ium chlorure hydrochlorure
1-{2-[(3-aminopyrazolo[1,5-a]pyridin-2-yl)amino]ethyl}-1-methylpyrrolidinium chlorure hydrochlorure
1-{2-[(3-aminopyrazolo[1,5-a]pyridin-2-yl)amino}ethyl}-1-methylpiperidinium chlorure hydrochlorure
4-{2-[(3-aminopyrazolo[1,5-a]pyridin-2-yl)amino]ethyl}-4-methylmorpholin-4-ium chlorure hydrochlorure
1-{2-[(3-aminopyrazolo[1,5-a]pyridin-2-yl)amino]ethyl}-N,N,N-trimethylpyrrolidin-3-aminium chlorure hydrochlorure
2-[(3-aminopyrazolo[1,5-a]pyridin-2-yl)oxy]-N,N,N-trimethylethanaminium chlorure hydrochlorure
2-{2-[(3-aminopyrazolo[1,5-a]pyridin-2-yl)amino]ethyl}-1-methylpyridinium chlorure hydrochlorure
3-{2-[(3-aminopyrazolo[1,5-a]pyridin-2-yl)amino]ethyl}-1-methylpyridinium chlorure hydrochlorure
4-{2-[(3-aminopyrazolo[1,5-a]pyridin-2-yl)amino]ethyl}-1-methylpyridinium chlorure hydrochlorure
1-{2-[(3-aminopyrazolo[1,5-a]pyridin-2-yl)oxy]ethyl}-1-methylpyrrolidinium chlorure hydrochlorure
1-{2-[(3-aminopyrazolo[1,5-a]pyridin-2-yl)oxy]ethyl}-1-methylpiperidinium chlorure hydrochlorure
N-{2-[(3-aminopyrazolo[1,5-a]pyridin-2-yl)oxy]ethyl}-N-isopropyl-N-methylpropan-2-aminium chlorure hydrochlorure
4-{2-[(3-aminopyrazolo[1,5-a]pyridin-2-yl)oxy]ethyl}-4-methylmorpholin-4-ium chlorure hydrochlorure
2-{2-[(3-aminopyrazolo[1,5-a]pyridin-2-yl)oxy]ethyl}-1-methylpyridinium chlorure hydrochlorure
3-{2-[(3-aminopyrazolo[1,5-a]pyridin-2-yl)oxy]ethyl}-1-methylpyridinium chlorure hydrochlorure
4-{2-[(3-aminopyrazolo[1,5-a]pyridin-2-yl)oxy]ethyl}-1-methylpyridinium chlorure hydrochlorure
2-[(3-amino-6,7-dimethylpyrazolo[1,5-a]pyridin-2-yl)amino]-N,N,N-trimethylethanaminium chlorure hydrochlorure
N-{2-[(3-amino-6,7-dimethylpyrazolo[1,5-a]pyridin-2-yl)amino]ethyl}-N-isopropyl-N-methylpropan-2-aminium chlorure hydrochlorure
1-{2-[(3-amino-6,7-dimethylpyrazolo[1,5-a]pyridin-2-yl)amino]ethyl}-1-methylpyrrolidinium chlorure hydrochlorure
1-{2-[(3-amino-6,7-dimethylpyrazolo[1,5-a]pyridin-2-yl)amino]ethyl}-1-methylpiperidinium chlorure hydrochlorure
4-{2-[(3-amino-6,7-dimethylpyrazolo[1,5-a]pyridin-2-yl)amino]ethyl}-4-methylmorpholin-4-ium chlorure hydrochlorure
2-{2-[(3-amino-6,7-dimethylpyrazolo[1,5-a]pyridin-2-yl)amino]ethyl}-1-methylpyridinium chlorure hydrochlorure
3-{2-[(3-amino-6,7-dimethylpyrazolo[1,5-a]pyridin-2-yl)amino]ethyl}-1-methylpyridinium chlorure hydrochlorure
4-{2-[(3-amino-6,7-dimethylpyrazolo[1,5-a]pyridin-2-yl)amino]ethyl}-1-methylpyridinium chlorure hydrochlorure
2-[(3-amino-6,7-dimethylpyrazolo[1,5-a]pyridin-2-yl)oxy]-N,N,N-trimethylethanaminium chlorure hydrochlorure
1-{2-[(3-amino-6,7-dimethylpyrazolo[1,5-a]pyridin-2-yl)oxy]ethyl}-1-methylpyrrolidinium chlorure hydrochlorure
1-{2-[(3-amino-6,7-dimethylpyrazolo[1,5-a]pyridin-2-yl)oxy]ethyl}-1-methylpiperidinium chlorure hydrochlorure
4-{2-[(3-amino-6,7-dimethylpyrazolo[1,5-a]pyridin-2-yl)oxy]ethyl}-4-methylmorpholin-4-ium chlorure hydrochlorure
2-{2-[(3-amino-6,7-dimethylpyrazolo[1,5-a]pyridin-2-yl)oxy]ethyl}-1-methylpyridinium chlorure hydrochlorure
3-{2-[(3-amino-6,7-dimethylpyrazolo[1,5-a]pyridin-2-yl)oxy]ethyl}-1-methylpyridinium chlorure hydrochlorure
4-{2-[(3-amino-6,7-dimethylpyrazolo[1,5-a]pyridin-2-yl)oxy]ethyl}-1-methylpyridinium chlorure hydrochlorure
2-[(3-amino-7,8-dihydro-6H-cyclopenta[e]pyrazolo[1,5-a]pyridin-2-yl)amino]-N,N,N-trimethylethanaminium chlorure hydrochlorure
N-{2-[(3-amino-7,8-dihydro-6H-cyclopenta[e]pyrazolo[1,5-a]pyridin-2-yl)amino]ethyl}-N-isopropyl-N-methylpropan-2-aminium chlorure hydrochlorure
1-{2-[(3-amino-7,8-dihydro-6H-cyclopenta[e]pyrazolo[1,5-a]pyridin-2-yl)amino]ethyl}-1-methylpyrrolidinium chlorure hydrochlorure
1-{2-[(3-amino-7,8-dihydro-6H-cyclopenta[e]pyrazolo[1,5-a]pyridin-2-yl)amino]ethyl}-1-methylpiperidinium chlorure hydrochlorure
4-{2-[(3-amino-7,8-dihydro-6H-cyclopenta[e]pyrazolo[1,5-a]pyridin-2-yl)amino]ethyl}-4-methylmorpholin-4-ium chlorure hydrochlorure
2-{2-[(3-amino-7,8-dihydro-6H-cyclopenta[e]pyrazolo[1,5-a]pyridin-2-yl)amino]ethyl}-1-methylpyridinium chlorure hydrochlorure
3-{2-[(3-amino-7,8-dihydro-6H-cyclopenta[e]pyrazolo[1,5-a]pyridin-2-yl)amino]ethyl}-1-methylpyridinium chlorure hydrochlorure
4-{2-[(3-amino-7,8-dihydro-6H-cyclopenta[e]pyrazolo[1,5-a]pyridin-2-yl)amino]ethyl}-1-methylpyridinium chlorure hydrochlorure
2-[(3-amino-7,8-dihydro-6H-cyclopenta[e]pyrazolo[1,5-a]pyridin-2-yl)oxy]-N,N,N-trimethylethanaminium chlorure hydrochlorure
N-{2-[(3-amino-7,8-dihydro-6H-cyclopenta[e]pyrazolo[1,5-a]pyridin-2-yl)oxy]ethyl}-N-isopropyl-N-methylpropan-2-aminium chlorure hydrochlorure
3-{2-[(3-amino-7,8-dihydro-6H-cyclopenta[e]pyrazolo[1,5-a]pyridin-2-yl)oxy]ethyl}-1-methylpyridinium chlorure hydrochlorure
2-{2-[(3-amino-7,8-dihydro-6H-cyclopenta[e]pyrazolo[1,5-a]pyridin-2-yl)oxy]ethyl}-1-methylpyridinium chlorure hydrochlorure
4-{2-[(3-amino-7,8-dihydro-6H-cyclopenta[e]pyrazolo[1,5-a]pyridin-2-yl)oxy]ethyl}-1-methylpyridinium chlorure hydrochlorure
1-{2-[(3-amino-7,8-dihydro-6H-cyclopenta[e]pyrazolo[1,5-a]pyridin-2-yl)oxy]ethyl}-1-methylpyrrolidinium chlorure hydrochlorure
1-{2-[(3-amino-7,8-dihydro-6H-cyclopenta[e]pyrazolo[1,5-a]pyridin-2-yl)oxy]ethyl}-1-methylpiperidinium chlorure hydrochlorure
4-{2-[(3-amino-7,8-dihydro-6H-cyclopenta[e]pyrazolo[1,5-a]pyridin-2-yl)oxy]ethyl}-4-methylmorpholin-4-ium chlorure hydrochlorure
2-{2-[(3-amino-7,8-dihydro-6H-cyclopenta[e]pyrazolo[1,5-a]pyridin-2-yl)oxy]ethyl}-1-methylpyridinium chlorure hydrochlorure
3-{2-[(3-amino-7,8-dihydro-6H-cyclopenta[e]pyrazolo[1,5-a]pyridin-2-yl)oxy]ethyl}-1-methylpyridinium chlorure hydrochlorure
4-{2-[(3-amino-7,8-dihydro-6H-cyclopenta[e]pyrazolo[1,5-a]pyridin-2-yl)oxy]ethyl}-1-methylpyridinium chlorure hydrochlorure

Selon un mode de réalisation encore plus particulier, le dérivé de formule (I) est tel que Z₁ est égal à NH et R₁ est un radical cationique ou Z₁ est égal à _-O- et R₁ est un radical cationique. La ou les bases d'oxydation de l'invention sont en général présentes chacune en quantité comprise entre 0,001 à 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation autres que celles décrites précédemment. A titre d'exemple, ces bases d'oxydation additionnelles sont choisies parmi les para-phénylènediamines, les bis-phénylalkylènediamines, les para-aminophénols, les bis-para-aminophénols, les ortho-aminophénols, les ortho-phénylènediamines, les bases hétérocycliques différentes des dérivés de formule (I) tels que définis précédemment et leurs sels d'addition.

Parmi les para-phénylènediamines, on peut citer à titre d'exemple, la para-phénylènediamine, la para-toluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluylènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques additionnelles différentes des bases d'oxydation de formule (I) et utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; le 3-amino-pyrazolo[1,5-a]pyridine-5-ol ; le 3-amino-pyrazolo[1,5-a]pyridine-4-ol ; le 3-amino-pyrazolo[1,5-a]pyridine-6-ol ; le 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation additionnelles présentes dans la composition de l'invention sont en général présentes chacune en quantité comprise entre 0,001 à 10 % en poids environ du poids total de la composition tinctoriale, de préférence entre 0,005 et 6 %.

D'une manière générale, les sels d'addition de ces bases d'oxydation additionnelles et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition tinctoriale.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 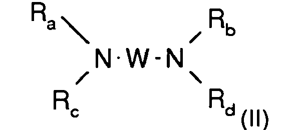 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

La présente invention a également pour objet l'utilisation pour la teinture d'oxydation des fibres kératiniques, et en particulier des fibres kératiniques humaines telles que les cheveux, d'un dérivé pyrazolopyridine de formule (I) ou d'un de ses sels d'addition tel que défini précédemment.

Les composés de formule (I) utiles dans la présente invention peuvent être obtenus à partir d'intermédiaires et de voies de synthèse décrites dans la littérature et notamment dans les références suivantes : J. Het. Chem., 2001, 38(3), 613-616 , Helvetica Chimica Acta, 1950, 33, 1183-1194, J.Org.Chem., 23, 2029 (1958), J.Am.Chem.Soc., 73, 3240 (1951), J.Am.Chem.Soc., 84, 590 (1962), Justus Liebig Ann.Chem., 686, 134 (1965), Tetrahedron. Lett., 31, 2859-2862 (1973), les brevets US4128425 et US 2841584 et les références citées.

Selon un mode de réalisation particulier, la synthèse des composés de formule (I) peut être réalisée selon le shéma suivant : 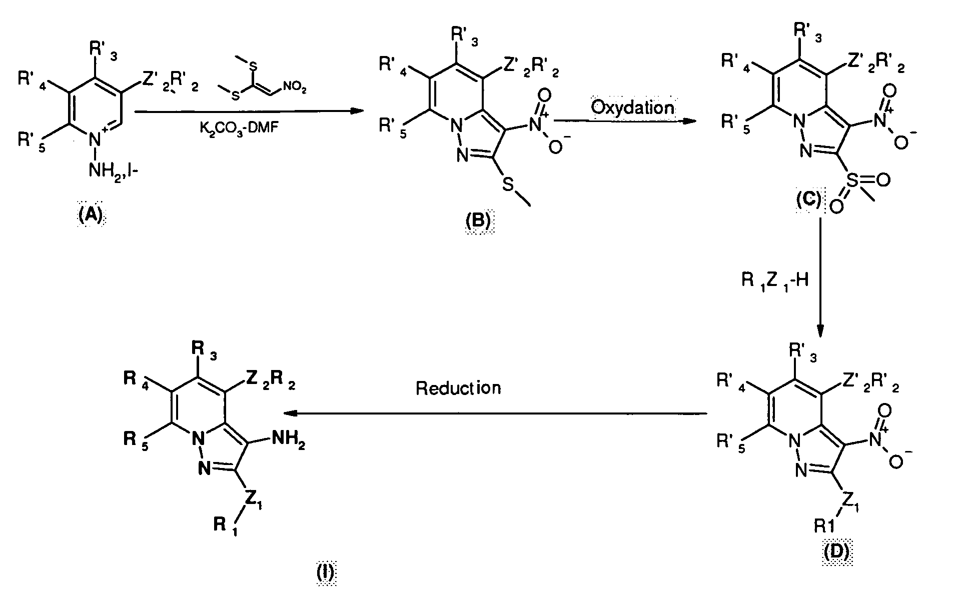 Z₁, R₁, Z'₂, R'₂, R'₃, R'₄ et R'₅ ont les mêmes significations que Z₂, R₂, R₃, R₄ et R₅ ou en sont des précurseurs permettant l'obtention des composés de la formule.

L'étape permettant d'obtenir le composé (B) à partir du composé (A) peut être réalisées selon les conditions décrites dans HETEROCYCLES, vol 6, N°4, 1977.

A titre d'exemple, on peut citer la synthèse suivante :

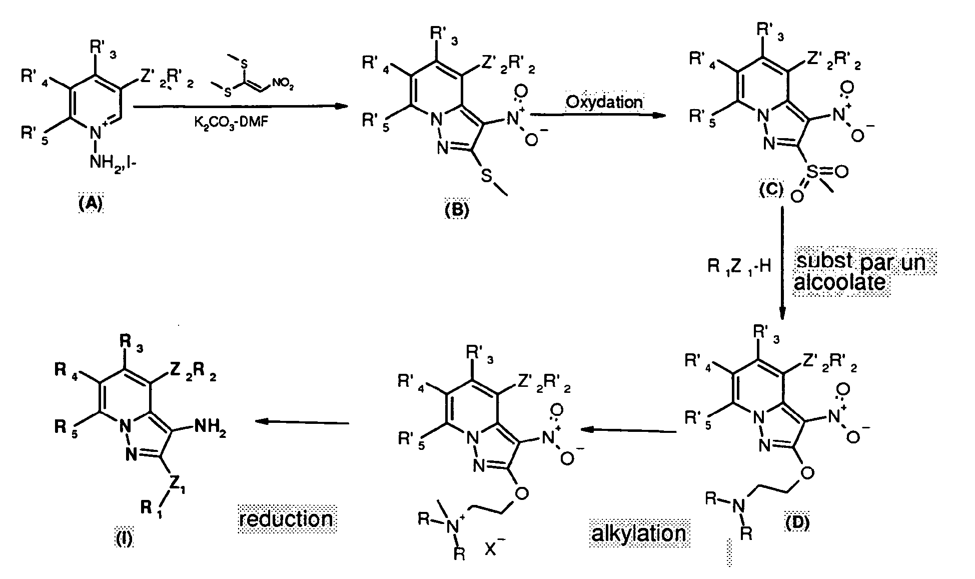

Une autre voie de synthèse peut être : 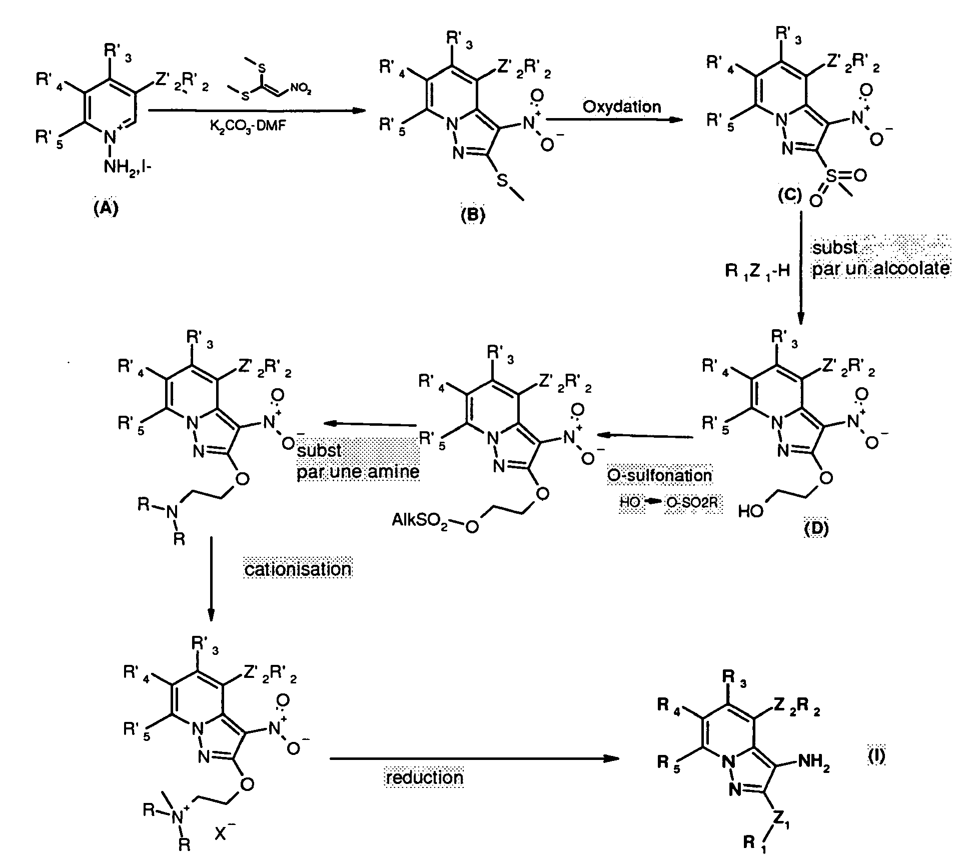 La dernière étape est toujours une réduction permettant l'obtention de I à partir des composés (D) est réalisée de manière classique, par exemple en effectuant une réaction d'hydrogénation par catalyse hétérogène en présence de Pd/C, Pd(II)/C, Ni/Ra, etc... ou encore en effectuant une réaction de réduction par un métal, par exemple par du zinc, fer, étain, etc. (voir Advanced Organic Chemistry, 3ème édition, J. March, 1985 , Willey Interscience et Reduction in organic Chemistry, M . Hudlicky, 1983 , Ellis Horwood Series Chemical Science).

L'invention a aussi pour objet les composés intermédiaires (A) et (B) tels que définis ci-dessus à l'exception du composé d'iodure de 1-amino-3-méthylpyridinium et 4-methyl-2-(méthylsulfanyl)-3-nitropyrazolo[1,5-a]pyridine

L'invention a de plus pour objet les composés intermédiaires de formule (C) et (D) tels que définis ci-dessus ainsi que les éventuels produits entre (D) et (I

Les exemples qui suivent servent à illustrer l'invention sans toutefois présenter un caractère limitatif.

### EXEMPLES

### Exemple 1

### Etape 1 : Synthèse du 2-méthylsulfanyl-3-nitro-pyrazolo[1,5-a]pyridine

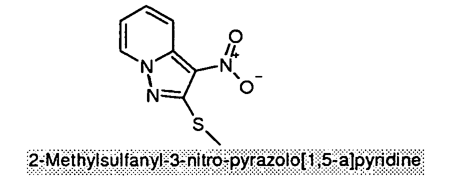

Dans un tricol de deux litres, équipé d'une agitation mécanique et d'une sonde de température interne et placé sous balayage d'azote, on prépare une solution de 111 g de 1-N-aminopyridinium (0,5 mol) dans le DMF (500ml).
Le carbonate de potassium (207,3, 3éq.) est alors ajouté en une fois, suivi du 1,1-bis(méthylthio)-2-nitroéthylène (165,2g, 2 éq.) également en une seule fois. Le milieu réactionnel prend en masse. 500 ml de DMF sont ajoutés afin de rendre le milieu réactionnel plus fluide.
Après 48 heures d'agitation à la température ambiante, le milieu réactionnel est versé sur 4 litres d'eau glacée. Le précipité qui se forme est filtré et lavé abondamment à l'eau (5 litres) puis séché à 80°C sous vide.
Le solide ainsi obtenu est débarrassé du 1,1-bis(méthylthio)-2-nitroéthylène en excès (30% molaire déterminé par 1 H-RMN) par un ré-empatage dans l'acétate d'éthyle. Après essorage et séchage, on obtient 72g d'un solide jaune beige correspondant au produit attendu.

RMN 1 H(300 MHz, DMSO-d6) :
8,98 (1H, d) ; 8,20 (1H, d), 7,89 (1H, m), 7,36 (1H, m), 2,63 (3H, s).

### Etape 2 : Synthèse du 2-méthanesulfonyl-3-nitro-pyrazolo[1,5-a]pyridine

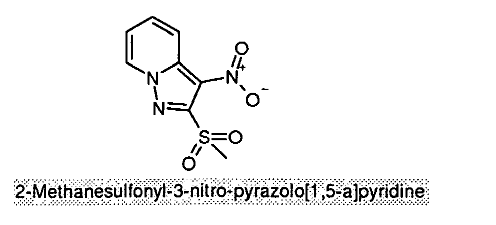

Dans un tricol de 4 litres, équipé d'une agitation mécanique et d'une sonde de température interne, sont chargés successivement 880g d'oxone (5 éq.), 2 litres d'eau et 60g de 2-méthylsulfonyl-3-nitro-pyrazolo[1,5-a]pyridine (0,287 éq.) obtenu précédemment. Le tout est agité à la température ambiante.
Pour compléter la réaction, de l'oxone (120g, 0,7 éq.) est additionnée et après 4 heures sous agitation à la température ambiante, la réaction est terminée.
Le solide formé est essoré et lavé abondamment à l'eau jusqu'à l'obtention d'un filtrat ne contenant plus de peroxyde. Il est ensuite placé sous vide à 40°C sur P₂O₅.
59g de produit attendu sont obtenus sous forme d'une poudre jaune-beige.

Les analyses RMN (¹H 400 MHz et ¹³C 100,61 MHz DMSO d₆) sont conformes à la structure attendue.

### Etape 3 : Synthèse du N-N-diméthyl-N'(3-nitro-pyrazolo[1,5-a]pyridin-2-yl)-éthane-1-2-diamine.

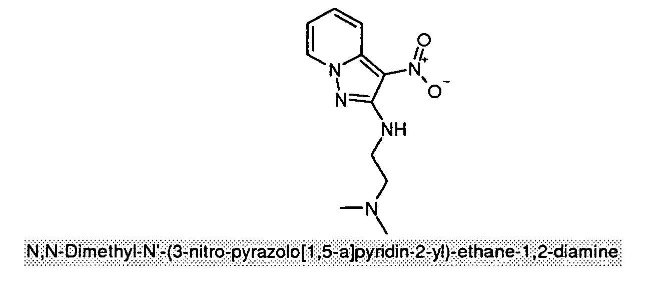

Dans un tricol de 500 ml, surmonté d'un réfrigérant à boules, d'un thermomètre et d'une agitation magnétique, on introduit 34,5g (0,143 moles) 2-méthanesulfanyl-3-nitro-pyrazol[1,5-a]pyridine et 71g (0,805mole) de diméthyl-éthylène diamine. Sous agitation, on porte à 80°C à l'aide d'un bain d'huile, pendant 5 heures en suivant par CCM (éluant : 98:2 Dichlorométhane / Méthanol).
On refroidit le milieu à la température ambiante, le composé jaune précipite dans le milieu, il est essoré sur fritté n°4 puis lavé à l'eau plusieurs fois. Après séchage à 40°C sous vide, en présence de P₂O₅, on récupère 35g de solide jaune correspondant au composé attendu.

Les analyses RMN (¹H 400 MHz et ¹³C 100,61 MHz DMSO d₆) sont conformes à la structure attendue.

La masse du composé attendu C₁₁H₁₁N₅O₂ est détectée en spectrométrie de masse.

### Etape 4: Synthèse du N,N,N-triméthyl-2-[(3-nitropyrazolo[1,5-a]pyridin-2-yl)amino]éthanaminium, méthosulfate

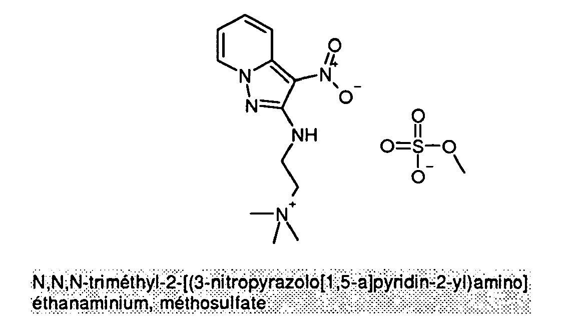

Dans un tricol de 500ml, équipé d'un réfrigérant à boules, d'un thermomètre et d'une ampoule de coulée de 50 mlml, sous agitation magnétique, et contenant 200 ml de THF, on introduit 35g ( 0,140 mole) de N-N-Dimethyl-N'(3-nitro-pyrazolo[1.5-a]pyridin-2-yl)-éthane-1,2-diamine. A l'aide d'un bain d'huile, on porte la température à 50°C et on coule goutte à goutte le diméthyl sulfate ; il se forme un précipité jaune. La réaction est suivie par CCM (éluant : dichlorométhane / méthanol 98 :2), jusqu'à la disparition du produit de départ. Le solide formé est essoré puis lavé plusieurs fois au THF. Apres séchage sous vide, en présence de P₂O₅ , on récupère 50,8g de poudre jaune.

Les analyses RMN (¹H 400 MHz et ¹³C 100,61 MHz DMSO d₆ sont conformes à la structure attendue.
Le cation attendu, C₁₂H₁₄N₅ O₂ +, est principalement détecté à m/z=264 en ES+.

### Etape 5 : Synthèse du [2-(3-amino-pyrazolo[1.5-a]pyridin-2-ylamino)éthyl]-triméthyl-ammonium.

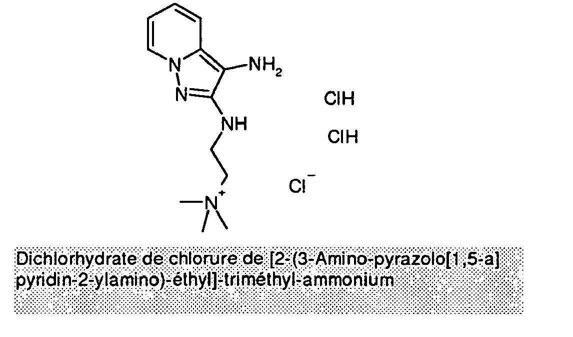

Dans un ballon de 4L, surmonté d'un réfrigérant, d'un thermomètre et d'une agitation mécanique, on introduit 120g de zinc et 2,2 litres d'éthanol.
A 40°C, on ajoute la solution de 17g de chlorure d'ammonium, 220ml d'eau et l'ensemble est porté au reflux. Le reflux est auto-entretenu par addition de 30g de N,N,N-triméthyl-2-[(3-nitropyrazolo[1,5-a]pyridin-2-yl)amino]éthanaminium, méthosulfate à l'aide d'une ampoule à solide.
Pour que la réaction soit totale, 5 ml d'acide acétique sont ajoutés.
Lorsque la réduction est terminée, le zinc est éliminé par filtration sur célite , lavé avec de l'éthanol et les jus réunis sont acidifiés par ajout disopropanol chlorhydrique préalablement refroidi par un bain froid de carboglace. On observe alors la précipitation d'un solide bleu.
Le solide est essoré sur fritté n°3. Après séchage sous vide, en présence de P₂O₅, on récupère 35,9g d'une poudre jaune correspondant au composé attendu.

Les ions quasi-moléculaires (MH)+, (MNa)+ et (M-H)- de la molécule attendue, C₉H₁₂N₄O, sont principalement détectés.

Les analyses RMN (¹H 400 MHz et ¹³C 100,61 MHz DMSO d₆) sont conformes à la structure attendue.

### EXEMPLE 2 :

### Etape 1 : Synthèse du 2-imidazol-1-yl-3-nitro-pyrazolo[1,5-a]pyridine.

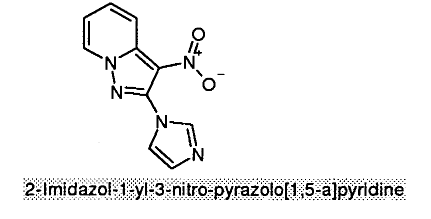

Dans un tricol de 100 ml, équipé d'une agitation magnétique, d'un thermomètre et d'un réfrigérant, sont chargés successivement 5g de 2-méthanesulfanyl-3-nitro-pyrazolo[1,5-a]pyridine, 20ml de N-méthylpyrrolidinone et 7g d'imidazole.
Ce milieu est chauffé à 100°C pendant 30 minutes puis après refroidissement, on précipite le 2-imidazol-1-yl-3-nitro-pyrazolo[1,5-a]pyridine dans 5 volumes d'eau. Après essorage et séchage sous vide en presence de penta-oxyde de phosphore, on récupère une masse de 4,23g de solide jaune.

Les analyses RMN (¹H 400 MHz et ¹³C 100,61 MHz DMSO d₆) sont conformes à la structure attendue.

La masse du composé attendu C₁₀H₇N₅O₂ est détectée en spectrométrie de masse.

### Etape 2 : Synthèse du Méthosulfate de 1-Méthyl-3-(3-nitro-pyrazolo[1,5-a]pyridin-2-yl)-3H-imidazol-1-ium..

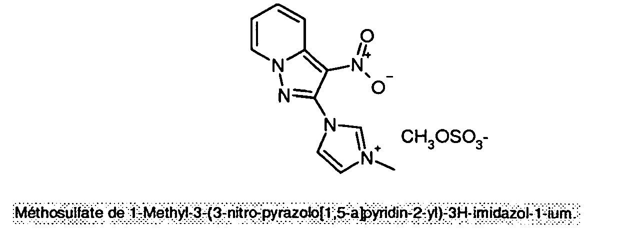

Dans un tricol de 100 ml, équipé d'une agitation magnétique, d'un thermomètre et d'un refrigérant et d'une ampoule de coulée, sont chargés successivement et portés à 60°C, 2,11g de 2-imidazol-1-yl-3-nitro-pyrazolo[1,5-a]pyridine et 55ml de THF.
Ce milieu est chauffé à une température de 60°C pour l'homogénéiser, puis 2ml de diméthylsulfate sont coulés en 1 heure.

Le solide formé est essoré sur fritté n°4 puis séché sous vide en présence de penta-oxyde de phosphore. Après séchage, on récupère une masse de 3g de poudre jaune.

Les analyses RMN (¹H 400 MHz et ¹³C 100,61 MHz DMSO d₆) sont conformes à la structure attendue.

Le cation attendu, C₁₁H₁₀N₅ O₂ +, est principalement détecté à m/z=244 en ES+.

### Etape 3 : Synthèse du dichlorhydrate de chlorure de 3-(3-Amino-pyrazolo[1,5-a]pyridin-2-yl)-1-méthyl-3H-imidazol-1-ium.

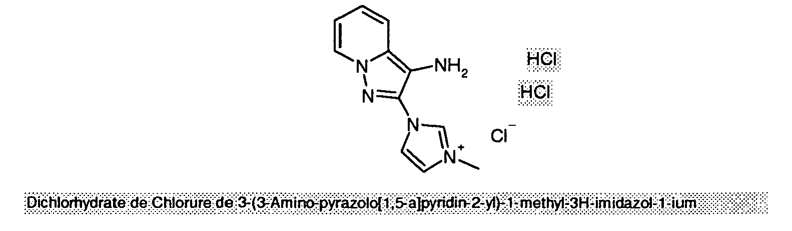

Dans un tricol de 500 ml, équipé d'une agitation magnétique, d'un thermomètre et d'un réfrigérant sont chargés successivement 200 ml d'eau, 20ml d'acide chlorhydrique concentré et le milieu est porté à 60°C. Le mélange de 5g de zinc et 3g de 2-imidazol-1-yl-3-nitro-pyrazolo[1,5-a]pyridine est introduit à l'aide d'une ampoule à solide en 2 heures.
En fin d'addition on maintient le reflux pendant 30 minutes et le zinc est éliminé par filtration sur un lit de célite. Les jus sont acidifiés avec de l'isopropanol chlorhydrique.

On concentre les jus au tiers et on précipite le solide à l'aide d'éther isopropylique.

Le solide formé est essoré sur fritté n°4 puis séché sous vide en présence de pentaoxyde de phosphore. Après séchage, on récupère une masse de 2,8g.

Le cation attendu, C₁₁H₁₂N₅ +, est principalement détecté à m/z=214 en ES+.
Les analyses RMN (¹H 400 MHz et ¹³C 100,61 MHz DMSO d₆ sont conformes à la structure attendue.

### EXEMPLE 3 : Synthèse du 3-nitro-N-(2-pipéridin-1-yléthyl)pyrazolo[1,5-a]pyridin-2-amine.

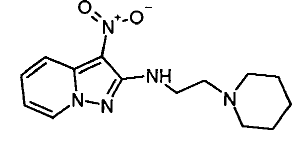

Dans un tricol de 250 ml, surmonté d'un réfrigérant à boules, d'un thermomètre et d'une agitation magnétique, on introduit 100 ml de NMP, 20g (0,0711 mole) 2-méthanesulfanyl-3-nitro-pyrazol[1,5-a]pyridine et 20,3 ml(0,142 mole) de 2-pipéridin-1-yléthanamine. Sous agitation, on porte à 70°C à l'aide d'un bain d'huile, pendant 4 heures en suivant par CCM (éluant : 95:5 Dichlorométhane / Méthanol).
On refroidit le milieu à la température ambiante et le milieu est versé sur un mélange de 400g de glace et d'eau. le composé beige qui a cristallisé est essoré sur fritté n°4 puis lavé à l'eau plusieurs fois. Après séchage à 35°C sous vide, en présence de P₂O₅ , on récupère 20,43g de solide jaune correspondant au composé attendu.

Les analyses RMN (¹H 400 MHz et ¹³C 100,61 MHz DMSO d₆) sont conformes à la structure attendue.
¹H NMR (DMSO-d6): 1,4 (m, 2H), 1,51 (m, 4H), 2,45 (m, 4H), 2.56 (t, 2H), 3,47 (q, 2H), 7,26 (m, 1 H), 7,32(t, 1 H), 7,76(m, 1 H), 8,04(m, 2H), 8,75(m, 1 H).

La masse du composé attendu C₁₄H₁₉N₅O₂ est détectée en spectrométrie de masse. Les ions quasi moléculaires [M+H]+, [M+Na]+, [M+Na+CH₃OH]+ de la molécule attendue C₁₄H₁₉N₅O₂ sont principalement détectés.

### Synthèse du 1-méthyl-1-{2-[(3-nitropyrazolo[1,5-a]pyridin-2-yl)amino]éthyl}pipéridinium méthyl sulfate

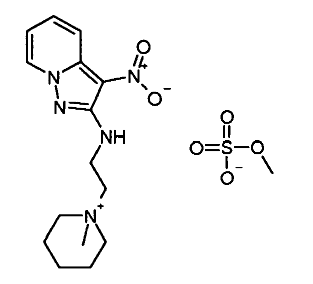

Dans un ballon de 250 ml munit d'un compte bulle et d'une agitation magnétique, on introduit 120 de THF et 17g (0,05875 mole) de 3-nitro-N-(2-pipéridin-1-yléthyl)pyrazolo[1,5-a]pyridin-2-amine, 11,12 ml (0,1175mole) de diméthylsulfate et l'ensemble est agité pendant 24 heures à la température ambiante.

L'insoluble formé est essoré sur fritté n°3 puis lavé avec 3x 100 d'éther isopropylique sous argon suivi du séchage à 35°C sous vide, en présence de P₂O₅.
Nous récupérons 23,84g de solide jaune correspondant au composé attendu.

Les analyses RMN (¹H 400 MHz et ¹³C 100,61 MHz DMSO d₆) sont conformes à la structure attendue.
Le cation attendu [C₁₅H₂₂N₅O₂]+ est principalement détecté à m/z, ESP+=304.

### Synthèse du dichlorhydrate de chlorure de 1-{2-[(3-aminopyrazolo[1,5-a]pyridin-2-yl)amino]éthyl}-1-méthylpipéridinium.

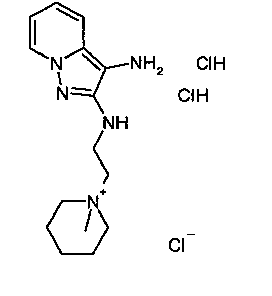

Dans un tricol de 500 ml, surmonté d'un réfrigérant à boules, d'un thermomètre et d'une agitation magnétique, on introduit 300 ml d'éthanol et 10g de poudre de zinc et on porte au reflux.
On ajoute alors au goutte à goutte 0,5 ml d'acide acétique. Une solution composée de 20ml d'éthanol, 8ml d'eau, 5ml d'acide acétique et 10g de 1-méthyl-1-{2-[(3-nitropyrazolo[1,5-a]pyridin-2-yl)amino]éthyl}pipéridinium méthyl sulfate est alors ajoutée en 15 minutes et le reflux est maintenu 20 minutes.

Lorsque la réduction est terminée, le zinc est éliminé par filtration sous argon sur un lit de célite et les jus récoltés dans une fiole contenant 100ml d'isopropanol chlorhydrique, lavé avec de l'éthanol et les jus réunis sont acidifiés avec de l'isopropanol chlorhydrique, 6N préalablement refroidi.
On observe alors la précipitation d'un solide bleu que l'on essore sur fritté n°3 et que l'on lave avec 2x15ml d'éthanol et 2x50 ml d'éther isopropylique. Après séchage sous vide, en présence de P₂O₅ et de pastilles de soude , on obtient 9,1g d'une poudre bleu correspondant au composé attendu.

Le cation attendu [C₁₅H₂₄N₅]+ ainsi que les ions fragments [C₁₄H₂₁N₅]+., [C₉H₁₁N₄]+, [C₆H₁₄N]+ (détectés respectivement à m/z,ESP+=274, 259, 175, 100) et l'adduit [M+2Cl]- sont principalement détectés.

Les analyses RMN (¹H 400 MHz et ¹³C 100,61 MHz DMSO d₆) sont conformes à la structure attendue.

### EXEMPLE 4 :

### Synthèse du 3-nitro-N-(2-pyrrolidin-1-yléthyl)pyrazolo[1,5-a]pyridin-2-amine.

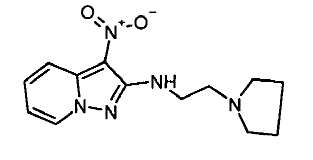

Dans un tricol de 100 ml, surmonté d'un réfrigérant à boules, d'un thermomètre et d'une agitation magnétique, on introduit 20 ml de NMP, 15g (0,062 mole) 2-méthanesulfanyl-3-nitro-pyrazol[1,5-a]pyridine et 16ml de 2,3-aminoéthylpyrrolidine. Sous agitation, on porte à 80°C à l'aide d'un bain d'huile, pendant 1 heure en suivant par CCM (éluant : 90:5 acétate d'éthyle / Méthanol).
Le milieu réactionnel est refroidi à la température ambiante puis versé sur un mélange de 400g de glace et d'eau. Le précipité jaune beige formé est essoré sur fritté n °4 puis lavé à l'eau plusieurs fois. Après séchage à 35°C sous vide, en présence de P₂O₅, on récupère 15,2g de solide jaune correspondant au composé attendu.

Les analyses RMN (¹H 400 MHz et ¹³C 100,61 MHz DMSO d₆) et spectrométrie de masse sont conformes à la structure attendue.

### Synthèse du 1-méthyl-1-{2-[(3-nitropyrazolo[1,5-a]pyridin-2-yl)amino]éthyl}pyrrolidinium méthyl sulfate

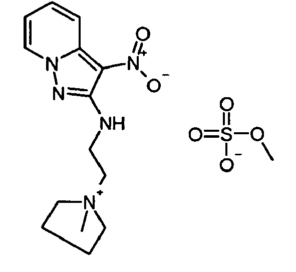

Dans un ballon de 250 ml équipé d'un compte bulle et d'une agitation magnétique, on introduit 100 de THF et 10g (0.0363 mole) de 3-nitro-N-(2-pyrrolidin-1-yléthyl)pyrazolo[1,5-a]pyridin-2-amine , 6,7 ml (0,0726 mole) de diméthylsulfate et l'ensemble est porté au reflux sous agitation pendant 2 heures.

L'insoluble formé après refroidissement à température ambiante est essoré sur fritté n°3 puis lavé avec 3x 20 de THF puis 3x100 ml d'éther de pétrole sous argon suivi du séchage sous vide, en présence de P₂O₅.
Nous récupérons 13,1g de solide jaune correspondant au composé attendu.

Les analyses RMN (¹H 400 MHz et ¹³C 100,61 MHz DMSO d₆) et spectrométrie de masse sont conformes à la structure attendue.

### Synthèse du chlorhydrate de chlorure de 1-{2-[(3-aminopyrazolo[1,5-a]pyridin-2-yl)amino]éthyl}-1-méthylpyrrolidinium.

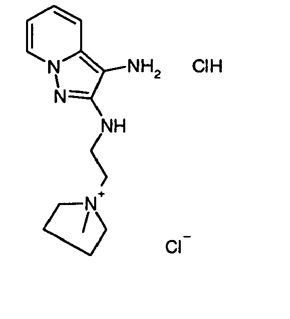

Dans un tricol de 500 ml, surmonté d'un réfrigérant à boules, d'un thermomètre et d'une agitation magnétique, on introduit 250 ml d'éthanol et 10g de poudre de zinc et on porte au reflux.
On ajoute alors au goutte à goutte 2 ml d'acide acétique puis une solution de 5ml d'eau et 5g de 1-méthyl-1-{2-[(3-nitropyrazolo[1,5-a]pyridin-2-yl)amino] éthyl} pyrrolidinium méthyl sulfate.
En fin de coulée, on ajoute au goutte à goutte 1 ml d'acide acétique et le reflux est maintenu 2 heures.

Lorsque la réduction est terminée, le zinc est éliminé par filtration sous argon sur un lit de célite et les jus récoltés dans une fiole contenant 50ml d'isopropanol chlorhydrique 6N préalablement refroidi.
On observe alors la précipitation d'un solide gris bleu que l'on essore sur fritté n°3 et que l'on lave avec 2x15ml d'éthanol et 2x50 ml d'éther isopropylique. Après séchage sous vide, en présence de P₂O₅ et de pastilles de soude, nous récupérons 4g d'une poudre gris bleu correspondant au composé attendu.
Les analyses RMN (¹H 400 MHz et ¹³C 100,61 MHz DMSO d₆) sont conformes à la structure attendue.

Le cation attendu [C₁₄H₂₂N₅]+ est principalement détecté à m/z,ESP+=260.

### EXEMPLE 5 :

### Synthèse du N-(2-morpholin-4-yléthyl)-3-nitropyrazolo[1,5-a]pyridin-2-amine.

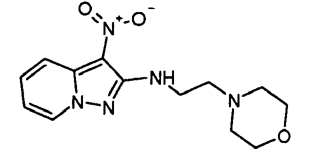

Dans un tricol de 100 ml, surmonté d'un réfrigérant à boules, d'un thermomètre et d'une agitation magnétique, on introduit 15 ml de NMP, 10g (0,0415 mole) de 2-méthanesulfanyl-3-nitro-pyrazol[1,5-a]pyridine et 11ml de 2-aminoéthylmorpholine. Sous agitation, on porte à 80°C à l'aide d'un bain d'huile, pendant 1 heure en suivant par CCM (éluant : 90:5 acétate d'éthyle / Méthanol).
On refroidit le milieu à la température ambiante et le milieu est versé sur un mélange de 400g de glace et d'eau. Le précipité jaune beige formé est essoré sur fritté n °4 puis lavé à l'eau plusieurs fois. Après séchage à 35°C sous vide, en présence de P₂O₅, on récupère 10,4g de solide jaune correspondant au composé attendu.

Les analyses RMN (¹H 400 MHz et ¹³C 100,61 MHz DMSO d₆) et spectrométrie de masse sont conformes à la structure attendue.

### Synthèse de 4-méthyl-4-{2-[(3-nitropyrazolo[1,5-a]pyridin-2-yl)amino]éthyl}morpholin-4-ium méthyl sulfate.

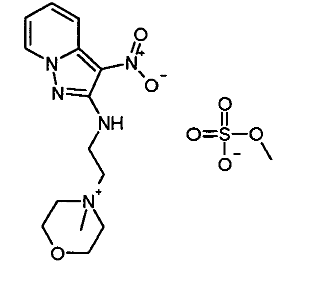

Dans un ballon de 250 ml équipé d'un compte bulle et d'une agitation magnétique, on introduit 100 ml de THF et 10g ( 0,03433 mole) de N-(2-morpholin-4-yleéthyl)-3-nitropyrazolo[1,5-a]pyridin-2-amine, 6,7 ml (0,0726 mole) de diméthylsulfate et l'ensemble est agité et porté au reflux pendant 2 heures.

L'insoluble formé après refroidissement à la température ambiante est essoré sur fritté n°3 puis lavé avec 3x 20 ml de THF puis 3x100 ml d'éther de pétrole sous argon suivi du séchage sous vide, en présence de P₂O₅.
Nous récupérons 13,9g de solide jaune correspondant au composé attendu.

Les analyses RMN (¹H 400 MHz et ¹³C 100,61 MHz DMSO d₆) et spectrométrie de masse sont conformes à la structure attendue.

### Synthèse du chlorhydrate de chlorure de 4-{2-[(3-aminopyrazolo[1,5-a]pyridin-2-yl)amino]éthyl}-4-méthylmorpholin-4-ium.

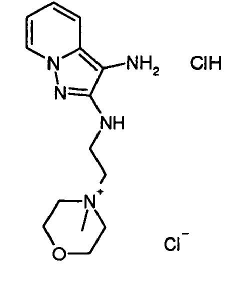

Dans un tricol de 500 ml, surmonté d'un réfrigérant à boules, d'un thermomètre et d'une agitation magnétique, on introduit 250 ml d'éthanol et 10g de poudre de zinc et on porte au reflux .
On ajoute alors, goutte à goutte, 2 ml d'acide acétique, puis une solution de 5ml d'eau et 5g de 1-méthyl-1-{2-[(3-nitropyrazolo[1,5-a]pyridin-2-yl)amino] éthyl} pyrrolidinium méthyl sulfate.
En fin de coulée, on ajoute au goutte à goutte 1 ml d'acide acétique et le reflux est maintenu 1,5 heures.

Lorsque la réduction est terminée, le zinc est éliminé par filtration sous argon sur un lit de célite et les jus récoltés dans une fiole contenant 50ml d'isopropanol chlorhydrique 6N préalablement refroidi.
On observe alors la précipitation d'un solide que l'on essore sur fritté n°3 et que l'on lave avec 2x15ml d'éthanol et 2x50 ml d'éther isopropylique. Après séchage sous vide, en présence de P₂O₅ et de pastilles de soude, nous récupérons 3,8g d'une poudre gris bleu correspondant au composé attendu.

Les analyses RMN (¹H 400 MHz et ¹³C 100,61 MHz DMSO d₆) sont conformes à la structure attendue.
Le cation attendu [C₁₄H₂₂N₅O] + est principalement détecté

### EXEMPLE 6 :

### Synthèse du N,N-diisopropyl-N'-(3-nitropyrazolo[1,5-a]pyridin-2-yl)éthane-1,2-diamine.

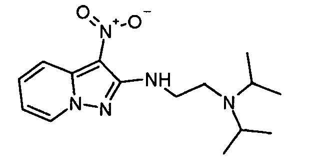

Dans un tricol de 100 ml, surmonté d'un réfrigérant à boules, d'un thermomètre et d'une agitation magnétique, on introduit 20ml de NMP, 10g (0,0414 mole) 2-méthanesulfanyl-3-nitro-pyrazol[1,5-a]pyridine et 14,4ml (0,082mole de N,N-diisopropyléthane-1,2-diamine. Sous agitation, on porte à 70°C à l'aide d'un bain d'huile, pendant 1 heure en suivant par CCM (éluant : 95:5 Dichlorométhane /Méthanol).

le milieu réactionnel est refroidi à la température ambiante versé sur un mélange de 100g de glace et d'eau . Le précipité jaune formé est essoré sur fritté n°3 puis lavé à l'eau puis avec 3x100ml d'éther de pétrole . Après séchage à 35°C sous vide, en présence de P₂O₅, on récupère 11,88g de solide jaune correspondant au composé attendu.

Les analyses RMN (¹H 400 MHz et ¹³C 100,61 MHz DMSO d₆) sont conformes à la structure attendue.

Les ions quasi moléculaires [M+H]+, [M+Na]+, [M+Na+CH₃OH]+ de la molécule attendue C₁₅H₂₃N₅O₂ sont principalement détectés.

### Synthèse du N-isopropyl-N-méthyl-N-{2-[(3-nitropyrazolo[1,5-a]pyridin-2-yl)amino]éthyl}propan-2-aminium méthyl sulfate

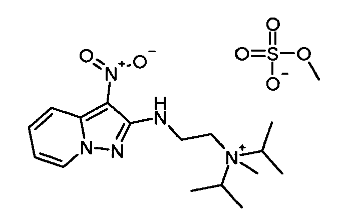

Dans un ballon de 10 ml équipé d'un compte bulle et d'une agitation magnétique, on introduit 45 ml de THF et 6,4g (0,02095 mole) de N-diisopropyl-N'-(3-nitropyrazolo[1,5-a]pyridin-2-yl)éthane-1,2-diamine et 3,96 ml (0,04191 mole) de diméthylsulfate et l'ensemble est agité à 65°C pendant 5 heures.

L'insoluble formé à température ambiante est essoré sur fritté n°3 puis lavé avec 15 ml de THF puis 3x 25ml de dichlorométhane THF et 3x15 ml d' éther isopropylique sous argon suivi du séchage à 35°C sous vide, en présence de P₂O₅.
Nous récupérons 4,85g de solide jaune correspondant au composé attendu.

Les analyses RMN (¹H 400 MHz et ¹³C 100,61 MHz DMSO d₆) et spectrométrie de masse sont conformes à la structure attendue.

### Synthèse du dichlorydrate de chlorure de N-{2-[(3-aminopyrazolo[1,5-a]pyridin-2-yl)amino]éthyl}-N-isopropyl-N-méthylpropan-2-aminium

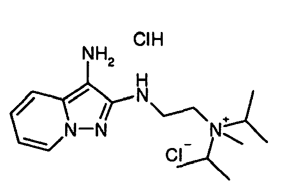

Dans un tricol de 250 ml, surmonté d'un réfrigérant à boules, d'un thermomètre et d'une agitation magnétique, on introduit 150 ml d'éthanol et 3g de poudre de zinc et on porte au reflux et on ajoute 0,2 ml d'acide acétique au goutte à goutte.
une solution contenant 8ml d'éthanol, 4ml d'eau, 3ml d'acide acétique et 3g de N,N-diéthyl-N-méthyl-2-[(3-nitropyrazolo[1,5-a]pyridin-2-yl)amino]éthanaminium méthyl sulfate est ensuite ajoutée en 15 minutes et le reflux est maintenu 15 minutes.

Lorsque la réduction est terminée, le zinc est éliminé par filtration sous argon sur un lit de célite et les jus récoltés dans une fiole contenant 25ml d'isopropanol chlorhydrique 6N préalablement refroidi.
Les jus sont alors concentrés jusqu'à obtention de 40ml et un début de cristallisation d'un solide gris bleu.
Ce solide est essoré sur fritté n°3, lavé avec de 2x60ml d' éther isopropylique et après séchage sous vide en présence de P₂O₅, nous récupérons 3,27 d'un solide gris bleu correspondant au composé attendu.

Les analyses RMN (¹H 400 MHz et ¹³C 100,61 MHz DMSO d₆) et spectrophotométrie de masse sont conformes à la structure attendue.

### EXEMPLE 7 :

### Synthèse du N,N-diéthyl-N'-(3-nitropyrazolo[1,5-a]pyridin-2-yl)éthane-1,2-diamine.

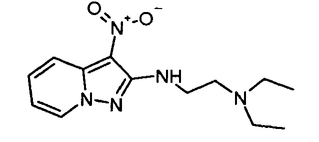

Dans un tricol de 100 ml, surmonté d'un réfrigérant à boules, d'un thermomètre et d'une agitation magnétique, on introduit 100 ml de NMP, 10g (0,0414 mole) 2-méthanesulfanyl-3-nitro-pyrazol[1,5-a]pyridine et 11,65 ml (0,0829mol) de N,N-diéthylènediamine . Sous agitation, on porte à 70°C à l'aide d'un bain d'huile, pendant 4 heures en suivant par CCM (éluant : 95:5 Dichlorométhane / Méthanol).

On refroidit le milieu à la température ambiante et le milieu est versé sur un mélange de 100g de glace et d'eau. Le composé jaune qui a cristallisé est essoré sur fritté n°3 puis lavé 3x100ml d'éther de pétrole. Après séchage à 35°C sous vide, en présence de P₂O₅, on récupère 9,80g de solide jaune correspondant au composé attendu.

Les analyses RMN (¹H 400 MHz et ¹³C 100,61 MHz DMSO d₆) sont conformes à la structure attendue.

Les ions quasi moléculaires [M+H]+, [M+Na]+, [M+Na+CH₃OH]+ de la molécule attendue C₁₃H₁₉N₅O₂ sont principalement détectés.

### Synthèse du N,N-diéthyl-N-methyl-2-[(3-nitropyrazolo[1,5-a]pyridin-2-yl)amino]éthanaminium méthyl sulfate

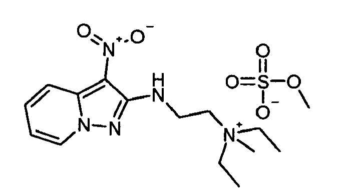

Dans un monocol de 50 ml équipé d'un compte bulle et d'une agitation magnétique, on introduit 20 ml de THF et 4g (0,01658 mole) de N,N-diéthyl-N'-(3-nitropyrazolo[1,5-a]pyridin-2-yl)éthane-1,2-diamine et 3,13ml (0,03316mole) de diméthylsulfate et l'ensemble est agité pendant 24 heures à la température ambiante.

L'insoluble formé est essoré sur fritté n°3 puis lavé avec 3x 15ml de THF et 3x15 ml d' éther isopropylique sous argon suivi du séchage à 35°C sous vide, en présence de P₂O₅.
Nous récupérons 5,48g de solide jaune correspondant au composé attendu.

Les analyses RMN (¹H 400 MHz et ¹³C 100,61 MHz DMSO d₆) sont conformes à la structure attendue.

Le cation attendu [C₁₄H₂₂N₅O₂]+ est principalement détecté à m/z,ESP+=292.

### Synthèse du chlorhydrate de chlorure de2-[(3-aminopyrazolo[1,5-a]pyridin-2-yl)amino]-N,N-diéthyl-N-méthyléthanaminium

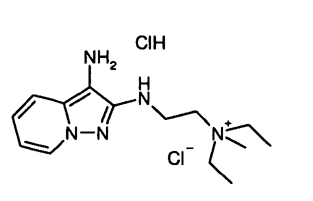

Dans un tricol de 250 ml, surmonté d'un réfrigérant à boules, d'un thermomètre et d'une agitation magnétique, on introduit 150 ml d'éthanol et 3g de poudre de zinc et on porte au reflux.
On ajoute alors au goutte à goutte 0,5 ml d'acide acétique. Une solution composée de 10ml d'éthanol, 2ml d'eau, 3ml d'acide acétique et 3g N-isopropyl-N-méthyl-N-{2-[(3-nitropyrazolo[1,5-a]pyridin-2-yl)amino]éthyl}propan-2-aminium méthyl sulfate est alors ajoutée en 15 minutes et le reflux est maintenu 15 minutes.

Lorsque la réduction est terminée, le zinc est éliminé par filtration sous argon sur un lit de célite et les jus récupérés dans une fiole contenant 25ml d'isopropanol chlorhydrique 6N préalablement refroidi.
Les jus sont alors concentrés jusqu'à obtention d'une solution laiteuse et début de cristallisation.

Le solide formé est essoré sur fritté n°3, lavé avec de 3x40ml d' éther isopropylique et après séchage sous vide en présence de P₂O₅ et de pastilles de soude, nous récupérons 2,25g d'un solide bleu vert correspondant au composé attendu.

Les analyses RMN (¹H 400 MHz et ¹³C 100,61 MHz DMSO d₆) et spectrophotométrie de masse sont conformes à la structure attendue.

### EXEMPLE 8 :

### Synthèse du 3-nitro-N-(2-pyridin-3-yléthyl)pyrazolo[1,5-a]pyridin-2-amine

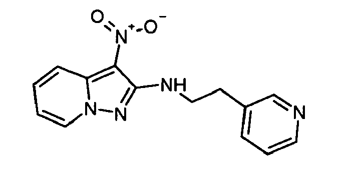

Dans un tricol de 100 ml, surmonté d'un réfrigérant à boules, d'un thermomètre et d'une agitation magnétique, on introduit 10 ml de NMP, 4,93g (0,02046 mole) 2-méthanesulfanyl-3-nitro-pyrazol[1,5-a]pyridine et 5g ml(0,04092 mole) de 3-(2-aminoéthyl)-pyridine. Sous agitation, on porte à 70°C à l'aide d'un bain d'huile, pendant 1 heure en suivant par CCM (éluant : 95:5 Dichlorométhane / Méthanol).

Le milieu réactionnel est refroidi jusqu'à la température ambiante puis versé sur un mélange de 100g de glace et d'eau. Le composé jaune qui a cristallisé est essoré sur fritté n °3, lavé à l'eau puis avec 3x100ml d'éther de pétrole. Après séchage à 35 °C sous vide, en présence de P₂O₅, on récupère 5,61 g de solide jaune correspondant au composé attendu.

Les analyses RMN (¹H 400 MHz et ¹³C 100,61 MHz DMSO d₆) sont conformes à la structure attendue.

Les ions quasi moléculaires [M+H]+, [M+Na]+, [M+Na+CH₃OH]+, [2M+H]+, [2M+Na]+ de la molécule attendue C₁₄H₁₃N₅O₂ sont principalement détectés.

### Synthèse du 1-méthyl-3-{2-[(3-nitropyrazolo[1,5-a]pyridin-2-yl)amino]éthyl}pyridinium méthyl sulfate

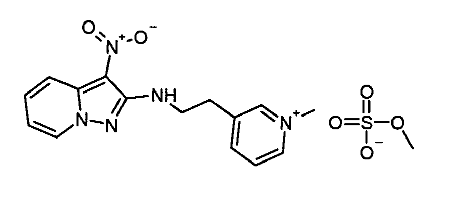

Dans un ballon de 50 ml équipé d'un compte bulle et d'une agitation magnétique, on introduit 50 ml de THF et 3g (0,01058mole) de 3-nitro-N-(2-pyridin-3-yléthyl)pyrazolo[1,5-a]pyridin-2-amine et 2ml (0,02117mole) de diméthylsulfate. L'ensemble est agité pendant 18 heures à la température ambiante. L'insoluble formé est essoré sur fritté n°3 puis lavé avec 3x 15ml de THF et 3x15 ml d' éther isopropylique sous argon suivi du séchage à 35°C sous vide, en présence de P₂O₅.
Nous récupérons 4,07g de solide jaune correspondant au composé attendu.

Les analyses RMN (1 H 400 MHz et 13C 100,61 MHz DMSO d6) sont conformes à la structure attendue. Le cation attendu [C₁₅H₁₆N₅O₂]+ est principalement détecté à m/z,ESP+=298

### Synthèse du chlorhydrate de chlorure de 3-{2-[(3-aminopyrazolo[1,5-a]pyridin-2-yl)amino]éthyl}-1-méthylpyridinium

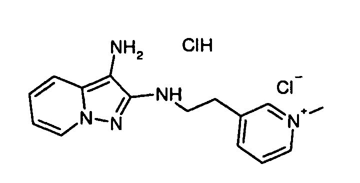

Dans un tricol de 250 ml, surmonté d'un réfrigérant à boules, d'un thermomètre et d'une agitation magnétique, on introduit 150 ml d'éthanol et 3g de poudre de zinc et on porte au reflux.
On ajoute alors au goutte à goutte 0,2 ml d'acide acétique. Une solution composée de 8ml d'éthanol, 4ml d'eau, 2ml d'acide acétique et 3g
1-méthyl-3-{2-[(3-nitropyrazolo[1,5-a]pyridin-2-yl)amino]éthyl}pyridinium méthyl sulfate est ensuite ajoutée en 15 minutes et le reflux est maintenu 15 minutes.

Lorsque la réduction est terminée, le zinc est éliminé par filtration sous argon sur un lit de célite et les jus récupérés dans une fiole contenant 25ml d'isopropanol chlorhydrique 6N préalablement refroidi.
Les jus sont alors concentrés jusqu'à l'obtention d'un début de cristallisation puis refroidis à zéro degré.

Le solide formé est essoré sur fritté n°3, lavé avec de 3x40ml d' éther isopropylique et après séchage sous vide en présence de P₂O₅ et de pastilles de soude, nous récupérons 3,39g de solide vert correspondant au composé attendu.

Les analyses RMN (¹H 400 MHz et ¹³C 100,61 MHz DMSO d₆) et spectrophotométrie de masse sont conformes à la structure attendue.

### EXEMPLE 9 :

### Synthèse du 3-nitro-N-(2-pyridin-4-yléthyl)pyrazolo[1,5-a]pyridin-2-amine

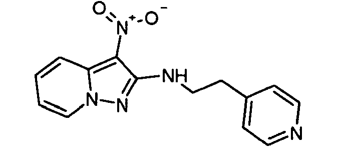

Dans un tricol de 100 ml, surmonté d'un réfrigérant à boules, d'un thermomètre et d'une agitation magnétique, on introduit 50 ml de NMP, 10g (0,03555 mole) 2-méthanesulfanyl-3-nitro-pyrazol[1,5-a]pyridine et 8,68g (0,07110mole) de 4-(2-aminoéthyl). Sous agitation, on porte à 70°C à l'aide d'un bain d'huile, pendant 4 heures en suivant par CCM (éluant : 95:5 Dichlorométhane / Méthanol).

On refroidit le milieu à la température ambiante et le milieu est versé sur un mélange de 200g de glace et d'eau. Le composé jaune qui a cristallisé est essoré sur fritté n°3, lavé à l'eau puis avec 3x100ml d'éther de pétrole. Après séchage à 35°C sous vide, en présence de P₂O₅, pendant 12 heures, on récupère 10,81g (produit non totalement sec) de solide jaune correspondant au composé attendu.

Les analyses RMN (¹H 400 MHz et ¹³C 100,61 MHz DMSO d₆) et spectrométrie de masse sont conformes à la structure attendue.

### Synthèse du 1-méthyl-4-{2-[(3-nitropyrazolo[1,5-a]pyridin-2-yl)amino]éthyl}pyridinium méthyl sulfate

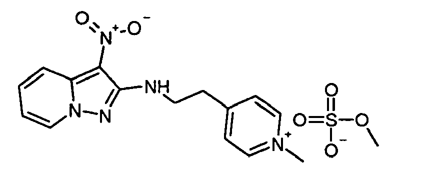

Dans un ballon de 100 ml équipé d'un compte bulle et d'une agitation magnétique, on introduit 50 ml de THF et 8g (0,01058mole) de 3-nitro-N-(2-pyridin-4-yléthyl)pyrazolo[1,5-a]pyridin-2-amine et 5,34ml (0,05648 mole) de diméthylsulfate et l'ensemble est agité pendant 24 heures à la température ambiante.
L'insoluble formé est essoré sur fritté n°3 puis lavé avec 3x 50ml de THF et 3x50 ml d' éther isopropylique sous argon suivi du séchage à 35°C sous vide, en présence de P₂O₅.
Nous récupérons 11,06g de solide jaune correspondant au composé attendu.

Les analyses RMN (¹H 400 MHz et ¹³C 100,61 MHz DMSO d₆) et spectrométrie de masse sont conformes à la structure attendue.

### Synthèse du chlorhydrate de chlorure de 3-{2-[(3-aminopyrazolo[1,5-a]pyridin-2-yl)amino]éthyl}-1-méthylpyridinium

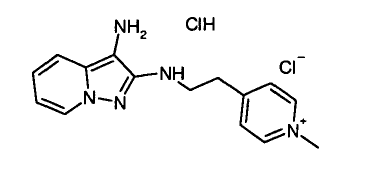

Dans un tricol de 250 ml, surmonté d'un réfrigérant à boules, d'un thermomètre et d'une agitation magnétique, on introduit 150 ml d'éthanol et 3g de poudre de zinc et on porte au reflux.
On ajoute alors au goutte à goutte 0,2 ml d'acide acétique. Une solution composée de 8ml d'éthanol, 3ml d'eau, 2ml d'acide acétique et 3g 1-méthyl-4-{2-[(3-nitropyrazolo[1,5-a]pyridin-2-yl)amino]éthyl}pyridinium méthyl sulfate est alors ajoutée en 15 minutes et le reflux est maintenu 15 minutes.

Lorsque la réduction est terminée, le zinc est éliminé par filtration sous argon sur un lit de célite et les jus récupérés dans une fiole contenant 25ml d'isopropanol chlorhydrique 6N préalablement refroidi à la glace.
Ces jus sont alors concentrés jusqu'à l'obtention d'un début de cristallisation puis refroidis à zéro degré.

Le solide formé est essoré sur fritté n°3, lavé avec de 3x40ml d' éther isopropylique et après séchage sous vide en présence de P₂O₅ et de pastilles de soude, nous récupérons 1,42g de solide vert correspondant au composé attendu.

Les analyses RMN (¹H 400 MHz et ¹³C 100,61 MHz DMSO d₆) et spectrophotométrie de masse sont conformes à la structure attendue.

### EXEMPLE 10 :

L'exemple est obtenu à partir du schéma général suivant :

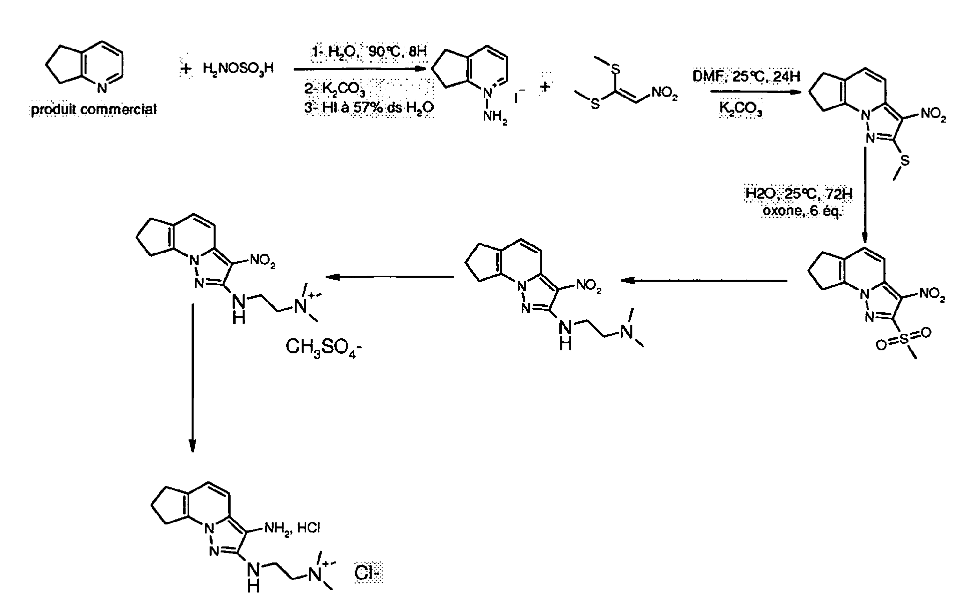

### Synthèse du 1-amino-6,7-dihydro-5H-cyclopenta[b]pyridinium iodide

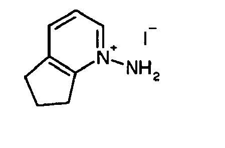

Dans un tricol de 1000 ml, surmonté d'un réfrigérant à boules, d'un thermomètre et d'une agitation mécanique, on introduit 400 ml d'eau, 123g (1,032 mole) 2,3-cyclopentenopyridine puis 46,6g (0,413mole) d'acide hydroxylamine-o-sulfonique par petite portion et porter au reflux pendant 18 heures.
On refroidit le milieu à la température ambiante et rajouter doucement 74,2g (0,537mole) de carbonate de potassium et agiter pendant 30 minutes.
Laver la phase aqueuse avec 4x200ml d'acétate d'éthyle, la phase aqueuse est co-évaporée avec du 2-propanol pour obtenir un solide marron que l'on reprend avec 400ml d'éthanol pour éliminer les sels.
La solution d'éthanol brune est introduite dans un tricol de 2 litres muni d'une ampoule isobare est sous agitation à -70°C. On coule, goutte à goutte, 67,5ml (0,516mole) d'acide iodhydrique.
En fin de coulée, laisser la température revenir à zéro degré et essorer l'insoluble beige sur fritté n°3. Ce solide est lavé avec 3x150ml d'éther isopropylique. Après séchage sous vide, en présence de P₂O₅, pendant 12 heures, on récupère 27,7g de solide beige correspondant au composé attendu.

Les analyses RMN (¹H 400 MHz et ¹³C 100,61 MHz DMSO d₆) et spectrométrie de masse sont conformes à la structure attendue.

Le cation attendu [C₈H₁₁N₂]+ est principalement détecté à m/z,ESP+=135. Détection d'ions I- en électrospray négatif.

### Synthèse du 2-(méthylsulfanyl)-3-nitro-7,8-dihydro-6H-cyclopenta[e]pyrazolo[1,5-a]pyridine

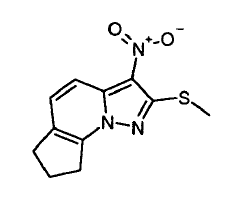

Dans un tricol de 2 l équipé d'un compte bulle, d'un réfrigèrent et d'une agitation mécanique, on introduit 500ml de NMP et 65,56g (0,025mole) de 1-amino-6,7-dihydro-5H-cyclopenta[b]pyridinium iodure, sous agitation on ajoute doucement 103,65g de carbonate de potassium en 15 minutes puis en une seule fois 41,33g (0,25mole) de 1,1-bis(méthythio)-2-nitroéthylène.
L'agitation est maintenue pendant 48 heures à la température ambiante est les jus sont versés sur 2,5 litres de mélange glace - eau.
L'insoluble vert foncé formé est essoré sur fritté n °3 puis lavé abondamment à l'eau, 3x 200ml d'acétate d'éthyle puis avec 3x200 ml d' éther isopropylique.
Après séchage sous vide, en présence de P₂O₅, nous récupérons 36,77g de solide vert foncé correspondant au composé attendu.

Les analyses RMN (¹H 400 MHz et ¹³C 100,61 MHz DMSO d₆) et spectrométrie de masse sont conformes à la structure attendue.

Les ions quasi moléculaires [M+H]+, [M+Na]+, [2M+Na]+ de la molécule attendue C₁₁H₁₁N₃O₂S sont principalement détectés .

### Synthèse du 2-(méthylsulfonyl)-3-nitro-7,8-dihydro-6H-cyclopenta[e]pyrazolo[1,5-a]pyridine

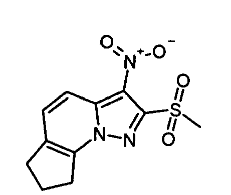

Dans un tricol de 3 litres, équipé d'une agitation mécanique et d'une sonde de température interne, sont chargés successivement 267,6 g d'oxone (3 éq.), 800 ml d'eau et 36,17g de 2-(méthylsulfanyl)-3-nitro-7,8-dihydro-6H-cyclopenta[e]pyrazolo[1,5-a]pyridine (0,145 mole) obtenu précédemment. Le tout est agité à la température ambiante.
Pour compléter la réaction, de l'oxone (89,2g, 1 éq.) est additionné et après 4 heures sous agitation à la température ambiante, la réaction est terminée.
Le solide formé est essoré et lavé abondamment à l'eau jusqu'à l'obtention d'un filtrat ne contenant plus de péroxydes. Il est ensuite placé sous vide à 40°C sur P₂O₅. 35,31g de produit attendu sont obtenus (solide jaune).

Les analyses RMN (¹H 400 MHz et ¹³C 100,61 MHz DMSO d₆) et spectrophotométrie de masse sont conformes à la structure attendue.
¹H NMR (DMSO-d6): 2.31 (m, 2H), 3.13(t,2H), 3.38 (t, 2H), 3.59 (s, 3H), 8.01 (d, 1H), 8.21 (d, 1 H).

Les ions quasi moléculaires [M-H]-, [M+H]+, [M+Na]+, [M+NA+CH₃OH]+, [2M+Na]+ de la molécule attendue C₁₁H₁₁N₃O₄S sont principalement détectés.

### Synthèse du N,N-diméthyl-N'-(3-nitro-7,8-dihydro-6H-cyclopenta[e]pyrazolo[1,5-a]pyridin-2-yl)éthane-1,2-diamine

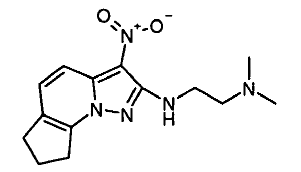

Dans un tricol de 250 ml, surmonté d'un réfrigérant à boules, d'un thermomètre et d'une agitation magnétique, on introduit 50 ml de NMP, 10g (0,03555 mole) 2-(méthylsulfonyl)-3-nitro-7,8-dihydro-6H-cyclopenta[e]pyrazolo[1,5-a]pyridine et 7,8 ml de N.N-diméthyléthylène diamine, on porte à 70°C à l'aide d'un bain d'huile, pendant 4 heures en suivant par CCM (éluant : 95:5 Dichlorométhane / Méthanol).

Le milieu réactionnel est refroidi à la température ambiante puis versé sur un mélange de 200g de glace et d'eau .Le composé jaune qui a cristallisé est essoré sur fritté n°3, lavé à l'eau 2x100ml puis avec 3x100ml d'éther isopropylique. Après séchage à 35°C sous vide, en présence de P₂O₅, pendant 12 heures, on récupère 8,32g de solide vert correspondant au composé attendu.

Les analyses RMN (¹H 400 MHz et ¹³C 100,61 MHz DMSO d₆) et spectrophotométrie de masse sont conformes à la structure attendue.
¹H NMR (DMSO-d6): 2.21 (m+s, 2H), 3.01 (t,2H), 3.20 (t, 2H), 3.48 (q, 2H), 7.27 (t, 1 H), 7.66 (d, 1 H), 7.83(d, 1 H).

Les ions quasi moléculaires de la molécule attendue C₁₄H₁₉N₅O₂ sont principalement détectés.

### Synthèse du N,N,N-triméthyl-2-[(3-nitro-7,8-dihydro-6H-cyclopenta[e]pyrazolo[1,5-a]pyridin-2-yl)amino]éthanaminium méthyl sulfate

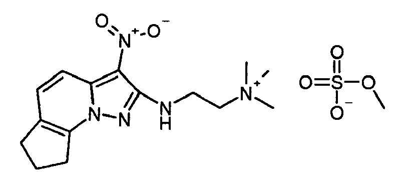

Dans un ballon de 100 ml équipé d'un compte bulle et d'une agitation magnétique, on introduit 45 ml de THF et 6,3g (0,02177 mole) de N,N-diméthyl-N'-(3-nitro-7,8-dihydro-6H-cyclopenta[e]pyrazolo[1,5-a]pyridin-2-yl)éthane-1,2-diamine et 4,12ml (0,04354mole) de diméthylsulfate et l'ensemble est agité pendant 24 heures à la température ambiante.

L'insoluble formé est essoré sur fritté n°3 puis lavé avec 3x 50ml de THF et 3x50 ml d'éther isopropylique sous argon suivi du séchage à 35°C sous vide, en présence de P₂O₅.
Nous récupérons 8,73g de solide jaune correspondant au composé attendu.

Les analyses RMN (¹H 400 MHz et ¹³C 100,61 MHz DMSO d₆) et spectrométrie de masse sont
Le cation attendu [C₁₅H₂₂N₅O₂]+ est principalement détecté. Nous détectons également en électrospray négatif le CH₃OSO₃-.

### Synthèse du chlorhydrate de chlorure de 3-[(3-amino-7,8-dihydro-6H-cyclopenta[e]pyrazolo[1,5-a]pyridin-2-yl)amino]-N,N,N-triméthylpropan-1-aminium

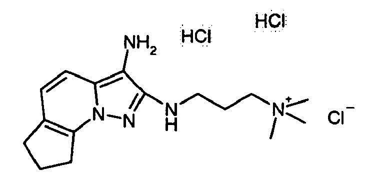

Dans un tricol de 250 ml, surmonté d'un réfrigérant à boules, d'un thermomètre , d'une ampoule de coulée et d'une agitation magnétique, on introduit 100 ml d'éthanol et 2g de poudre de zinc et on porte au reflux.
On ajoute alors au goutte à goutte 0,5 ml d'acide acétique puis une solution composée de 8ml d'éthanol, 3ml d'eau, 2ml d'acide acétique et 2g de N,N,N-triméthyl-2-[(3-nitro-7,8-dihydro-6H-cyclopenta[e]pyrazolo[1,5-a]pyridin-2-yl)amino]éthanaminium méthyl sulfate. Le reflux est maintenu 90 minutes.

Lorsque la réduction est terminée, le zinc est éliminé par filtration sous argon sur un lit de célite et le filtrat dans une fiole contenant 25ml d'isopropanol chlorhydrique 6N préalablement refroidi.

Ce solide bleu clair formé est essoré sur fritté n°3, lavé avec de 2x60ml d'éther isopropylique et après séchage sous vide en présence de P₂O₅ et de pastilles de soude, nous récupérons 2,64g de solide bleu-vert correspondant au composé attendu.

Les analyses RMN (¹H 400 MHz et ¹³C 100,61 MHz DMSO d₆) et spectrophotométrie de masse sont conformes à la structure attendue.

### EXEMPLE 11 :

### Synthèse du dichlorhydrate de chlorure de2-[(3-amino-6,7-diméthylpyrazolo[1,5-a]pyridin-2-yl)amino]-N,N,N-triméthyléthanaminium

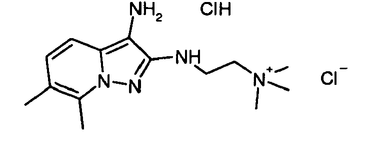

Dans un tricol de 500 ml, surmonté d'un réfrigérant à boules, d'un thermomètre et d'une agitation magnétique, on introduit 250 ml d'éthanol et 10g de poudre de zinc et on porte au reflux.
On ajoute alors au goutte à goutte 2 ml d'acide acétique puis une solution composée de 5ml d'eau et 5g de 2-[(6,7-diméthyl-3-nitropyrazolo[1,5-a]pyridin-2-yl)amino]-N,N,N-triméthyléthanaminium méthyl sulfate.
En fin de coulée, on ajoute au goutte à goutte 1 ml d'acide acétique et le reflux est maintenu 1,5 heures.

Lorsque la réduction est terminée, le zinc est éliminé par filtration sous argon sur un lit de célite et le filtrat dans une fiole contenant 50ml d'isopropanol chlorhydrique 6N préalablement refroidi.
On observe alors la précipitation d'un solide que l'on essore sur fritté n°3 et que l'on lave avec 2x15ml d'éthanol et 2x50 ml d'éther isopropylique. Après séchage sous vide, en présence de P₂O₅ et de pastilles de soude, nous récupérons 3,8g d'une poudre gris bleu correspondant au composé attendu.

Les analyses RMN (¹H 400 MHz et ¹³C 100,61 MHz DMSO d₆) sont conformes à la structure attendue.
Le cation attendu [C₁₄H₂₄N₅]+ est détecté à m/z,ESP+=262 et 131 (correspondant respectivement à [M]+ et [M+H]2+).

### EXEMPLE 12

### Synthèse du N-[3-(1H-imidazol-1-yl)propyl]-3-nitropyrazolo[1,5-a]pyridin-2-amine

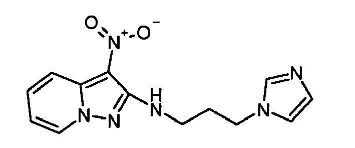

Dans un tricol de 100 ml, surmonté d'un réfrigérant à boules, d'un thermomètre et d'une agitation magnétique, on introduit 50 ml de NMP, 5g (0,021 mole) de 2-méthanesulfanyl-3-nitro-pyrazol[1,5-a]pyridine et 26g de 3-(1H-imidazol-1-yl)propan-1-amine et on maintient l'agitation pendant 6 heures en suivant par CCM (éluant : 95:5 Dichlorométhane / Méthanol).
Le milieu est versé sur un mélange de 200g de glace et d'eau. Le composé jaune qui a cristallisé est essoré sur fritté n°3, lavé à l'eau 2x100ml puis avec 3x100ml d'éther isopropylique. Après séchage à 35°C sous vide, en présence de P₂O₅, pendant 12 heures, on récupère 5g de solide vert correspondant au composé attendu.

Les analyses RMN (¹H 400 MHz et ¹³C 100,61 MHz DMSO d₆) et spectrophotométrie de masse sont conformes à la structure attendue.
Les ions quasi moléculaires [M+H]+, [2M+H]+ de la molécule attendue C₁₃H₁₄N₆O₂ sont principalement détectés.

### Synthèse du 3-méthyl-1-{3-[(3-nitropyrazolo[1,5-a]pyridin-2-yl)amino]propyl}-1H-imidazol-3-ium méthyl sulfate

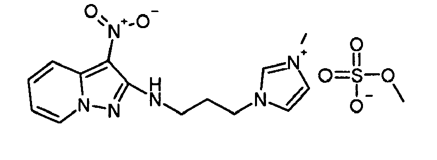

Dans un tricol de 100 ml, surmonté d'un réfrigérant à boules, d'un thermomètre et d'une agitation magnétique, on introduit 50 ml de THF, 2,7g (0,0095 mole) de N-[3-(1 H-imidazol-1-yl)propyl]-3-nitropyrazolo[1,5-a]pyridin-2-amine et 1,8 ml (0,019 mole) de diméthylsulfate au goutte à goutte au reflux du THF.
la réaction est suivie par CCM (éluant : 95:5 Dichlorométhane / Méthanol).

Le milieu est ramené à la température ambiante et le solide jaune formé est essoré sur fritté n°3, lavé avec 2x100ml de THF puis avec 3x100ml d'éther isopropylique. Après séchage à 35°C sous vide, en présence de P₂O₅, pendant 12 heures, on récupère 3,91 g de solide jaune correspondant à la structure attendue.

Les analyses RMN (¹H 400 MHz et ¹³C 100,61 MHz DMSO d₆) et spectrométrie de masse sont conformes .

### Synthèse du dichlorhydrate de chlorure de 1-{3-[(3-aminopyrazolo[1,5-a]pyridin-2-yl)amino]propyl}-3-méthyl-1H-imidazol-3-ium

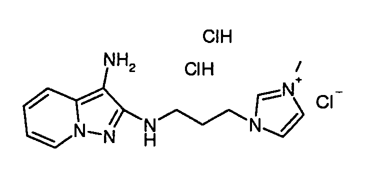

Dans un tricol de 250 ml, surmonté d'un réfrigérant à boules, d'un thermomètre, d'une ampoule de coulée et d'une agitation magnétique, on introduit 80 ml de propanol-2 et 6g de poudre de zinc et on porte au reflux.
On ajoute alors au goutte à goutte 1 ml d'acide chlorhydrique 10 N suivi par portion de 2,5g de 3-méthyl-1-{3-[(3-nitropyrazolo[1,5-a]pyridin-2-yl)amino]propyl}-1H-imidazol-3-ium méthyl sulfate en 10 minutes.

En fin d'addition, on ajoute 1 ml d'acide chlorhydrique au goutte à goutte et on maintient 2 heures au reflux.

Lorsque la réduction est terminée, le zinc est éliminé par filtration sous argon sur un lit de célite et le filtrat récupéré dans une fiole contenant 25ml d'isopropanol chlorhydrique 6N préalablement refroidi.

Cette solution est concentrée, puis en refroidissant à la glace, un solide bleu clair cristallise.
Ce solide bleu clair formé est essoré sur fritté n°3, lavé avec de 2x60ml d' éther isopropylique et après séchage sous vide en présence de P₂O₅ et de pastilles de soude, nous récupérons 1,8g de solide bleu clair correspondant au composé attendu.

Les analyses RMN (¹H 400 MHz et ¹³C 100,61 MHz DMSO d₆) et spectrophotométrie de masse sont conformes à la structure attendue.

Les analyses RMN (¹H 400 MHz et ¹³C 100,61 MHz DMSO d₆) et spectrophotométrie de masse sont conformes à la structure attendue.
Le cation attendu C₁₄H₁₉N₆+ est principalement détecté à m/z=271.

### EXEMPLE 13 Synthèse du N,N-diméthyl-N'-(3-nitropyrazolo[1,5-a]pyridin-2-yl)propane-1,3-diamine

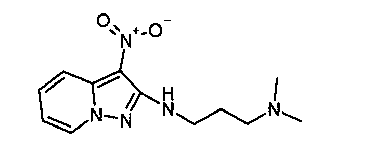

Dans un tricol de 100 ml, surmonté d'un réfrigérant à boules, d'un thermomètre et d'une agitation magnétique, on introduit 5 ml de NMP, 3g de 2-(méthylsulfonyl)-3-nitropyrazolo[1,5-a]pyridine et 3,78 ml de N,N-diméthyl-1,3-propanediamine et on porte à 80°C pendant une heure.

Le milieu est versé sur un mélange de 200g de glace et d'eau. Le composé jaune qui a cristallisé est essoré sur fritté n°3, lavé à l'eau 2x100ml puis avec 3x100ml d'éther isopropylique. Après séchage à 35 °C sous vide, en présence de P₂O₅, pendant 12 heures, on récupère 2,69g de solide vert correspondant au composé attendu.

Les analyses RMN (¹H 400 MHz et ¹³C 100,61 MHz DMSO d₆) et spectrophotométrie de masse sont conformes à la structure attendue.

### Synthèse du N,N,N-triméthyl-3-[(3-nitropyrazolo[1,5-a]pyridin-2-yl)amino]propan-1-aminium méthyl sulfate

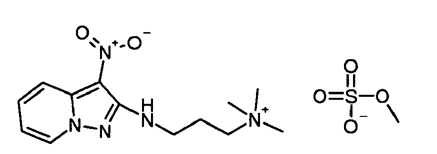

Dans un tricol de 100 ml, surmonté d'un réfrigérant à boules, d'un thermomètre et d'une agitation magnétique, on introduit 25 ml de THF, 2,63g (0,01 mole) de N,N-diméthyl-N'-(3-nitropyrazolo[1,5-a]pyridin-2-yl)propane-1,3-diamine et 1,85 ml (0,02 mole) de diméthylsulfate au goutte à goutte à 50°C.
La réaction est suivie par CCM (éluant : 95:5 Dichlorométhane / Méthanol).

Le milieu est ramené à la température ambiante et le solide jaune formé est essoré sur fritté n°3, lavé avec 2x100m de THF puis avec 3x100ml d'éther isopropylique. Après séchage à 35°C sous vide, en présence de P₂O₅, pendant 12 heures, on récupère 3,84g de solide jaune correspondant à la structure attendue.

Les analyses RMN (¹H 400 MHz et ¹³C 100,61 MHz DMSO d₆) et spectrométrie de masse sont conformes.

### Synthèse du dichlohydrate de chlorure de 3-[(3-aminopyrazolo[1,5-a]pyridin-2-yl)amino]-N,N,N-triméthylpropan-1-aminium

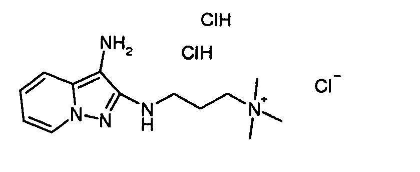

Dans un tricol de 250 ml, surmonté d'un réfrigérant à boules, d'un thermomètre , d'une ampoule de coulée et d'une agitation magnétique, on introduit 190 ml d'éthanol et 9g de poudre de zinc et on porte au reflux .
On ajoute alors au goutte à goutte 2 ml d'acide chlorhydrique 10 N puis au goutte à goutte une solution de 3g de N,N,N-triméthyl-3-[(3-nitropyrazolo[1,5-a]pyridin-2-yl)amino]propan-1-aminium méthyl sulfate et 5ml d'eau.

En fin d'addition , on maintient 45 minutes au reflux.

Lorsque la réduction est terminée, le zinc est éliminé par filtration sous argon sur un lit de célite et le filtrat récupéré dans une fiole contenant 100ml d'isopropanol chlorhydrique 6N préalablement refroidi.

Le solide bleu très clair formé est essoré sur fritté n°3, lavé avec de 2x60ml d' éther isopropylique et après séchage sous vide en présence de P₂O₅ et de pastilles de soude, nous récupérons 2,37g de solide bleu clair correspondant au composé attendu.

Les analyses RMN (¹H 400 MHz et ¹³C 100,61 MHz DMSO d₆) et spectrophotométrie de masse sont conformes à la structure attendue.

Les analyses RMN (¹H 400 MHz et ¹³C 100,61 MHz DMSO d₆) et spectrophotométrie de masse sont conformes à la structure attendue.
Les ions [C₁₃H₂₂N₅]+, [M+H]2+ ainsi que l'ion fragment [C₁₀H₁₂N₄]+ relatif au cation attendu sont principalement détectés.

### EXEMPLE 14

### Synthèse du N,N-dimethyl-1-(3-nitropyrazolo[1,5-a]pyridin-2-yl)pyrrolidin-3-amine

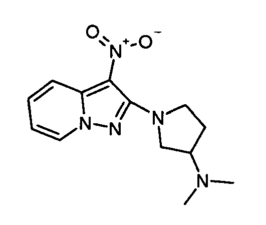

Dans un tricol de 100 ml, surmonté d'un réfrigérant à boules, d'un thermomètre et d'une agitation magnétique, on introduit 5 ml de NMP, 3g de 2-(méthylsulfonyl)-3-nitropyrazolo[1,5-a]pyridine et 3,77 ml de 3(dimethyamino)pyrrolidine et on porte à 80°C pendant une heure.

Le milieu est versé sur un mélange de 200g de glace et d'eau. Le composé jaune qui a cristallisé est essoré sur fritté n°3, lavé à l'eau 2x100ml puis avec 3x100ml d'éther isopropylique. Après séchage à 35 °C sous vide, en présence de P₂O_{5,} pendant 12 heures, on récupère 3,26g de solide jaune correspondant au composé attendu.

Les analyses RMN (¹H 400 MHz et ¹³C 100,61 MHz DMSO d₆) et spectrophotométrie de masse sont conformes à la structure attendue.
L'ion quasi-moléculaire (MH)+ de la molécule attendue, C₁₃H₁₇N₅O₂, est principalement détecté

### Synthèse du N,N,N-trimethyl-1-(3-nitropyrazolo[1,5-a]pyridin-2-yl)pyrrolidin-3-aminium methyl sulfate

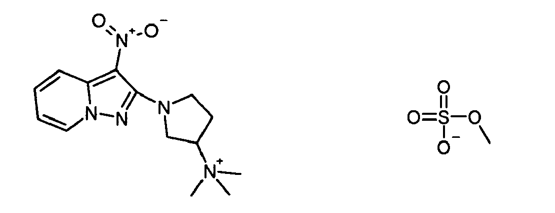

Dans un tricol de 100 ml, surmonté d'un réfrigérant à boules, d'un thermomètre et d'une agitation magnétique, on introduit 25 ml de THF, 3,26g ( 0,01 mole) de N,N-dimethyl-1-(3-nitropyrazolo[1,5-a]pyridin-2-yl)pyrrolidin-3-amine et 1,89 ml (0,02 mole) de diméthylsulfate au goutte à goutte à 50°C.
La réaction est suivie par CCM (éluant : 95:5 Dichlorométhane / Méthanol).

Le milieu est ramené à la température ambiante et le solide jaune formé est essoré sur fritté n°3, lavé avec 2x100ml de THF puis avec 3x100ml d'éther isopropylique. Après séchage à 35°C sous vide, en présence de P₂O₅, pendant 12 heures, on récupère 3,2g de solide jaune correspondant à la structure attendue.

Les analyses RMN (¹H 400 MHz et ¹³C 100,61 MHz DMSO d₆) et spectrométrie de masse sont conformes.

### Synthèse du dichlohydrate de chlorure de 1-(3-aminopyrazolo[1,5-a]pyridin-2-yl)-N,N,N-trimethylpyrrolidin-3-aminium

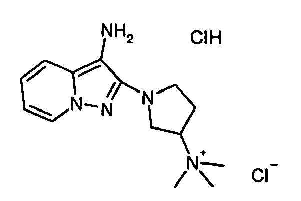

Dans un tricol de 250 ml, surmonté d'un réfrigérant à boules, d'un thermomètre, d'une ampoule de coulée et d'une agitation magnétique, on introduit 190 ml d'éthanol et 9g de poudre de zinc et on porte au reflux.
On ajoute alors au goutte à goutte 2 ml d'acide chlorhydrique 10 N puis au goutte à goutte une solution de 3g de N,N,N-triméthyl-3-[(3-nitropyrazolo[1,5-a]pyridin-2-yl)amino]propan-1-aminium méthyl sulfate et 5ml d'eau.

En fin d'addition, on maintient 45 minutes au reflux.

Lorsque la réduction est terminée, le zinc est éliminé par filtration sous argon sur un lit de célite et le filtrat récupéré dans une fiole contenant 100ml d'isopropanol chlorhydrique 6N préalablement refroidi.

Le solide beige formé est essoré sur fritté n°3, lavé avec de 2x60ml d'éther isopropylique et après séchage sous vide en présence de P₂O₅ et de pastilles de soude, nous récupérons 2,83g de solide crème correspondant au composé attendu.

Les analyses RMN (¹H 400 MHz et ¹³C 100,61 MHz DMSO d₆) et spectrophotométrie de masse sont conformes à la structure attendue.

Les analyses RMN (¹H 400 MHz et ¹³C 100,61 MHz DMSO d₆) et spectrophotométrie de masse sont conformes à la structure attendue.
Les ions [C₁₄H₂₂N₅]+, [M+H]₂+ ainsi que l'ion fragment [C₁₁H₁₃N₄]+ relatif au cation attendu sont principalement détectés..

### EXEMPLE 15

### Synthèse du 2-(4-methylpiperazin-1-yl)-3-nitropyrazolo[1,5-a]pyridine

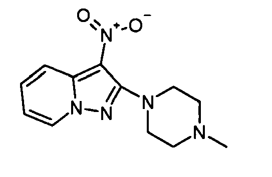

Dans un tricol de 100 ml, surmonté d'un réfrigérant à boules, d'un thermomètre et d'une agitation magnétique, on introduit 5 ml de NMP, 3g de 2-(méthylsulfonyl)-3-nitropyrazolo[1,5-a]pyridine et 3,3 ml de 1-méthylpiperazine et on porte à 80 °C pendant 3 heures.

Le milieu est versé sur un mélange de 200g de glace et d'eau. Le composé jaune qui a cristallisé est essoré sur fritté n°3, lavé à l'eau 2x100ml puis avec 3x100ml d'éther isopropylique. Après séchage à 35°C sous vide, en présence de P₂O₅, pendant 12 heures, on récupère 2,35g de solide jaune correspondant au composé attendu.

Les analyses RMN (¹H 400 MHz et ¹³C 100,61 MHz DMSO d₆) et spectrophotométrie de masse sont conformes à la structure attendue.

¹H NMR (DMSO-d6): 2.23(s, 3H), 2.5(m,4H), 3.41 (m, 4H), 7.31 (m, 1 H), 7.82 (m, 1 H), 8.19 (m, 1 H), 8.82(m, 1 H)

Les ions quasi moléculaires [M+H]+, [M+H+CH₃CN]+, [2M+H]+ ainsi que l'ion de la molécule attendue C₁₂H₁₅N₅O₂ sont principalement détectés

### Synthèse du 1,1-dimethyl-4-(3-nitropyrazolo[1,5-a]pyridin-2-yl)piperazin-1-ium methyl sulfate

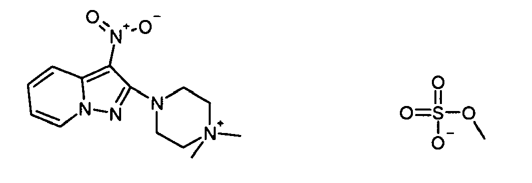

Dans un tricol de 100 ml, surmonté d'un réfrigérant à boules, d'un thermomètre et d'une agitation magnétique, on introduit 25 ml de THF, 2,35g ( 0,01 mole) de 2-(4-methylpiperazin-1-yl)-3-nitropyrazolo[1,5-a]pyridine et 1,85 ml (0,02 mole) de diméthylsulfate au goutte à goutte à 50°C.
La réaction est suivie par CCM (éluant : 95:5 Dichlorométhane / Méthanol).

Le milieu est ramené à la température ambiante et le solide jaune formé est essoré sur fritté n°3, lavé avec 2x100ml de THF puis avec 3x100ml d'éther isopropylique. Après séchage à 35°C sous vide, en présence de P₂O₅, pendant 12 heures, on récupère 3,2g de solide jaune correspondant à la structure attendue.

Les analyses RMN (¹H 400 MHz et ¹³C 100,61 MHz DMSO d₆) et spectrométrie de masse sont conformes.

### Synthèse du dichlohydrate de chlorure de 4-(3-aminopyrazolo[1,5-a]pyridin-2-yl)-1,1-diméthylpiperazin-1-ium

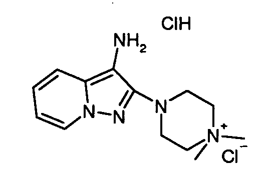

Dans un tricol de 250 ml, surmonté d'un réfrigérant à boules, d'un thermomètre, d'une ampoule de coulée et d'une agitation magnétique, on introduit 190 ml d'éthanol et 9g de poudre de zinc et on porte au reflux.
On ajoute alors au goutte à goutte 2 ml d'acide chlorhydrique 10 N puis au goutte à goutte une solution de 3g de 1,1-dimethyl-4-(3-nitropyrazolo[1,5-a]pyridin-2-yl)piperazin-1-ium méthyl sulfate et 5ml d'eau.

En fin d'addition, on maintient 45 minutes au reflux.

Lorsque la réduction est terminée, le zinc est éliminé par filtration sous argon sur un lit de célite et le filtrat récupéré dans une fiole contenant 100ml d'isopropanol chlorhydrique 6N préalablement refroidi.

Le solide beige formé est essoré sur fritté n°3, lavé avec de 2x60ml d'éther isopropylique et après séchage sous vide en présence de P₂O₅ et de pastilles de soude, nous récupérons 3.31g de solide crème correspondant au composé attendu.

Les analyses RMN (¹H 400 MHz et ¹³C 100,61 MHz DMSO d₆) et spectrophotométrie de masse sont conformes à la structure attendue.

Le cation attendu [C₁₃H₂₀N₅]+ est principalement détecté à m/z,ESP+=246.

### Exemple 16

### Synthèse du chlorhydrate de chlorure de 1-{2-[(3-aminopyrazolo[1,5-a]pyridin-2-yl)oxy]ethyl}-1-methylpiperidinium

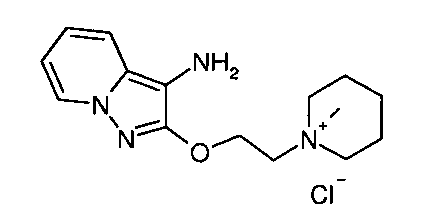

### Synthèse générale

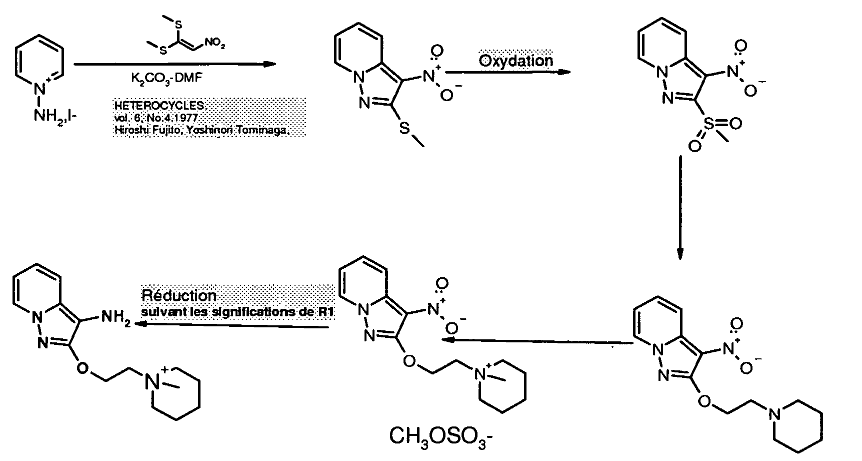

### Synthèse du 3-nitro-2-(2-piperidin-1-ylethoxy)pyrazolo[1,5-a]pyridine

Dans un tricol de 250 ml, surmonté d'un réfrigérant à boules, d'un thermomètre et d'une agitation magnétique, on introduit 10ml d'eau et 13.8 ml(0.103mole) de N-(2-hydroxyethyl)pipéridine que l'on refroidit à zéro degrés puis une solution issue de 8.29 g(0.207mole) de soude et 20 ml d'eau est ajoutée en 15 minutes.
Cette solution est agitée à cette même température pendant 15 minutes puis une solution issue de 100ml de N-methylpyrrolidinone et 10g (0,04145 mole) 2-(methylsulfonyl)-3-nitropyrazolo[1,5-a]pyridine est ajoutée au goutte à goutte en 20 minutes.
Le milieu violet hétérogène obtenu est agité pendant 4 heures puis à température ambiante. Le solide formé est essoré lavé à l'eau jusqu'à pH neutre puis avec 4x50ml d'éther isopropylique. Après séchage à 35°C sous vide, en présence de P2O5, pendant 12 heures, on récupère 3.70g de solide beige correspondant au composé attendu.

Les analyses RMN (1 H 400 MHz et 13C 100,61 MHz DMSO d6) et spectrophotométrie de masse sont conformes à la structure attendue.

Les ions quasi moléculaires [M+H]+, [M+Na]+, [M+Na+CH3OH]+, [2M+Na]+ sont principalement détectés

### 1- methyl-1-{2-[(3-nitropyrazolo[1,5-a]pyridin-2-yl)oxy]ethyl}piperidinium methyl sulfate

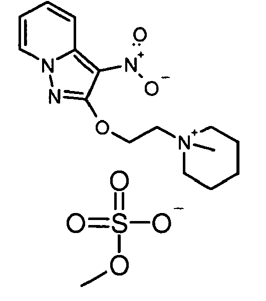

Dans un tricol de 100 ml, surmonté d'un réfrigérant à boules, d'un thermomètre et d'une agitation magnétique, on introduit 60 ml de THF, 6g (0,0206 mole) de 3-nitro-2-(2-piperidin-1-ylethoxy)pyrazolo[1,5-a]pyridine et on ajoute goutte à goutte 3,9 ml (0,0413mole) de diméthylsulfate au goutte à goutte à 20°C. La réaction est suivie par CCM (éluant : 95:5 Dichlorométhane / Méthanol).
Le solide formé pendant la nuit est essoré sur fritté n°3, lavé avec 3x100m de THF puis avec 3x100ml d'éther isopropylique. Après séchage à 35°C sous vide, en présence de P2O5, pendant 12 heures, on récupère 8,09g de solide beige correspondant à la structure attendue.

Les analyses RMN (1H 400 MHz et 13C 100,61 MHz DMSO d6) et spectrométrie de masse sont conformes.

### Synthèse du chlorhydrate de 2-(2-piperidin-1-ylethoxy)pyrazolo[1,5-a]pyridin-3-amine

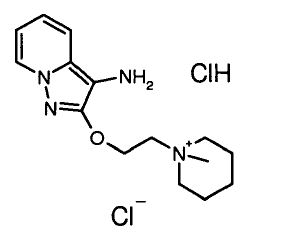

Dans un tricol de 100 ml, surmonté d'un réfrigérant à boules, d'un thermomètre et d'une agitation magnétique, on introduit 50 ml d'éthanol et 5g de poudre de zinc et on porte au reflux.
On ajoute alors au goutte à goutte 550 micro litre d'acide acétique puis la solution issue de 5 ml d'eau et5g de methyl-1-{2-[(3-nitropyrazolo[1,5-a]pyridin-2-yl)oxy]ethyl}piperidinium methyl sulfate. En fin de coulée, on laisse au reflux pendant 18 heures.
Lorsque la réduction est terminée, le zinc est éliminé par filtration sous argon sur un litde célite et le filtrat est recueilli dans une fiole contenant 120ml d'isopropanol chlorhydrique 6N préalablement refroidi.

Le filtrat est évaporé puis repris plusieurs fois avec 60 ml d'isopropanol, On observe alors la cristallisation lente d'un solide violet que l'on essore sur fritté n°3 et que l'on lave avec 3x30 ml d'éther. Après séchage sous vide, en présence de P2O5 et de pastilles de soude, nous récupérons 3,5g d'une poudre rose pale correspondant au composé attendu.
Les analyses RMN (1 H 400 MHz et 13C 100,61 MHz DMSO d6) et spectrophotométrie de masse sont conformes à la structure attendue.
Les ions quasi moléculaires [M+H]+, [M+Na]+, [M+H+CH3OH] de la molécule attendue C14H20N4O sont détectés.

### Exemple 17

### Chlorhydrate de chlorure de 2-[(3-aminopyrazolo[1,5-a]pyridin-2-yl)oxy] N,N,Ntrimethylethanaminium

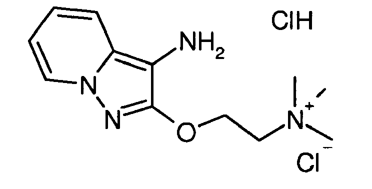

### Synthèse générale

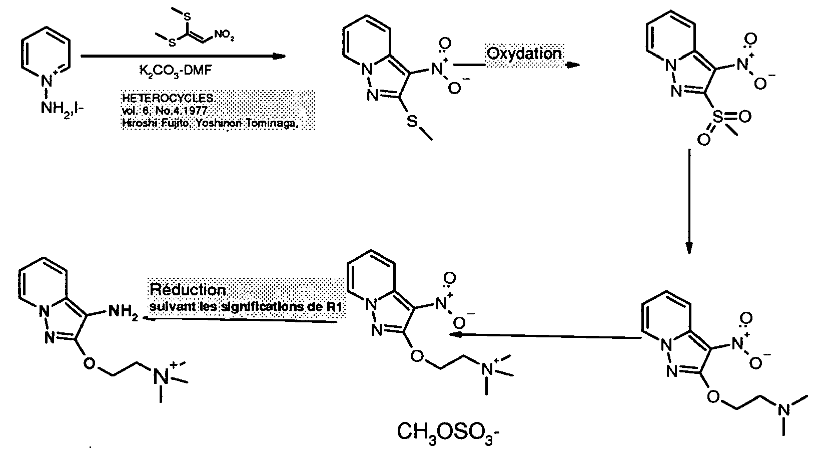

### Synthèse du N,N-dimethyl-2-[(3-nitropyrazolo[1,5-a]pyridin-2-yl)oxy]ethanamine

Dans un tricol de 250 ml, surmonté d'un réfrigérant à boules, d'un thermomètre et d'une agitation magnétique, on introduit 10ml d'eau et 10,3ml(0.103mole) de N, N-dimethylethanolamine que l'on refroidit à zéro degrés puis une solution issue de 8.29 g(0.207mole) de soude et 20 ml d'eau est ajoutée en 15 minutes.
Cette solution est agitée à cette même température pendant 15 minutes puis une solution issue de 100ml de N-methylpyrrolidinone et 10g (0,04145 mole) 2-(methylsulfonyl)-3-nitropyrazolo[1,5-a]pyridine est ajoutée au goutte à goutte en 20 minutes.

Le milieu est légèrement violet obtenu est agité pendant 6 heures puis à température ambiante. Le milieu réactionnel est versé sur 500 ml d'eau et le solide beige formé est essoré lavé à l'eau jusqu'à pH neutre puis avec 4x50ml d'éther isopropylique. Après séchage à 35°C sous vide, en présence de P2O5, pendant 12 heures, on récupère 5,01 g de solide beige correspondant au composé attendu.

Les analyses RMN (1 H 400 MHz et 13C 100,61 MHz DMSO d6) et spectrophotométrie de masse sont conformes à la structure attendue.

Les ions quasi moléculaires [M+H]+, [M+Na]+, [M+Na+CH3OH]+, [2M+Na]+ sont principalement détectés

### Synthèse du N,N,N-trimethyl-2-[(3-nitropyrazolo[1,5-a]pyridin-2-yl)oxy]ethanaminium methyl sulfate

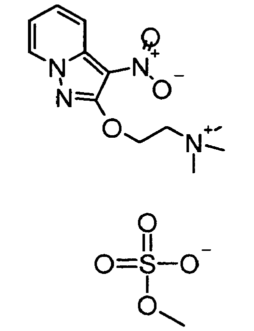

Dans un tricol de 100 ml, surmonté d'un réfrigérant à boules, d'un thermomètre et d'une agitation magnétique, on introduit 30 ml de THF, 3g (0,01198 mole) de N,N-dimethyl-2-[(3-nitropyrazolo[1,5-a]pyridin-2-yl)oxy]ethanamine et on ajoute goutte à goutte 2,27 ml (0,02397mole) de diméthylsulfate au goutte à goutte à 20°C. La réaction est suivie par CCM (éluant : 95:5 Dichlorométhane / Méthanol).
Le solide formé pendant la nuit est essoré sur fritté n°3, lavé avec 2x50m de THF puis avec 2x50ml d'éther isopropylique. Après séchage à 35°C sous vide, en présence de P2O5, pendant 12 heures, on récupère 4,50g de solide blanc correspondant à la structure attendue.

Les analyses RMN (1 H 400 MHz et 13C 100,61 MHz DMSO d6) et spectrométrie de masse sont conformes.

### Synthèse du chlorhydrate de chlorure de 2-[(3-aminopyrazolo[1,5-a]pyridin-2-yl)oxy]-N,N,N-trimethylethanaminium

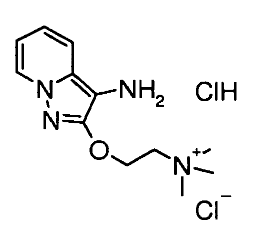

Dans un tricol de 100 ml, surmonté d'un réfrigérant à boules, d'un thermomètre et d'une agitation magnétique, on introduit 40 ml d'éthanol et 4.5g de poudre de zinc et on porte au reflux. On ajoute alors deux gouttes d'acide acétique puis la solution issue de 4,5 ml d'eau et 4,5g N,N,N-trimethyl-2-[(3-nitropyrazolo[1,5-a]pyridin-2-yl)oxy]ethanaminium methyl sulfate. En fin d'ajout, on ajoute 2 gouttes d'acide acétique et le reflux est maintenu 18 heures.
Lorsque la réduction est terminée, le zinc est éliminé par filtration sous argon sur un lit de célite et le filtrat est recueilli dans une fiole contenant 100ml d'isopropanol chlorhydrique 6N préalablement refroidi.
Le filtrat est concentré au 1/3 puis repris plusieurs fois à l'éther, On observe alors la cristallisation lente d'un solide violet que l'on essore sur fritté n°3 et que l'on lave avec le minimum d'isopropanol puis avec 3x30 ml d'éther . Après séchage sous vide, en présence de P2O5 et de pastilles de soude, nous récupérons 2,85g d'une poudre violet pale correspondant au composé attendu.
Les analyses RMN (1 H 400 MHz et 13C 100,61 MHz DMSO d6) et spectrophotométrie de masse sont conformes à la structure attendue.
Les ions quasi moléculaires [M+H]+, [M+Na]+, [M+H+CH3OH] de la molécule attendue C11H16N4O sont détectés.

### Exemple 18

### Chlorhydrate de chlorure de 1-{2-[(3-aminopyrazolo[1,5-a]pyridin-2-yl)amino]ethyl}-1,2,6-trimethylpiperidinium

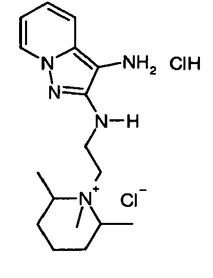

### Synthèse du N-[2-(2,6-dimethylpiperidin-1-yl)ethyl]-3-nitropyrazolo[1,5-a]pyridin-2-amine.

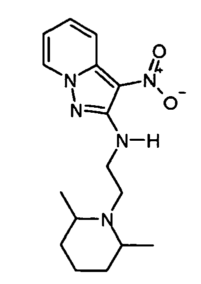

Dans un tricol de 30 mL, surmonté d'un réfrigérant à boules, d'un thermomètre et d'une agitation magnétique, on introduit 10 ml de NMP, 5g de g (0,0207 moles) 2-methanesulfanyl-3-nitro-pyrazol[1,5-a]pyridine et 5g (0,032mol) de 3-aminopropyldimethylpiperidin-1-yl)ethanamine . Sous agitation, on porte à 70°C à l'aide d'un bain d'huile, pendant 4 heures en suivant par CCM (éluant : 98:2 Dichlorométhane / Méthanol).
Refroidir le milieu à la température ambiante, le composé est précipité sur 150 g de glace, le composé jaune formé est essoré sur fritté n °4 puis lavé à l'eau plusieurs fois, Après séchage à 40°C sous vide, en présence de P₂O₅ , on récupère 3, 49g de solide jaune.

Les analyses RMN (¹H 400 MHz et ¹³C 100,61 MHz DMSO d₆) sont conformes à la structure attendue.

La masse du composé attendu C₁₆H₂₃N₅O₂ est détectée en spectrométrie de masse.

### Synthèsedu1,2,6-trimethyl-1-{2-[(3-nitropyrazolo[1,5-a]pyridin-2-yl)amino]ethyl}piperidinium methyl sulfate

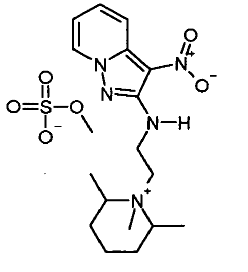

Dans un tricol de 30ml, équipé d'un réfrigérant à boules, d'un thermomètre et d'une ampoule de coulée de 50 mL, sous agitation magnétique, dans 15 mL de THF, on introduit 3,25g ( 0,0107 moles) N-[2-(2,6-dimethylpiperidin-1-yl)ethyl]-3-nitropyrazolo[1,5-a]pyridin-2-amine. On ajoute goutte à goutte 2.13 ml (0,02250mole) de dimethylsulfate; il se forme un précipité jaune. La réaction est suivie par CCM (éluant : dichlorométhane / méthanol 98 :2), jusqu'à la disparition du produit de départ pendant 24 heures. Le solide formé est essoré puis lavé plusieurs fois au THF. Apres séchage sous vide, en présence de P₂O₅, on récupère 4,37g de poudre jaune. Les analyses RMN (¹H 400 MHz et ¹³C 100,61 MHz DMSO d₆) sont conformes à la structure attendue.
L'ion moléculaire M+ attendue [C17H25N5O]+ est principalement détecté ainsi que son contre-ion [CH3O4S]-

### Synthèse du chlorhydrate de chlorure de 1-{2-[(3-aminopyrazolo[1,5-a]pyridin-2-yl)amino]ethyl}-1,2,6-trimethylpiperidinium

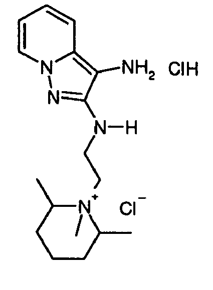

Dans un tricol de 250ml L, surmonté d'un réfrigérant, d'un thermomètre et d'une agitation magnétique, on introduit 100m l d'éthanol et 3,75g de zinc. Ce milieu est porte au reflux et 5 gouttes d'acide acétiques sont ajoutées. Une solution issue de 3,75g de 1,2,6-trimethyl-1-{2-[(3-nitropyrazolo[1,5-a]pyridin-2yl)amino]ethyl}piperidinium methyl sulfate, de 20 ml d'éthanol et 4 ml d'eau est ajoutée goutte à goutte en 30 minutes et le reflux est maintenu pendant 24 heures.

Lorsque la réduction est terminée, éliminer le zinc par filtration sur célite et récupérer les jus dans 70 ml l'isopropanol chlorhydrique préalablement refroidi par un bain froid de carboglace. Laver le zinc avec de l'éthanol. Les jus sont concentrés au 1/3 et à zéro degrés la cristallisation est amorcée..
Le solide gris bleu est essoré sur fritté n °3, après séchage sous vide, en présence de P₂O₅. On récupère ainsi 1,09g d'une poudre gris bleu.

Les analyses RMN (¹H 400 MHz et ¹³C 100,61 MHz DMSO d₆) sont conformes à la structure attendue.
L'ion moléculaire M+ attendue [C17H28N5]+ est principalement détecté .

### Exemple 19

### Chlorhydrate de chlorure de 1-{3-[(3-aminopyrazolo[1,5-a]pyridin-2-yl)amino]propyl}-1-methylpiperidinium

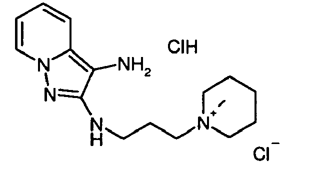

### Synthèse du 3-nitro-N-(3-piperidin-1-ylpropyl)pyrazolo[1,5-a]pyridin-2-amine

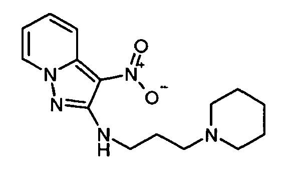

Dans un tricol de 30 mL, surmonté d'un réfrigérant à boules, d'un thermomètre et d'une agitation magnétique, on introduit 10 ml de NMP, 4.5g de g (0,0159 moles) 2-methanesulfanyl-3-nitro-pyrazol[1,5-a]pyridine et 5g (0,032mol) de 2-(2,6-dimethylpiperidin-1-yl)ethanamine . Sous agitation, on porte à 70°C à l'aide d'un bain d'huile, pendant 4 heures en suivant par CCM (éluant : 98:2 Dichlorométhane /Méthanol).
Refroidir le milieu à la température ambiante, le composé est précipité sur 150 g de glace, le composé jaune formé est esssoré sur fritté n °4 puis lavé à l'eau plusieurs fois, Après séchage à 40°C sous vide, en présence de P₂O₅, on récupère 3, 49g de solide jaune.

Les analyses RMN (¹H 400 MHz et ¹³C 100,61 MHz DMSO d₆) sont conformes à la structure attendue.

La masse du composé attendu C₁₆H₂₃N₅O₂ est détectée en spectrométrie de masse.

### Synthèsedu1-methyl-1-{3-[(3-nitropyrazolo[1,5-a]pyridin-2-yl)amino]propyl}piperidinium methyl sulfate

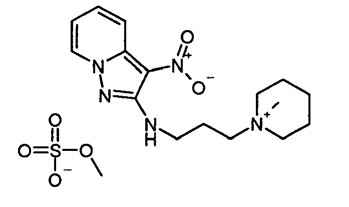

Dans un tricol de 30ml, équipé d'un réfrigérant à boules, d'un thermomètre et d'une ampoule de coulée de 50 mL, sous agitation magnétique, dans 15 mL de THF, on introduit 3,25g ( 0,0107 moles) de 3-nitro-N-(3-piperidin-1-ylpropyl)pyrazolo[1,5-a]pyridin-2-amine. On ajoute goutte à goutte 2.13 ml (0,0225mole) de dimethylsulfate; il se forme un précipité jaune. La réaction est suivie par CCM (éluant : dichlorométhane / méthanol 98 :2), jusqu'à la disparition du produit de départ pendant 24 heures. Le solide formé est essoré puis lavé plusieurs fois au THF. Apres séchage sous vide, en présence de P₂O₅, on récupère 4,37g de poudre jaune.

Les analyses RMN (¹H 400 MHz et ¹³C 100,61 MHz DMSO d₆) sont conformes à la structure attendue.
L'ion moléculaire M+ attendue [C17H24N5O2]+ est principalement détecté ainsi que son contre-ion [CH3O4S]

### Synthèse du chlorhydrate de chlorure de 1-{3-[(3-aminopyrazolo[1,5-a]pyridin-2-yl)amino]propyl}-1-methylpiperidinium

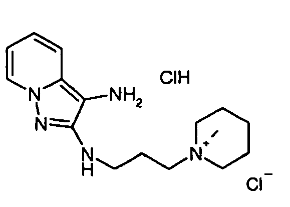

Dans un tricol de 250ml L, surmonté d'un réfrigérant, d'un thermomètre et d'une agitation magnétique, on introduit 100ml d' éthanol et 4,37g de zinc. Ce milieu est porte au reflux et 5 gouttes d'acide acétiques sont ajoutées. Une solution issue de 4,37g de 1-methyl-1-{3-[(3-nitropyrazolo[1,5-a]pyridin-2-yl)amino]propyl}piperidiniummethyl sulfate, de 15 ml d'éthanol et 5 ml d'eau est ajoutée goutte à goutte en 30 minutes et le reflux est maintenu pendant 24 heures.

Lorsque la réduction est terminée, éliminer le zinc par filtration sur célite et récupérer les jus dans 70 ml l'isopropanol chlorhydrique préalablement refroidi par un bain froid de carboglace. Laver le zinc avec de l'éthanol. Les jus sont concentrés au 1/3 et à zéro degrés la cristallisation est amorcée..
Le solide bleu clair est essoré sur fritté n°3, après séchage sous vide, en présence de P₂O₅. On récupère ainsi 3,41g d'une poudre bleu clair.

Les analyses RMN (¹H 400 MHz et ¹³C 100,61 MHz DMSO d₆) sont conformes à la structure attendue.
L'ion moléculaire M+ attendue [C16H26N5]+ est principalement détecté.

### Exemple 20

### Chlorhydrate de chlorure de 1-{4-[(3-aminopyrazolo[1,5-a]pyridin-2-yl)amino]butyl}-1-methylpiperidinium

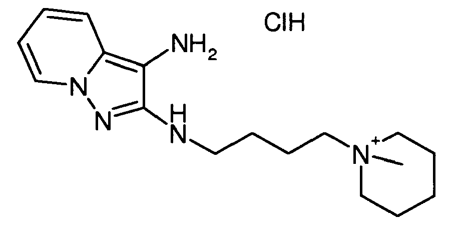

### Synthèse générale

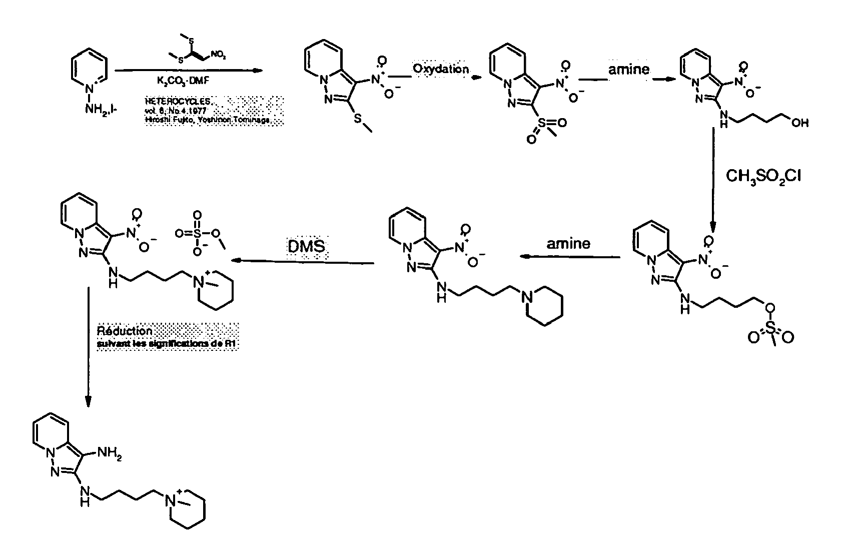

### Synthèse du 4-[(3-nitropyrazolo[1,5-a]pyridin-2-yl)amino]butan-1-ol

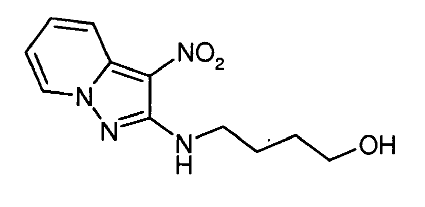

Dans un tricol de 30 mL, surmonté d'un réfrigérant à boules, d'un thermomètre et d'une agitation magnétique, on introduit 10 ml de NMP, 5g de g (0,0207 moles) 2-methanesulfanyl-3-nitro-pyrazol[1,5-a]pyridine et 5g (0,032mol) de 4-amino-1-butanol . Sous agitation, on porte à 70°C à l'aide d'un bain d'huile, pendant 4 heures en suivant par CCM (éluant : 98:2 Dichlorométhane / Méthanol).
Refroidir le milieu à la température ambiante, le composé est précipité sur 150 g de glace, le composé jaune formé est essoré sur fritté n °4 puis lavé à l'eau plusieurs fois, Après séchage à 40°C sous vide, en présence de P₂O₅, on récupère 4, 4g de solide jaune.

Les analyses RMN (¹H 400 MHz et ¹³C 100,61 MHz DMSO d₆) sont conformes à la structure attendue.

La masse du composé attendu C₁₁H₁₄N₄O₂ est détectée en spectrométrie de masse.

### Synthèse du 4-[(3-nitropyrazolo[1,5-a]pyridin-2-yl)amino]butyl methanesulfonate

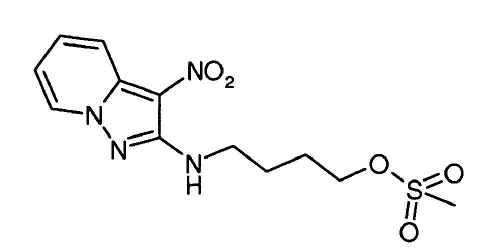

Dans un tricol de 100 mL, surmonté d'un réfrigérant à boules, d'un thermomètre et d'une agitation magnétique, on introduit 40 ml de THF, 3,4g (0,01358 moles) 4-[(3-nitropyrazolo[1,5-a]pyridin-2-yl)amino]butan-1-ol. A zéro degrés, ajouter 5,67 ml(0, 0407 mole) de triethylamine et couler au goutte à goutte 3,27 ml(0,04075 mole) de chlorure de mesyle en 30 minutes. Maintenir l'agitation pendant 30 minutes à zéro degrés puis 2 heures à température ambiante en suivant par CCM (éluant : 98:2 Dichlorométhane / Méthanol).

le solide jaune formé est essoré sur fritté n°4 puis lavé plusieurs fois au THF , 2x 30 ml d'eau et 2x20 ml de THF. Après séchage à 40°C, sous vide, en présence de P₂O₅, on récupère 3, 87g de solide jaune.

Les analyses RMN (¹H 400 MHz et ¹³C 100,61 MHz DMSO d₆) sont conformes à la structure attendue.

La masse du composé attendu C₁₂H₁₆N₄O₅S est détectée en spectrométrie de masse.
Les ions quasi-moléculaires MH+ ; [M+Na] + ; [2M+Na]+ de la molécule attendue :C12H 16N405S
sont principalement détectés.

### Exemple 21

### Chlorhydrate de chlorure de1-{4-[(3-aminopyrazolo[1 ,5-a]pyridin-2-yl)amino]butyl}-1-methylpiperidinium

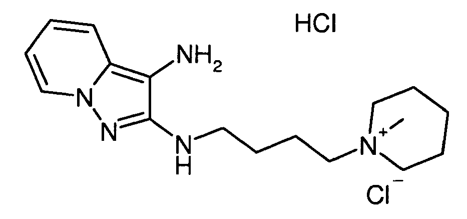

### Synthèse du 3-nitro-N-(4-piperidin-1-ylbutyl)pyrazolo[1,5-a]pyridin-2-amine

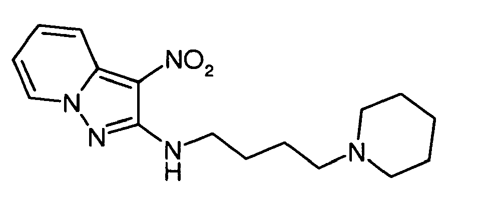

Dans un tricol de 30 mL, surmonté d'un réfrigérant à boules, d'un thermomètre et d'une agitation magnétique, on introduit 20 ml de butanol, 3, 87g de g (0,01178 moles) 4-[(3-nitropyrazolo[1,5-a]pyridin-2-yl)amino]butyl methanesulfonate et 3,09 ml (0,0353mol) de pipéridine. Sous agitation, on porte à 100°C à l'aide d'un bain d'huile, pendant 4 heures en suivant par CCM (éluant : 98:2 Dichlorométhane / Méthanol).
Refroidir le milieu à zéro degrés, le composé cristallise, il est essoré sur fritté n°4 puis lavé à l' isopropanol plusieurs fois, Après séchage à 40°C sous vide, en présence de P₂O₅, on récupère 2,21 g de solide jaune.

Les analyses RMN (¹H 400 MHz et ¹³C 100,61 MHz DMSO d₆) sont conformes à la structure attendue.

Les ions quasi-moléculaires MH+;[M+Na]+,[M+K+CH3COOH]+,[2M+H]+ de la molécule attendue : C16H23N5O2 sont principalement détectés

### Synthèse du1-methyl-1-{4-[(3-nitropyrazolo[1,5-a]pyridin-2-yl)amino]butyl}piperidiniummethylsulfate

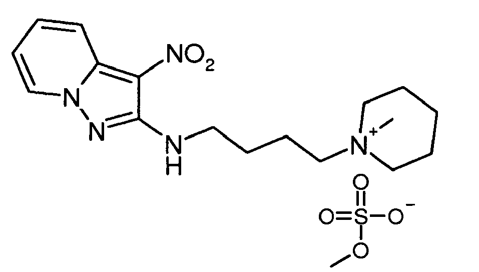

Dans un tricol de 30ml, équipé d'un réfrigérant à boules, d'un thermomètre et d'une ampoule de coulée de 50 mL, sous agitation magnétique, dans 15 mL de THF, on introduit 2,21g ( 0,00696 moles) de 3-nitro-N-(4-piperidin-1-ylbutyl)pyrazolo[1,5-a]pyridin-2-amine. On ajoute goutte à goutte 1, 38 ml (0,0146mole) de dimethylsulfate; il se forme un précipité jaune. La réaction est suivie par CCM (éluant : dichlorométhane / méthanol 98 :2), jusqu'à la disparition du produit de départ pendant 24 heures. Le solide formé est essoré puis lavé plusieurs fois au THF. Apres séchage sous vide, en présence de P₂O₅, on récupère 2,80g de poudre jaune.

Les analyses RMN (¹H 400 MHz et ¹³C 100,61 MHz DMSO d₆) sont conformes à la structure attendue.
L'ion moléculaire M+ attendue [C17H26N5O2]+ est principalement détecté ainsi que son contre-ion [CH3O4S]

### Synthèse du chlorhydrate de chlorure de1-{4-[(3-aminopyrazolo[1,5-a]pyridin-2-yl)amino]butyl}-1-methylpiperidinium

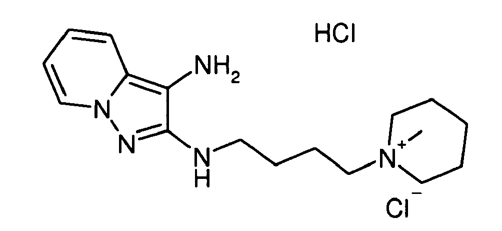

Dans un tricol de 250ml L, surmonté d'un réfrigérant, d'un thermomètre et d'une agitation magnétique, on introduit 70ml d' éthanol et 2,80g de zinc. Ce milieu est porte au reflux et 5 gouttes d'acide acétiques sont ajoutées. Une solution issue de 2,80g de 1-methyl-1-{4-[(3-nitropyrazolo[1,5-a]pyridin-2-yl)amino]butyl}piperidinium methyl sulfate, de 15 ml d'éthanol et 3 ml d'eau est ajoutée goutte à goutte en 30 minutes et le reflux est maintenu pendant 24 heures.

Lorsque la réduction est terminée, éliminer le zinc par filtration sur célite et récupérer les jus dans 70 ml l'isopropanol chlorhydrique préalablement refroidi par un bain froid de carboglace. Laver le zinc avec de l'éthanol. Les jus sont concentrés au 1/3 et à zéro degrés la cristallisation est amorcée.
Le solide bleu clair est essoré sur fritté n°3, après séchage sous vide, en présence de P₂O₅. On récupère ainsi 2,16g d'une poudre bleu clair.

Les analyses RMN (¹H 400 MHz et ¹³C 100,61 MHz DMSO d₆) sont conformes à la structure attendue.
L'ion moléculaire M+ attendue [C17H28N5]+ est principalement détecté.

### Exemple 22

### Chlorhydrate de chlorure de 1-{5-[(3-aminopyrazolo[1,5-a]pyridin-2-yl)amino]pentyl}-1-methylpiperidinium

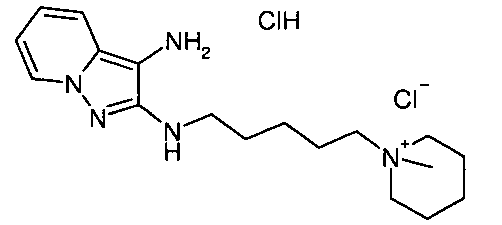

### Synthèse générale

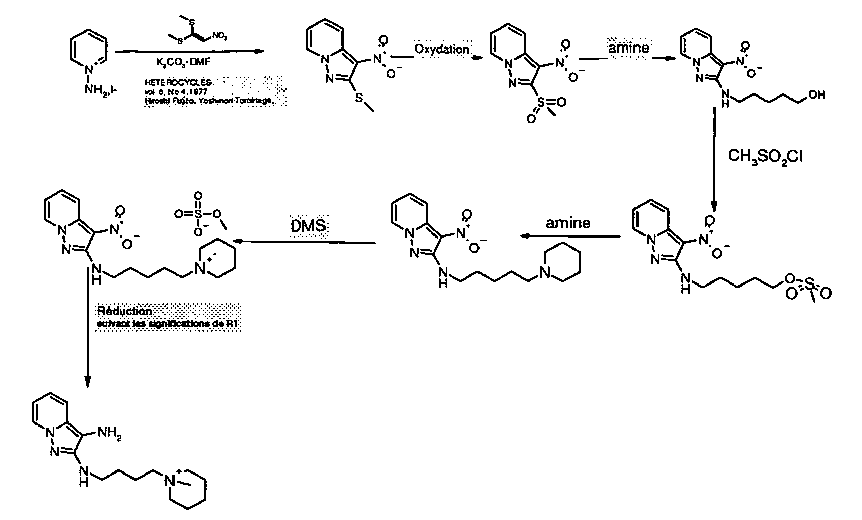

### Synthèse du 5-[(3-nitropyrazolo[1,5-a]pyridin-2-yl)amino]pentan-1-ol

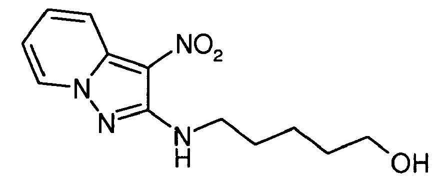

Dans un tricol de 30 mL, surmonté d'un réfrigérant à boules, d'un thermomètre et d'une agitation magnétique, on introduit 10 ml de NMP, 5g de g (0,0207 moles) 2-methanesulfanyl-3-nitro-pyrazol[1,5-a]pyridine et 5,34g (0,0518mol) de 5-amino-1-pentanol . Sous agitation, on porte à 70°C à l'aide d'un bain d'huile, pendant 4 heures en suivant par CCM (éluant : 98:2 Dichlorométhane / Méthanol).
Refroidir le milieu à la température ambiante, le milieu est versé sur 150 g de glace et le composé jaune formé est essoré sur fritté n°4 puis lavé à l'eau plusieurs fois, Après séchage à 40°C sous vide, en présence de P₂O₅, on récupère 4, 48g de solide jaune.

Les analyses RMN (¹H 400 MHz et ¹³C 100,61 MHz DMSO d₆) sont conformes à la structure attendue.

La masse du composé attendu C₁₁H₁₄N₄O₂ est détectée en spectrométrie de masse.
Les ions quasi-moléculaires MH+; [M+Na]+; [2M+Na]+ de la molécule attendue : C12H16N4O3 sont principalement détectés.

### Synthèse du 5-[(3-nitropyrazolo[1,5-a]pyridin-2-yl)amino]pentyl methanesulfonate

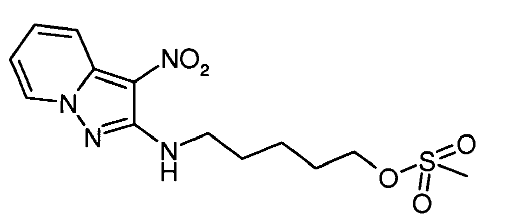

Dans un tricol de 100 mL, surmonté d'un réfrigérant à boules, d'un thermomètre et d'une agitation magnétique, on introduit 40 ml de THF, 3,48g (0,01316 moles) 5-[(3-nitropyrazolo[1,5-a]pyridin-2-yl)amino]pentan-1-ol. A zéro degrés, ajouter 5,5 ml(0, 0395 mole) de triethylamine et couler au goutte à goutte 3,17 ml(0,03950 mole) de chlorure de mesyle en 30 minutes. Maintenir l'agitation pendant 30 minutes à zéro degrés puis 2 heures à température ambiante en suivant par CCM (éluant : 98:2 Dichlorométhane / Méthanol).

le solide jaune formé est essoré sur fritté n °4 puis lavé plusieurs fois au THF , 2x 30 ml d'eau et 2x20 ml de THF. Après séchage à 40°C sous vide, en présence de P₂O₅, on récupère 3, 66g de solide jaune.

Les analyses RMN (¹H 400 MHz et ¹³C 100,61 MHz DMSO d₆) sont conformes à la structure attendue.

La masse du composé attendu C13H18N4O5S est détectée en spectrométrie de masse.

Les ions quasi-moléculaires MH+; [M+Na]+, [2M+Na]+ de la molécule attendue C13H18N4O5S sont principalement détectés

### Synthèse du 3-nitro-N-(5-piperidin-1-ylpentyl)pyrazolo[1,5-a]pyridin-2-amine

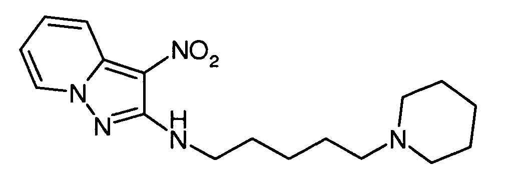

Dans un tricol de 30 mL, surmonté d'un réfrigérant à boules, d'un thermomètre et d'une agitation magnétique, on introduit 20 ml de butanol, 3, 66g de g (0,0069 moles) 5-[(3-nitropyrazolo[1,5-a]pyridin-2-yl)amino]pentyl methanesulfonate et 2,80 ml (0,03207mol) de pipéridine. Sous agitation, on porte à 100°C à l'aide d'un bain d'huile, pendant 4 heures en suivant par CCM (éluant : 98:2 Dichlorométhane / Méthanol).
Refroidir le milieu à zéro degrés, le composé cristallise, il est essoré sur fritté n°4 puis lavé à l' isopropanol plusieurs fois, Après séchage à 40°C sous vide, en présence de P₂O₅ , on récupère 2,19g de solide beige.

Les analyses RMN (¹H 400 MHz et ¹³C 100,61 MHz DMSO d₆) sont conformes à la structure attendue.

Les ions quasi-moléculaires MH+;[M+Na]+,[M+K+CH3COOH]+,[2M+H]+ de la molécule attendue : C17H25N5O2 sont principalement détectés

### Synthèse du 1-methyl-1-{5-[(3-nitropyrazolo[1,5-a]pyridin-2-yl)amino]pentyl}piperidiniummethyl sulfate

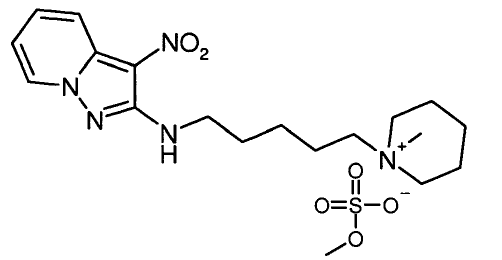

Dans un tricol de 30ml, équipé d'un réfrigérant à boules, d'un thermomètre et d'une ampoule de coulée de 50 mL, sous agitation magnétique, dans 15 mL de THF, on introduit 2,19g ( 0,0066 moles) de 3-nitro-N-(5-piperidin-1-ylpentyl)pyrazolo[1,5-a]pyridin-2-amine. On ajoute goutte à goutte 1, 31 ml (0,01387mole) de dimethylsulfate; il se forme un précipité jaune. La réaction est suivie par CCM (éluant : dichlorométhane / méthanol 98 :2), jusqu'à la disparition du produit de départ pendant 24 heures. Le solide formé est essoré puis lavé plusieurs fois au THF. Apres séchage sous vide, en présence de P₂O₅ , on récupère 2,82g de poudre jaune clair.

Les analyses RMN (¹H 400 MHz et ¹³C 100,61 MHz DMSO d₆) sont conformes à la structure attendue.
L'ion moléculaire M+ attendue [C18H28N5O2]+ est principalement détecté ainsi que son contre-ion [CH3O4S]

### Synthèse du chlorhydrate de chlorure de 1-{5-[(3-aminopyrazolo[1,5-a]pyridin-2-yl)amino]pentyl}-1-methylpiperidinium

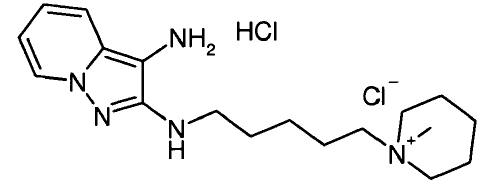

Dans un tricol de 250ml L, surmonté d'un réfrigérant, d'un thermomètre et d'une agitation magnétique, on introduit 70ml d' éthanol et 2,80g de zinc. Ce milieu est porte au reflux et 5 gouttes d'acide acétiques sont ajoutées. Une solution issue de 2,80g de 1-methyl-1-{4-[(3-nitropyrazolo[1,5-a]pyridin-2-yl)amino]butyl}piperidinium methyl sulfate, de 15 ml d'éthanol et 3 ml d'eau est ajoutée goutte à goutte en 30 minutes et le reflux est maintenu pendant 24 heures.

Lorsque la réduction est terminée, éliminer le zinc par filtration sur célite et récupérer les jus dans 70 ml l'isopropanol chlorhydrique préalablement refroidi par un bain froid de carboglace. Laver le zinc avec de l'éthanol . Les jus sont concentrés au 1/3 et à zéro degrés la cristallisation est amorcée.
Le solide bleu clair est essoré sur fritté n°3, après séchage sous vide, en présence de P₂O₅. On récupère ainsi 2,16g d'une poudre bleu clair.

Les analyses RMN (¹H 400 MHz et ¹³C 100,61 MHz DMSO d₆) sont conformes à la structure attendue.
Le cation attendu [C18H30N5]+ est principalement détecté à m/z,ESP+=316, 158, 174 (correspondant respectivement à [M]+, [M+H]2+, [M+H+CH3OH]2+.

### Exemple 23

### Chlorhydrate de chlorure de 1-{2-[(3-aminopyrazolo[1,5-a]pyridin-2-yl)amino]ethyl}-3-methyl-1H-imidazol-3-ium

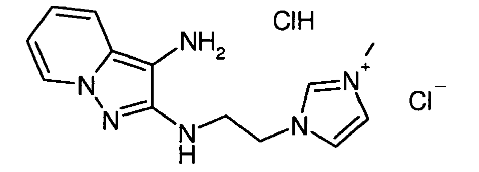

### Synthèse générale

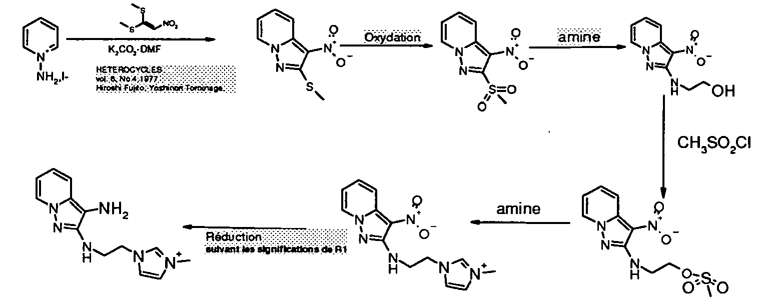

### Synthèse du 2-[(3-nitropyrazolo[1,5-a]pyridin-2-yl)amino]ethanol

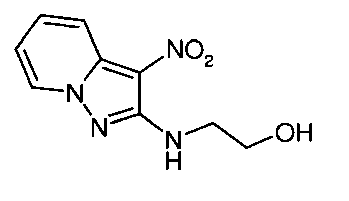

Dans un tricol de 100 mL, surmonté d'un réfrigérant à boules, d'un thermomètre et d'une agitation magnétique, on introduit 50 ml de NMP, 5g de g (0,0207 moles) 2-methanesulfanyl-3-nitro-pyrazol[1,5-a]pyridine et 6,25ml (0,0103mol) d'éthanolamine . Sous agitation, on porte à 80°C à l'aide d'un bain d'huile, pendant 2 heures en suivant par CCM (éluant : 98:2 Dichlorométhane / Méthanol).

Refroidir le milieu à la température ambiante, le milieu est versé sur 300 g de glace et le composé jaune formé est essoré sur fritté n°4 puis lavé à l'eau plusieurs fois, Après séchage à 40°C sous vide, en présence de P₂O₅, on récupère 4, 20g de solide jaune.

Les analyses RMN (¹H 400 MHz et ¹³C 100,61 MHz DMSO d₆) sont conformes à la structure attendue.

La masse du composé attendu C9H10N4O3 est détectée en spectrométrie de masse.

### Synthèse du 2-[(3-nitropyrazolo[1,5-a]pyridin-2-yl)amino]ethyl methanesulfonate

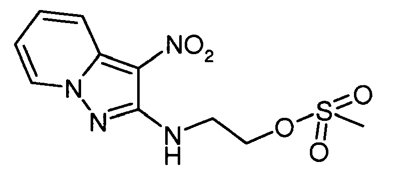

Dans un tricol de 100 mL, surmonté d'un réfrigérant à boules, d'un thermomètre et d'une agitation magnétique, on introduit 40 ml de THF, 4,2g (0,01316 moles) 2-[(3-nitropyrazolo[1,5-a]pyridin-2-yl)amino]ethanol. A zéro degrés, ajouter 3,16 ml(0, 02268 mole) de triethylamine et couler au goutte à goutte 1,66 ml(0,02079 mole) de chlorure de mesyle en 30 minutes. Maintenir l'agitation pendant 30 minutes à zéro degrés puis 2 heures à température ambiante en suivant par CCM (éluant : 98:2 Dichlorométhane /Méthanol).

le solide jaune formé est essoré sur fritté n °4 puis lavé plusieurs fois au THF , 2x 30 ml d'eau et 2x20 ml de THF. Après séchage à 40°C sous vide, en présence de P₂O₅, on récupère 4, 03g de solide jaune.

Les analyses RMN (¹H 400 MHz et ¹³C 100,61 MHz DMSO d₆) sont conformes à la structure attendue.

La masse du composé attendu C10H12N4O5S est détectée en spectrométrie de masse, Les ions quasi-moléculairesMH+;[M+Na]+,[2M+Na]+,[M-H]-de la molécule attendue: C10H12N4O5S sont principalement détectés.

### Synthèse du chlorure de 3-methyl-1-{2-[(3-nitropyrazolo[1,5-a]pyridin-2-yl)amino]ethyl}-1H-imidazol-3-ium

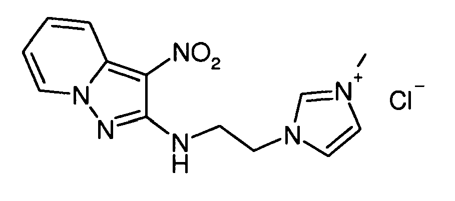

Dans un tricol de 30 mL, surmonté d'un réfrigérant à boules, d'un thermomètre et d'une agitation magnétique, on introduit 3 ml de butanol, 1, 0g de g (0,0033 moles) 2-[(3-nitropyrazolo[1,5-a]pyridin-2-yl)amino]ethyl methanesulfonate et (0,0099mol) de 1-methyimidazole. Sous agitation, on porte à 100°C à l'aide d'un bain d'huile, pendant 4 heures en suivant par CCM (éluant : 98:2 Dichlorométhane / Méthanol).
Refroidir le milieu à zéro degrés,ajouter 5 ml d'isopropanol chlorhydrique, le composé cristallise, il est essoré sur fritté n °4 puis lavé à l' isopropanol plusieurs fois, Après séchage à 40°C sous vide, en présence de P₂O₅ , on récupère 0,714g de solide beige.

Les analyses RMN (¹H 400 MHz et ¹³C 100,61 MHz DMSO d₆) sont conformes à la structure attendue.

L' ion moléculaire M+ de la molécule attendue : [C13H15N6O2]+ est principalement détectéé

### Synthèse du chlorhydrate de chlorure de 1-{2-[(3-aminopyrazolo[1,5-a]pyridin-2-yl)amino]ethyl}-3-methyl-1H-imidazol-3-ium

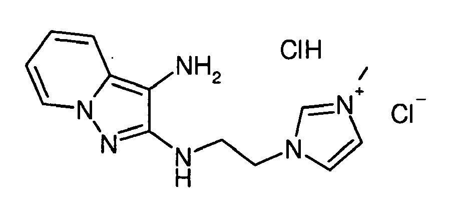

Dans un tricol de 250ml L, surmonté d'un réfrigérant, d'un thermomètre et d'une agitation magnétique, on introduit 1000ml d' éthanol et 3,25g de zinc. Ce milieu est porte au reflux et 5 gouttes d'acide acétiques sont ajoutées. Une solution issue de 3,25g de chlorure de 3-methyl-1-{2-[(3-nitropyrazolo[1,5-a]pyridin-2-yl)amino]ethyl}-1H-imidazol-3-ium , de 10 ml d'éthanol et 4 ml d'eau est ajoutée goutte à goutte en 30 minutes et le reflux est maintenu pendant 24 heures.

Lorsque la réduction est terminée, éliminer le zinc par filtration sur célite et récupérer les jus dans 70 ml l'isopropanol chlorhydrique préalablement refroidi par un bain froid de carboglace. Laver le zinc avec de l'éthanol. Les jus sont concentrés au 1/3 et à zéro degrés la cristallisation est amorcée.
Le solide gris clair est essoré sur fritté n°3, après séchage sous vide, en présence de P₂O₅. On récupère ainsi 2,85g d'une poudre gris clair.

Les analyses RMN (¹H 400 MHz et ¹³C 100,61 MHz DMSO d₆) sont conformes à la structure attendue.
L' ion moléculaire M+ attendue : C13H17N6 est principalement détecté

### EXEMPLES DE TEINTURE

Les compositions tinctoriales suivantes sont préparées

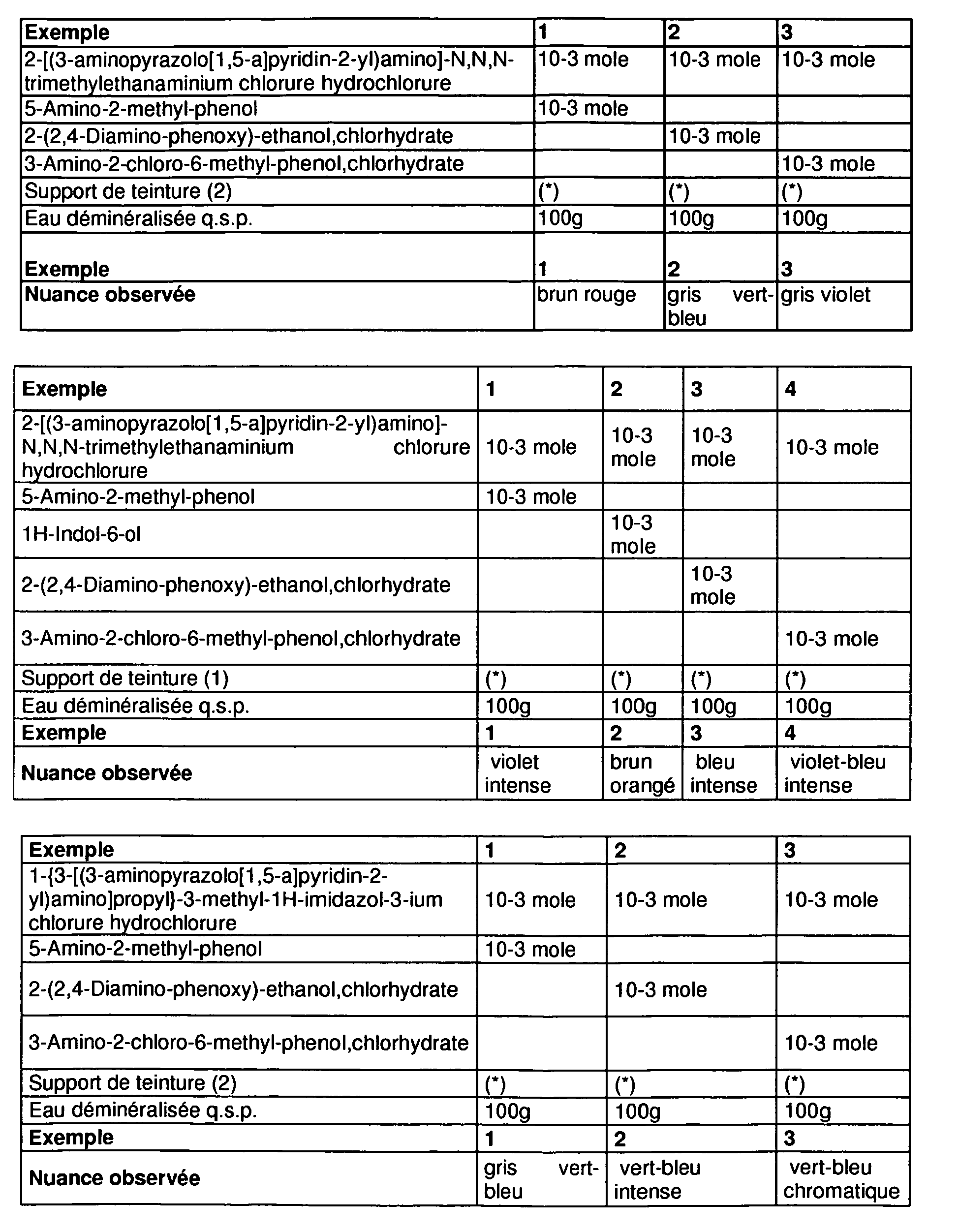

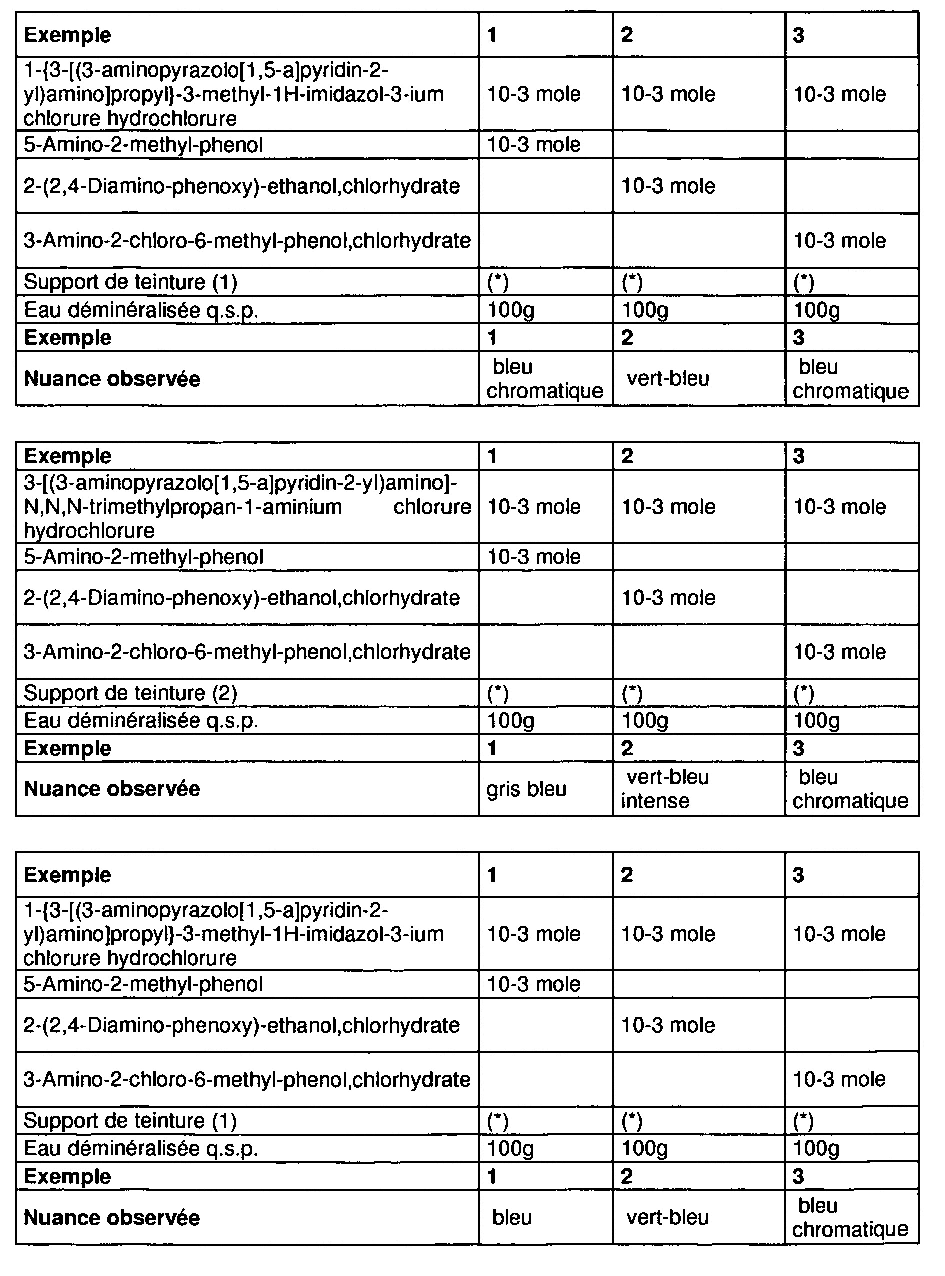

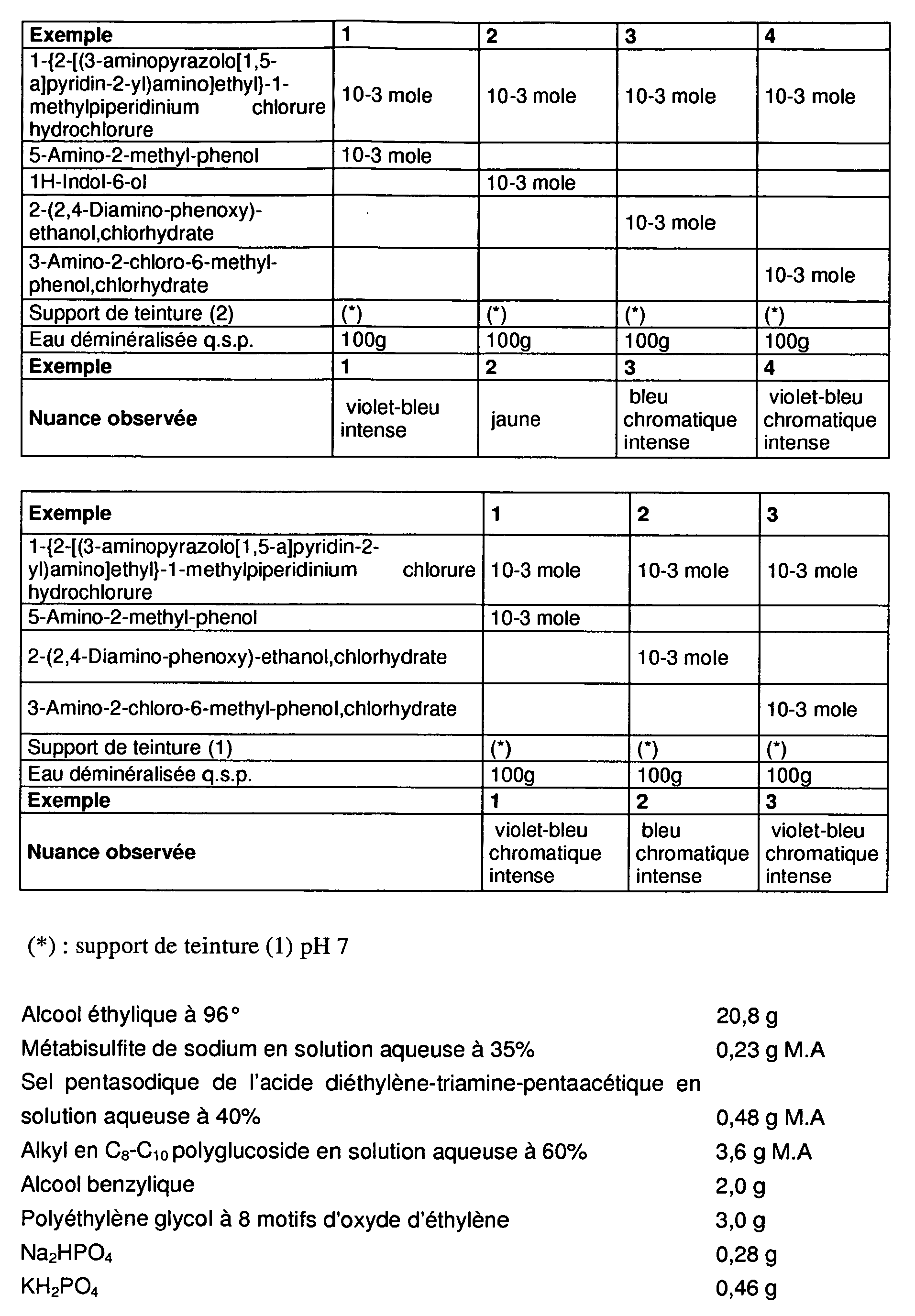

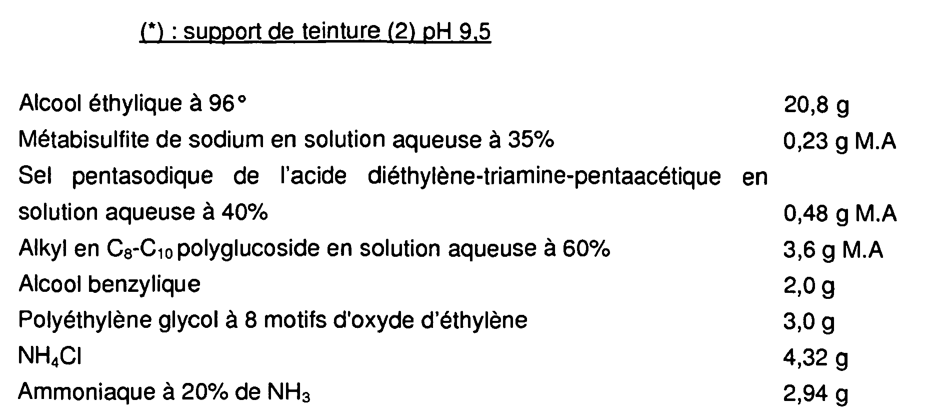

Au moment de l'emploi, chaque composition est mélangée avec un poids égal d'eau oxygénée à 20 volumes (6% en poids). On obtient un pH final de 7, resp, 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées. Les résultats obtenus sont reportés dans les tableaux ci-dessus.

## Revendications

1. Composition de teinture des fibres kératiniques comprenant, dans un milieu approprié à la teinture, à titre de base de coloration d'oxydation, au moins un dérivé 3-amino pyrazolo-[1,5-a]-pyridine de formule (I) suivante ainsi que ses sels et solvates: 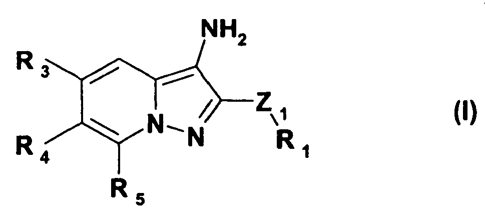 dans laquelle
• Z₁ représente un radical -O-, un radical -NR₆- avec R₆ qui représente un atome d'hydrogène, un radical alkyle, ou R₆ forme avec R₁, un hétérocycle cationique et/ou substitué par un radical cationique
• R₁ représente
- un hydrogène
- un radical alkyle en C₁-C₁₀ substitué ou interrompu par un radical cationique,
• R₃, R₄ et R₅, identiques ou différents, représentent
- un atome d'hydrogène,
- un radical alkyle en C₁-C₄
- R₄ et R₅, forment ensemble un cycle saturé ou insaturé à 5 ou 8 chaînons,
• X représente un anion ou groupe d'anions permettant d'assurer l'électronégativité du dérivé de formule (I),
avec la condition qu'au moins un des groupements Z₁, R₁, représente un radical cationique

2. Composition selon la revendication 1 dans laquelle R₆ forme avec R₁, un hétérocycle cationique ou un hétérocycle substitué par un radical cationique.

3. Composition selon la revendication 2 dans laquelle R₆ forme avec R₁, les imidazoles substitués par un radical ammonium quaternaire ou les imidazoliums, les pipérazines substitués par un radical ammonium quaternaire ou les pipéraziniums, les pyrrolidines substitués par un radical ammonium quaternaire ou les pyrrolidiniums, les diazépanes substitués par un radical ammonium quaternaire ou les diazépaniums..

4. Composition selon la revendication 1 dans laquelle Z₁ représente -NH-ou NR₆ avec R₆ qui forme avec R₁ un hétérocycle cationique ou substitué par un radical cationique.

5. Composition selon l'une quelconque des revendications 1 à 4 dans laquelle le composé de formule (I) est choisi parmi /

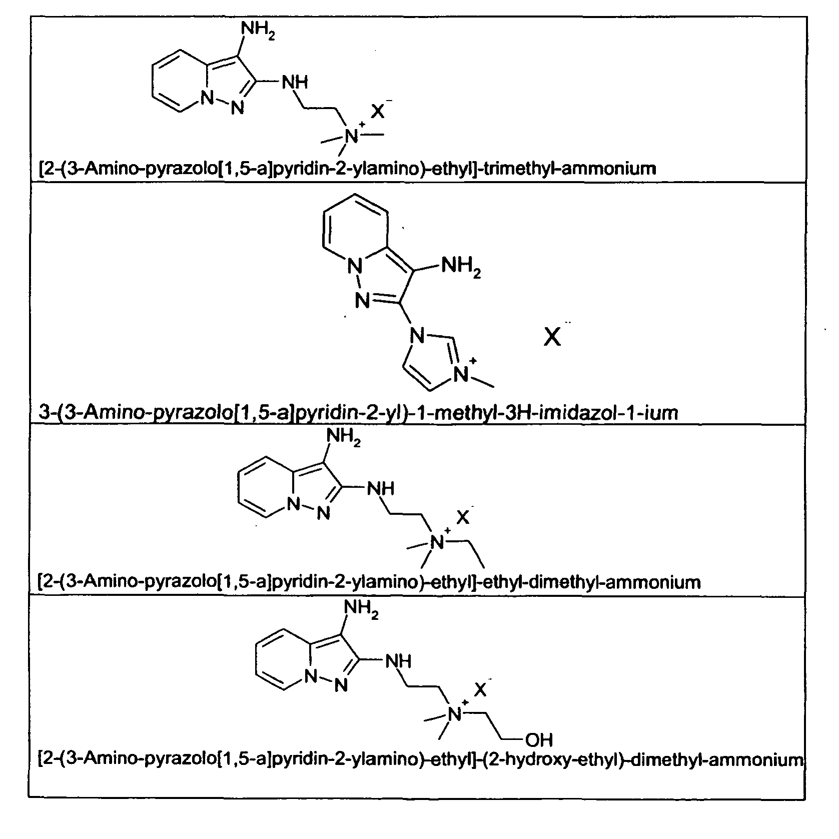

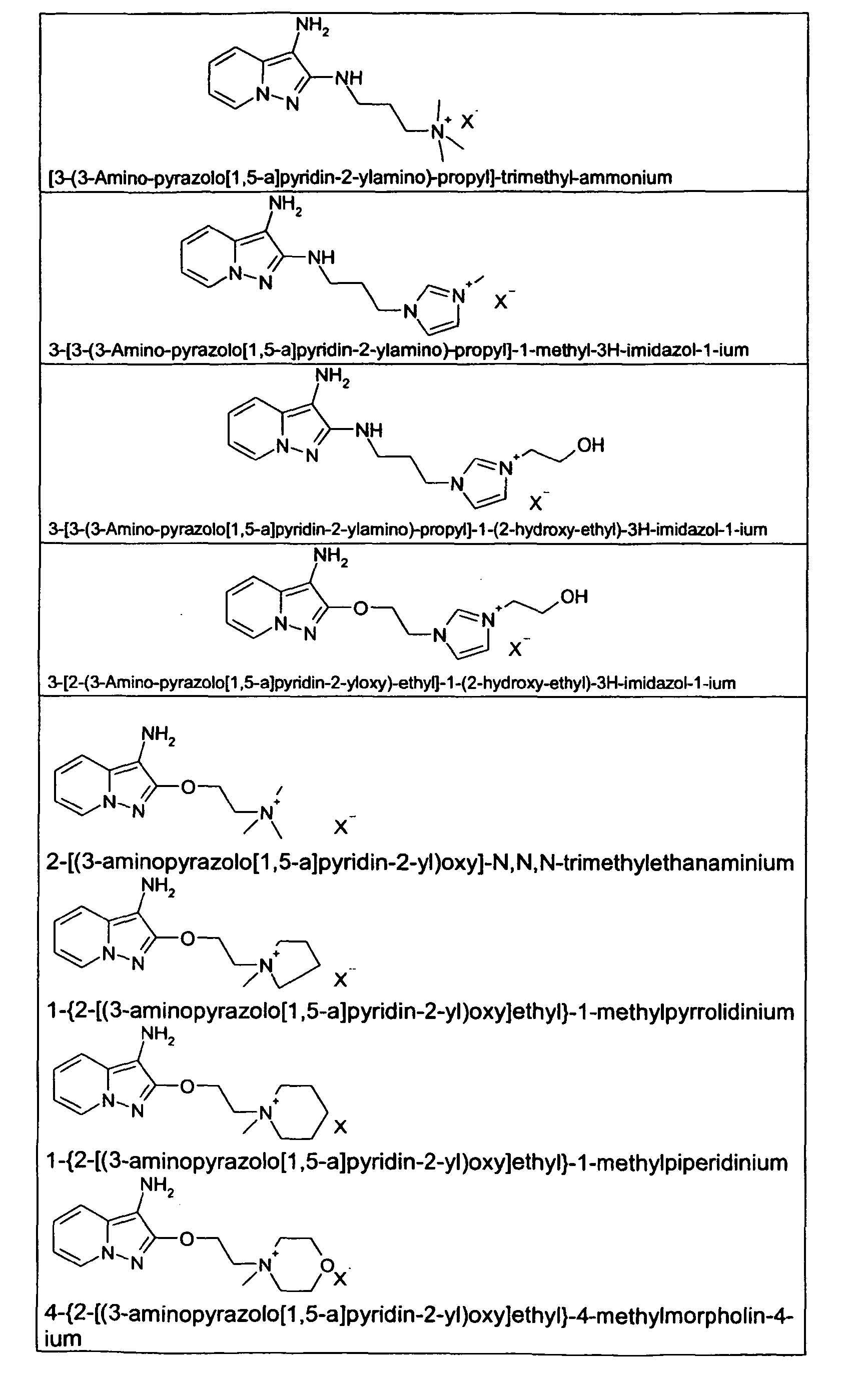

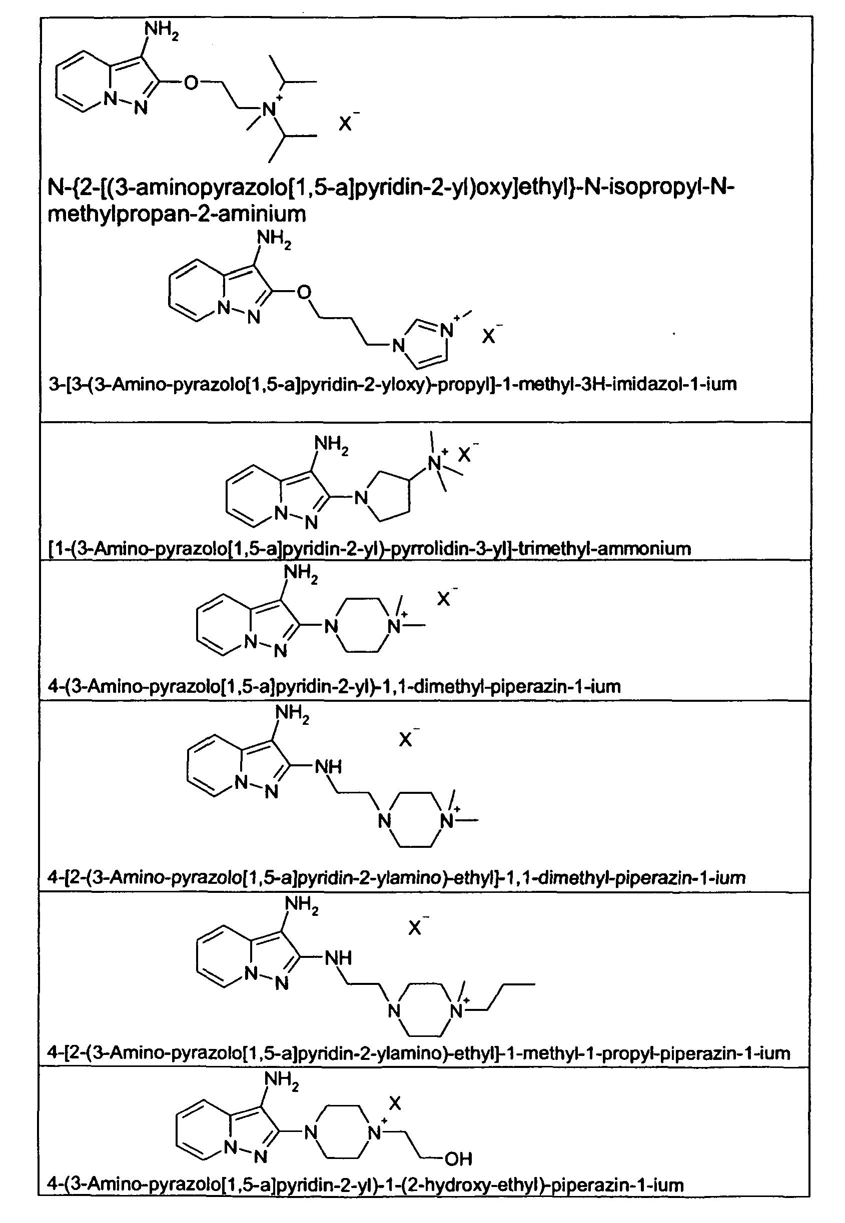

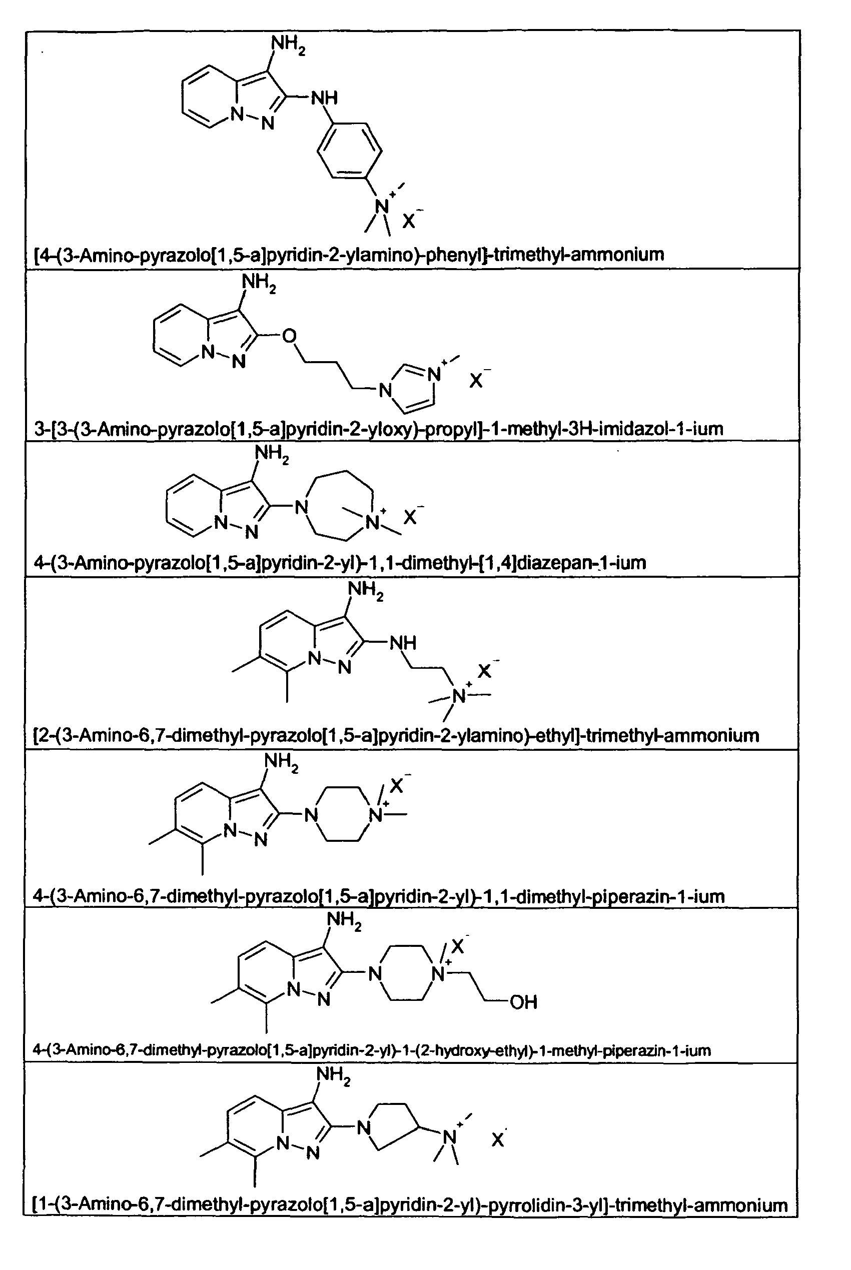

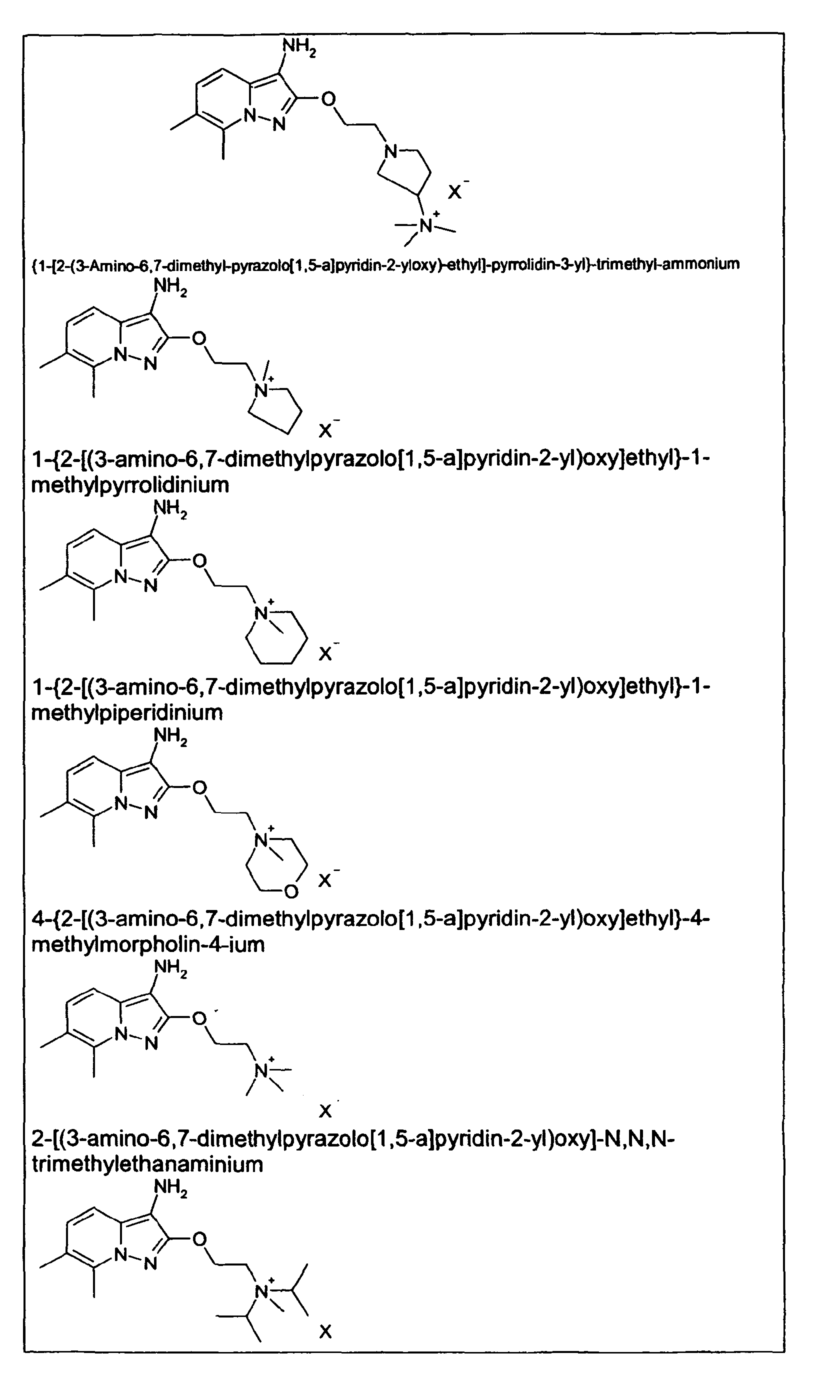

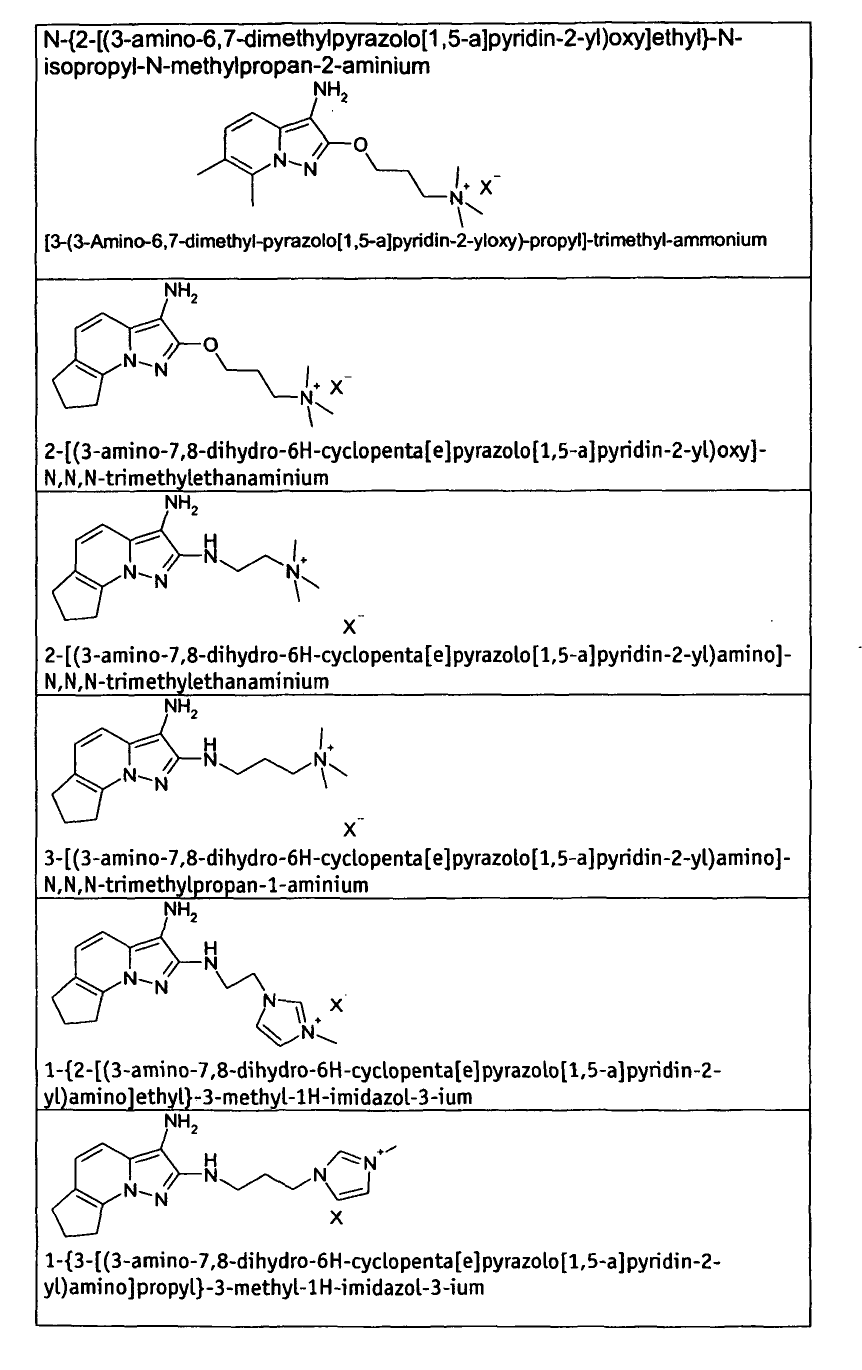

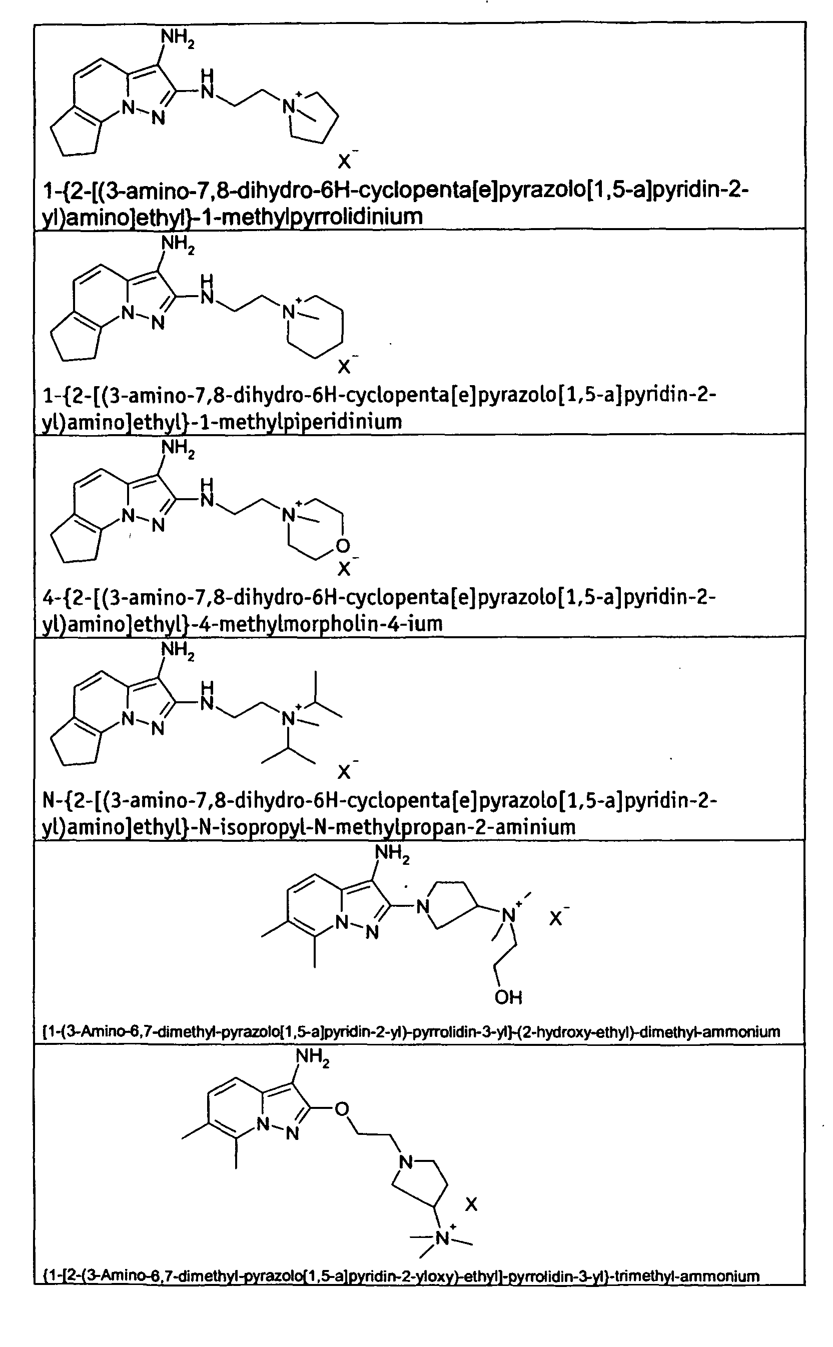

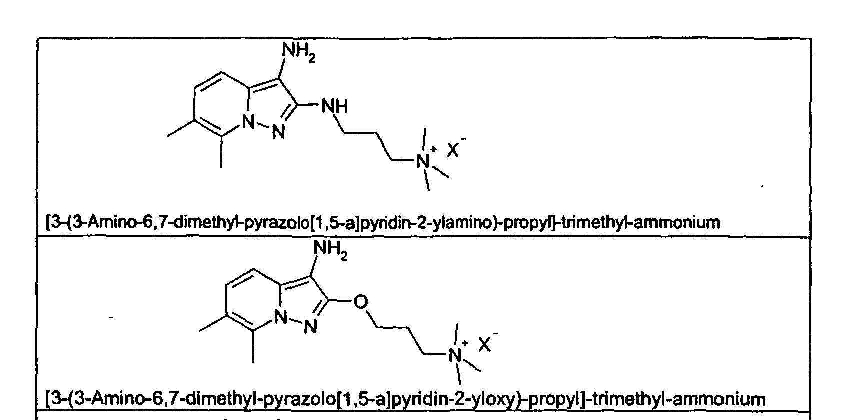

6. Composition selon la revendication 5 dans laquelle le dérivé de formule (I) est choisi parmi les dérivés dans lesquels Z₁ est égal à -NH- et R1 est un radical cationique ou Z₁ est égal à -0- et R₁ est un radical cationique.

7. Composition selon l'une quelconque de revendications précédentes dans laquelle la quantité de chacune des bases d'oxydation de formule (I) est comprise entre 0,001 à 10 % en poids environ du poids total de la composition tinctoriale.

8. Composition selon l'une quelconque des revendications précédentes comprenant de plus un coupleur choisi parmi les méta-phénylènediamines, les méta-aminophénols, les méta-diphénols, les coupleurs naphtaléniques, les coupleurs hétérocycliques et leurs sels d'addition, ainsi que leurs mélanges.

9. Composition selon la revendication 8 dans laquelle la quantité de chacun des coupleurs est comprise entre 0,001 et 10 % en poids du poids total de la composition tinctoriale.

10. Procédé de teinture des fibres kératiniques, **caractérisé en ce qu'**une composition telle que définie dans l'une quelconque des revendications 1 à 9 est appliquée sur les fibres kératiniques en présence d'un agent oxydant pendant un temps suffisant pour développer la coloration désirée.

11. Procédé selon la revendication 10 dans lequel l'agent oxydant est choisi parmi le peroxyde d'hydrogène, le peroxyde d'urée, les bromates de métaux alcalins, les persels, les peracides et les enzymes oxydases.

12. Dispositif à plusieurs compartiments dans lequel un premier compartiment contient une composition tinctoriale telle que définie dans l'une quelconque des revendications 1 à 9 et un deuxième compartiment contient un agent oxydant.

13. Dérivés de formule (I) tels que définis selon l'une quelconque des revendications 1 à 7.

14. Procédé de préparation d'un composé de formule (I) tel que défini dans les compositions selon l'une quelconque des revendications 1 à 6 qui comprend les étapes suivantes 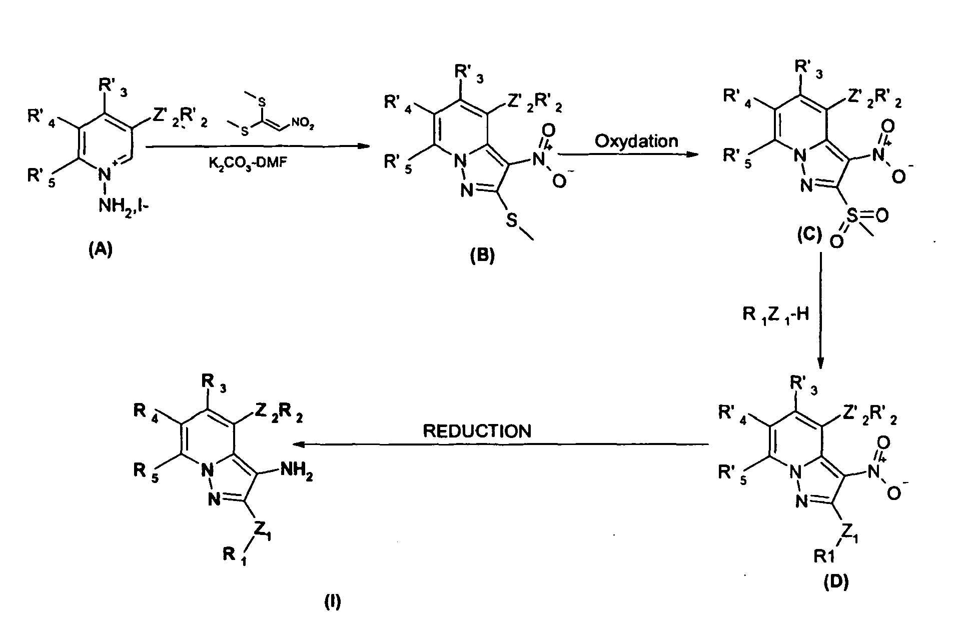 dans laquelle Z1, R1, Z'2, R'2, R'3, R'4 et R'5 ont les mêmes significations que Z2, R2, R3, R4 et R5 ou en sont des précurseurs.

15. Composés intermédiaires de formules (A) et (B) tels que définis à la revendication 14 à l'exception des composés 1-amino-3-methylpyridinium iodide. et 4-methyl-2-(methylsulfanyl)-3-nitropyrazolo[1,5-a]pyridine.

16. Composés intermédiaires de formule (C) ou (D) tels que définis à la revendication 14.

## Claims

1. Composition for dyeing keratin fibres comprising, in a suitable dyeing medium, as oxidation dyeing base, at least one 3-amino pyrazolo-[1,5-a]-pyridine derivative of the following formula (I) as well as its salts and solvates: 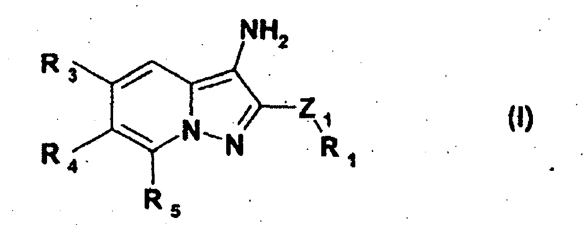 in which
Z₁ represents a radical -O-, a radical -NR₆- with R₆ which represents a hydrogen atom, an alkyl radical, or R₆ forms with R₁ a heterocycle that is cationic and/or is substituted with a cationic radical
• R₁ represents
- a hydrogen,
- a C₁-C₁₀ alkyl radical substituted or interrupted by a cationic radical,
• R₃, R₄ and R₅, which may be identical or different, represent
- a hydrogen atom,
- a C₁-C₄ alkyl radical,
- R₄ and R₅ together form a saturated or unsaturated ring with 5 or 8 ring members,
• X represents an anion or group of anions that can ensure electronegativity of the derivative of formula (I),
with the condition that at least one of the groups Z₁, R, represents a cationic radical.

2. Composition according to Claim 1, in which R₆ forms with R₁ a cationic heterocycle or a heterocycle substituted with a cationic radical

3. Composition according to Claim 2 in which R₆ forms with R₁ the imidazoles substituted with a quaternary ammonium radical or the imidazoliums, the piperazines substituted with a quaternary ammonium radical or the piperaziniums, the pyrrolidines substituted with a quaternary ammonium radical or the pyrrolidiniums, the diazepanes substituted with a quaternary ammonium radical or the diazepaniums.

4. Composition according to Claim 1 in which Z₁ represents -NH- or NR₆ with R₆ which forms with R₁ a heterocycle that is cationic or is substituted with a cationic radical.

5. Composition according to any one of Claims 1 to 4 in which the compound of formula (I) is selected from /

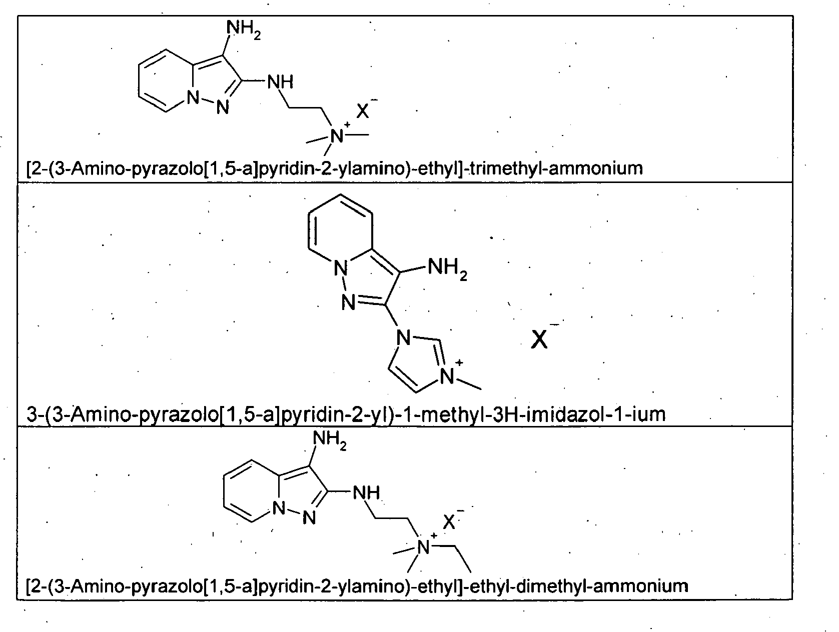

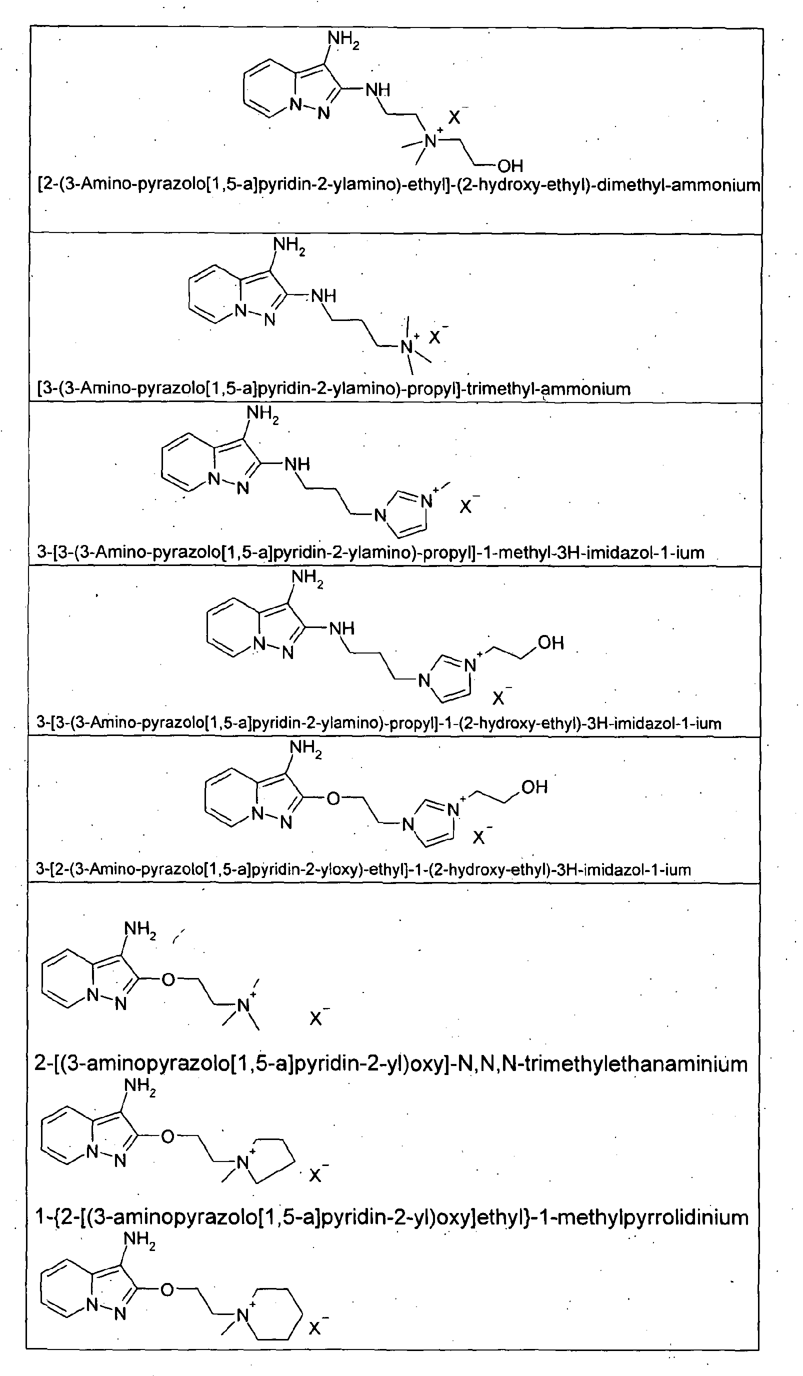

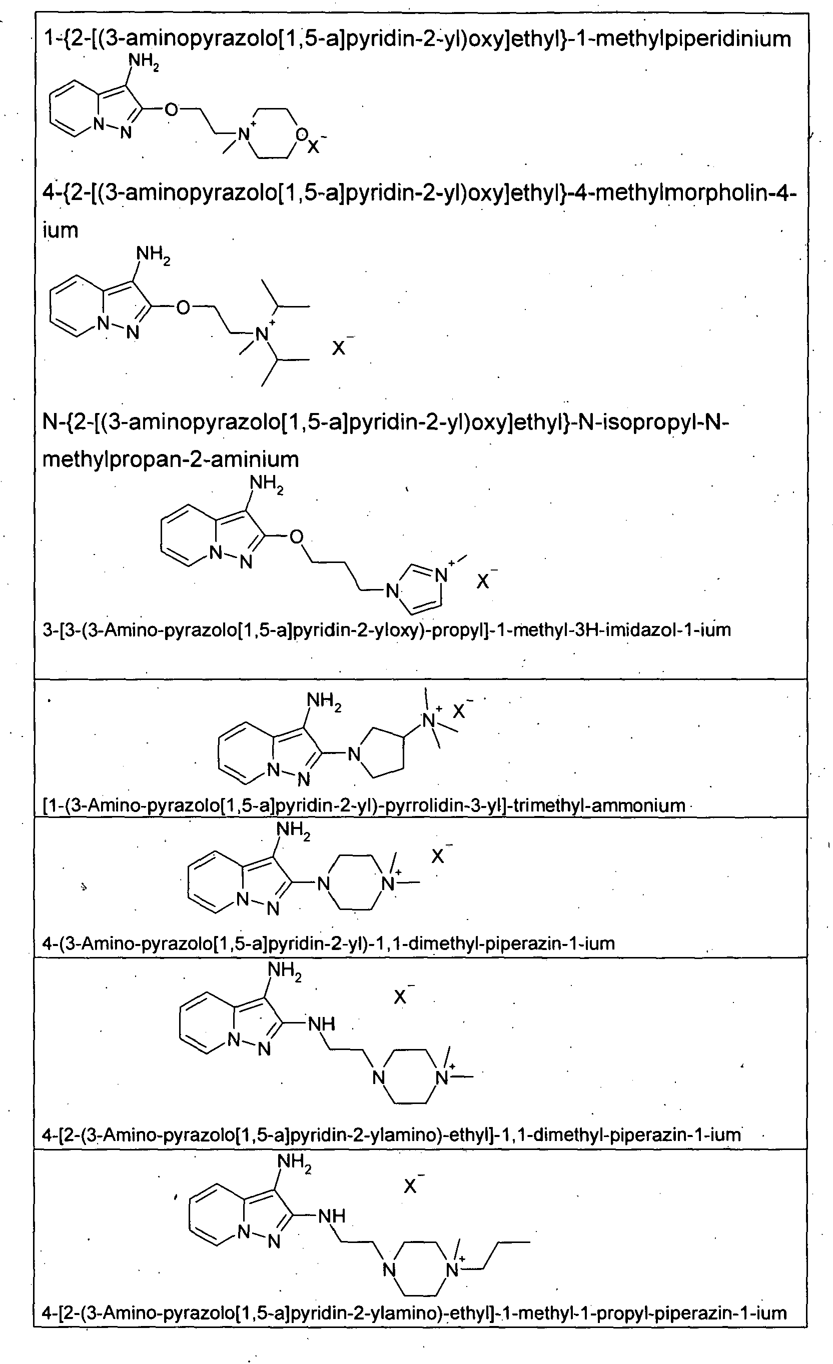

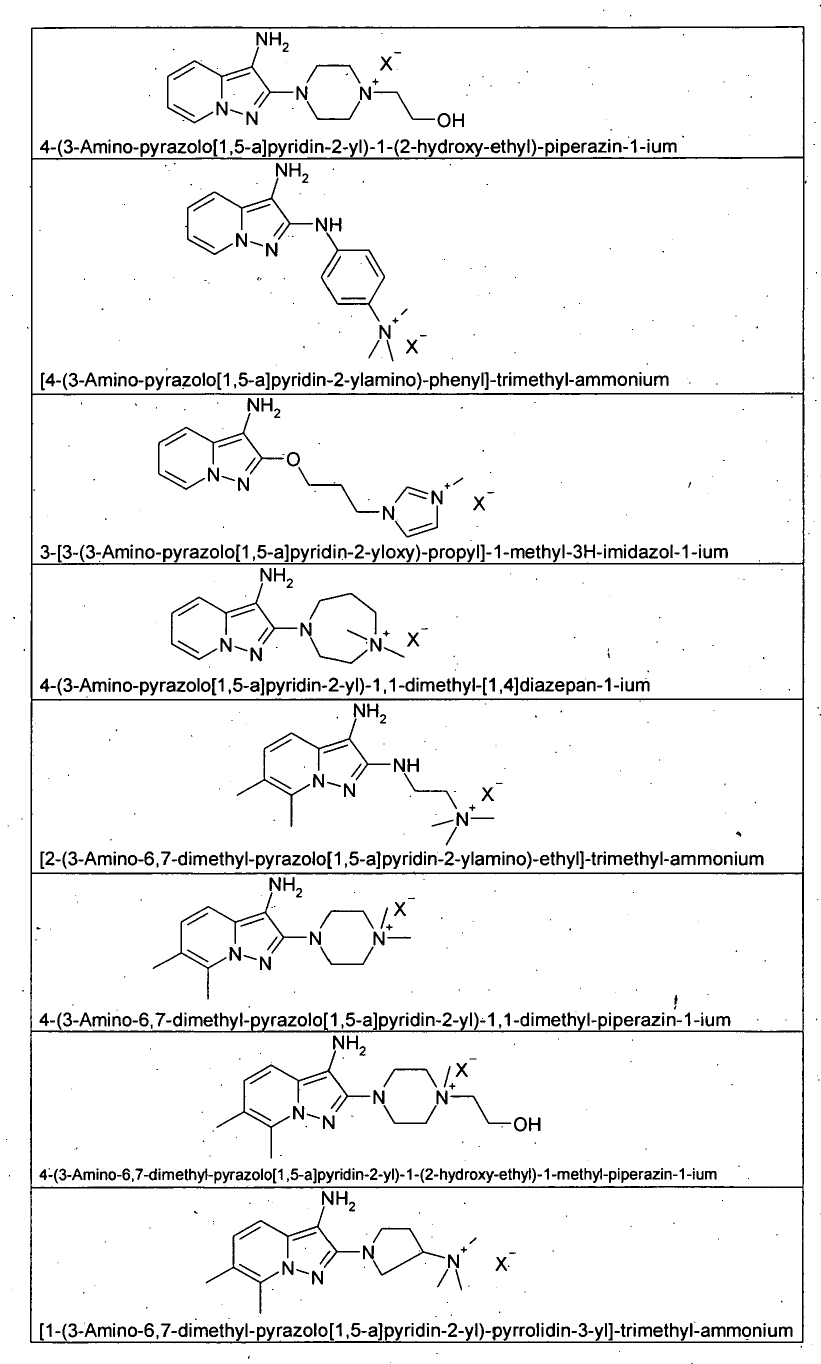

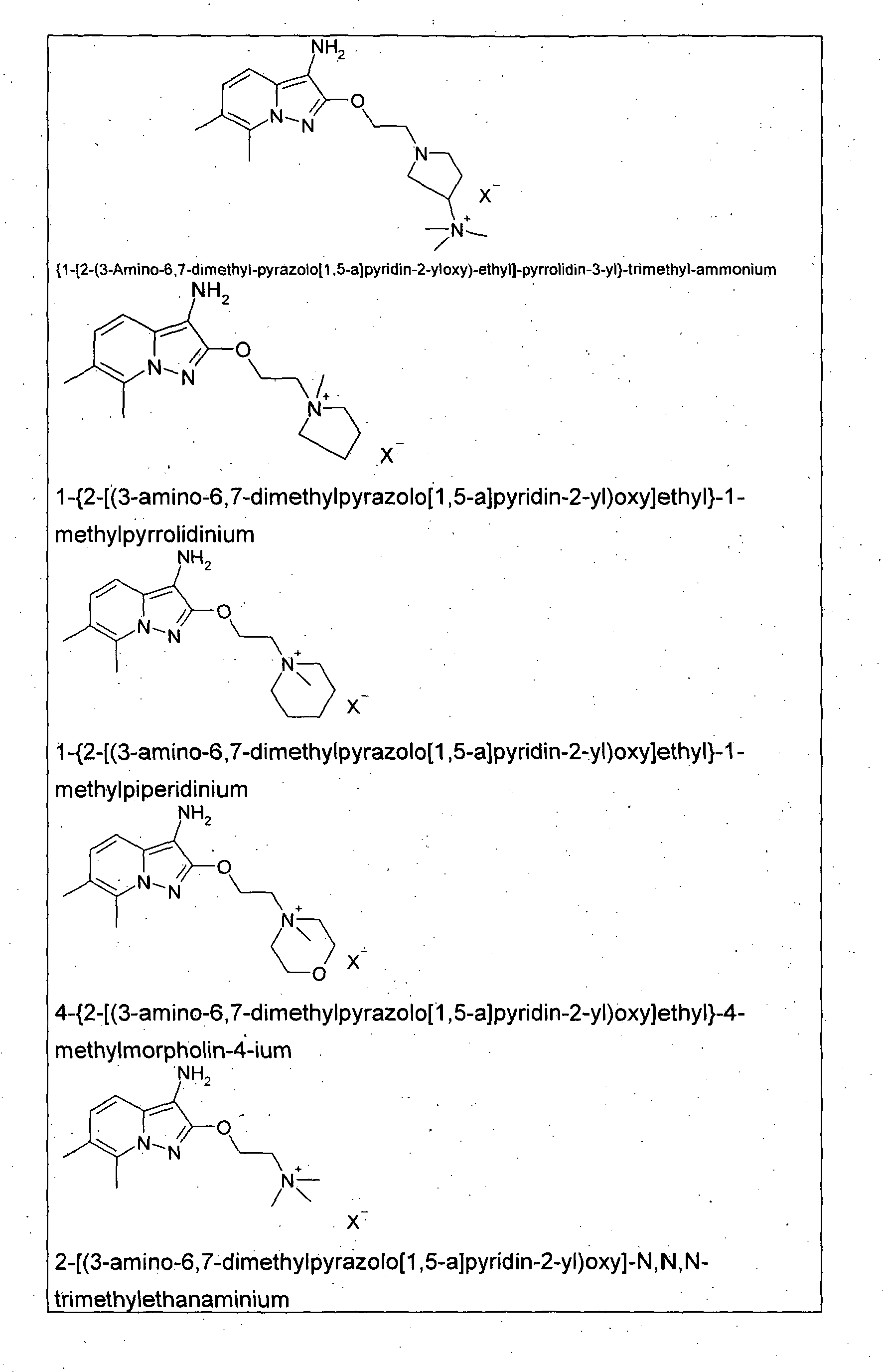

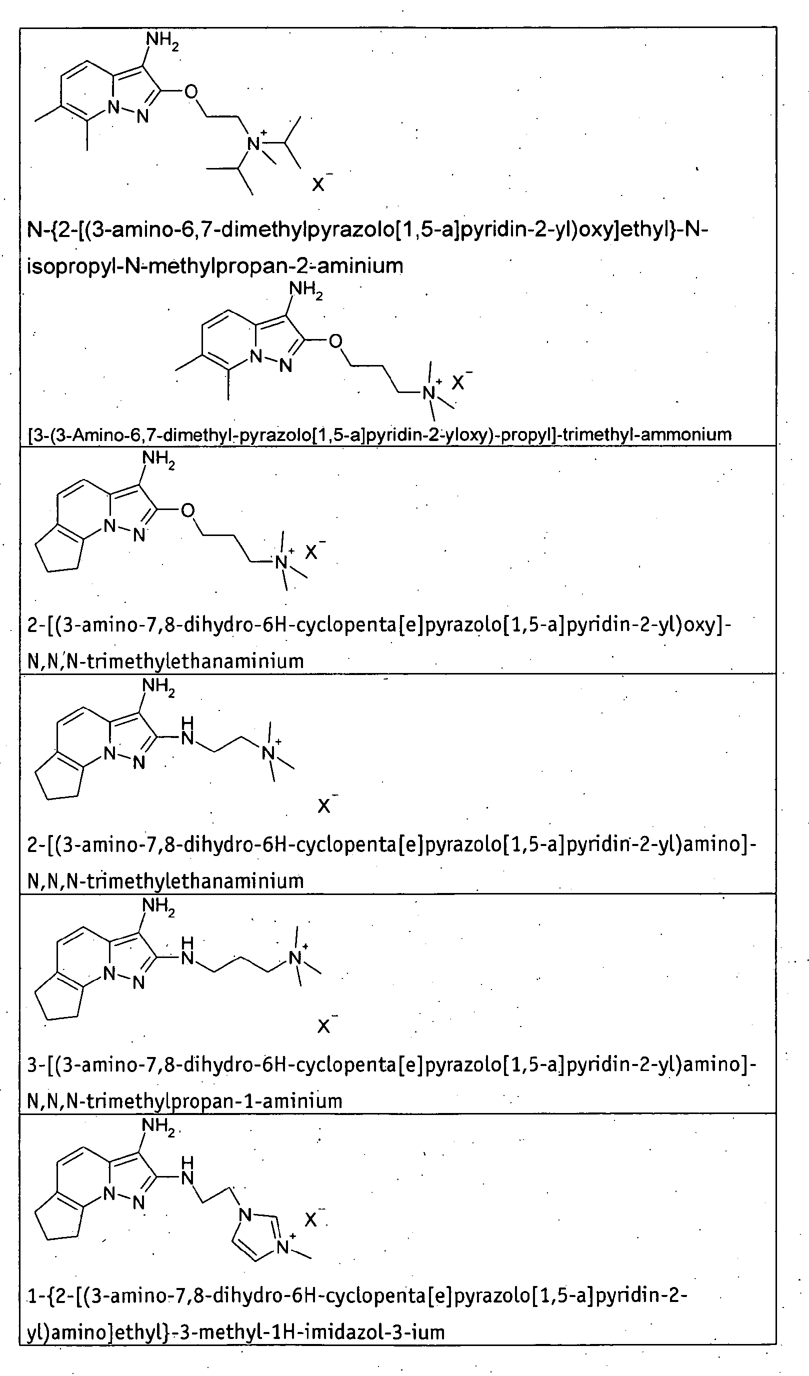

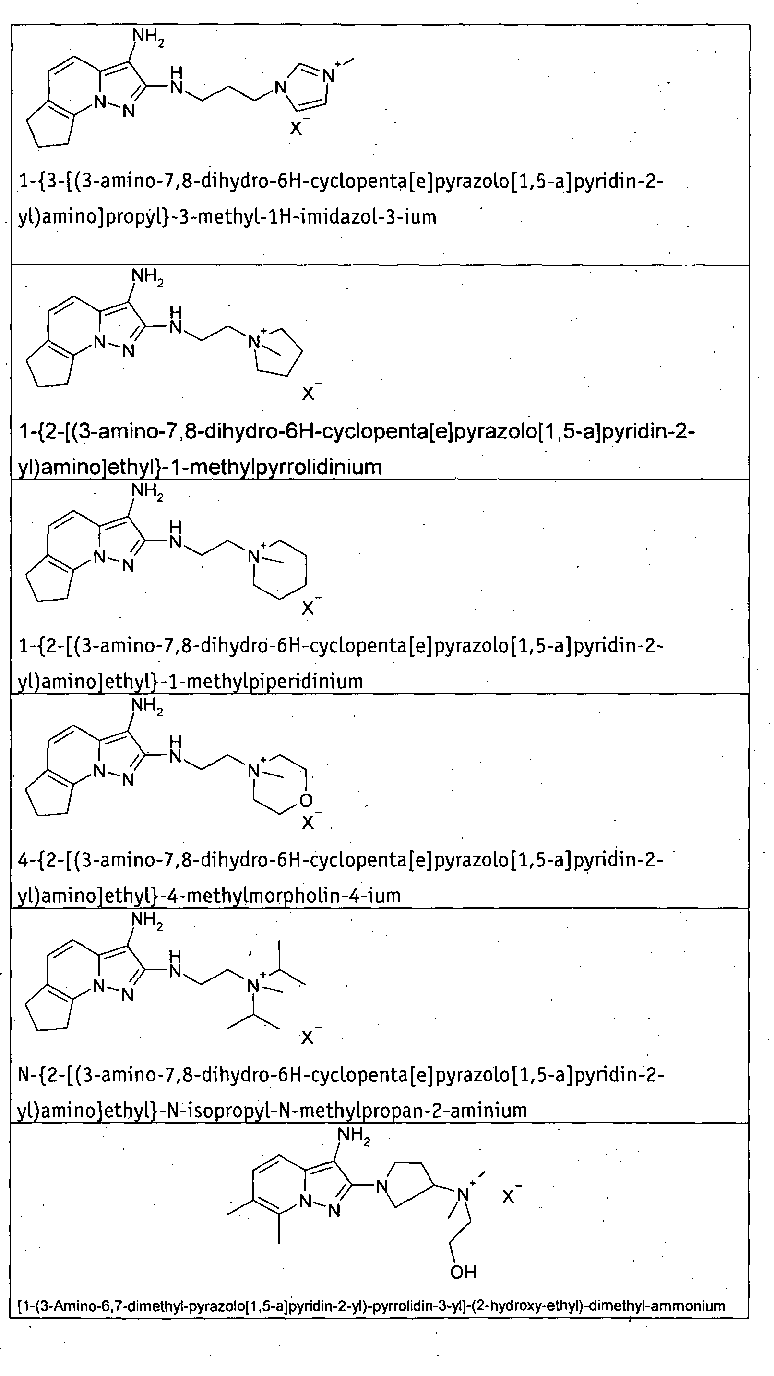

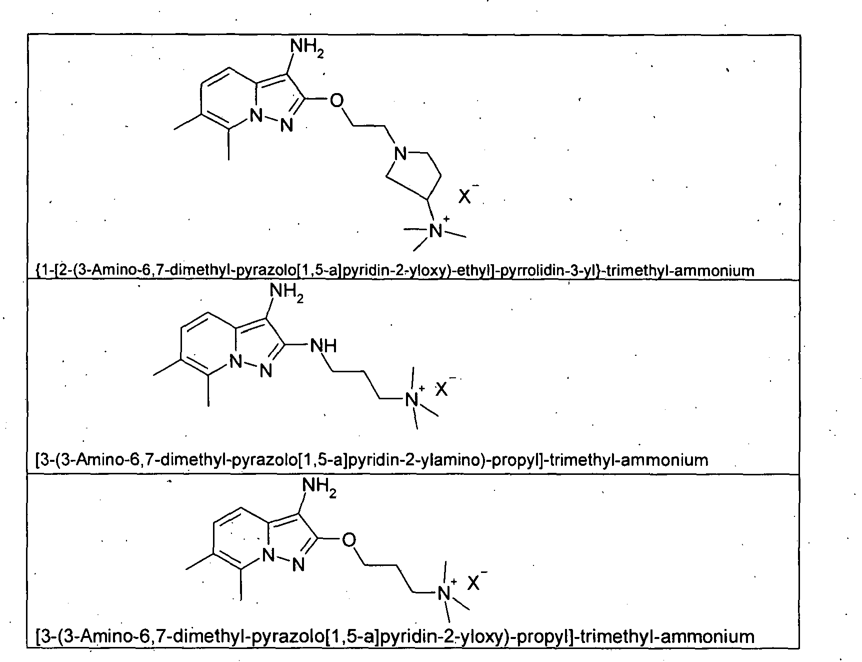

6. Composition according to Claim 5 in which the derivative of formula (I) is selected from the derivatives in which Z₁ is equal to -NH- and R₁ is a cationic radical or Z₁ is equal to -O- and R₁ is a cationic radical.

7. Composition according to any one of the preceding claims in which the quantity of each of the oxidation bases of formula (I) is between about 0.001 and 10 wt % of the total weight of the dyeing composition.

8. Composition according to any one of the preceding claims, additionally comprising a coupler selected from the meta-phenylenediamines, meta-aminophenols, meta-diphenols, naphthalenic couplers, heterocyclic couplers and their salts of addition, as well as mixtures thereof

9. Composition according to Claim 8 in which the quantity of each of the couplers is between 0 001 and 10 wt.% of the total weight of the dyeing composition.

10. Method for dyeing keratin fibres, **characterized in that** a composition as defined in any one of Claims 1 to 9 is applied on keratin fibres in the presence of an oxidizing agent for a time that is sufficient to develop the desired colouring

11. Method according to Claim 10 in which the oxidizing agent is selected from hydrogen peroxide, urea peroxide, alkali metal bromates, per-salts, per-acids and oxidase enzymes.

12. Kit with several compartments in which a first compartment contains a dyeing composition as defined in any one of Claims 1 to 9 and a second compartment contains an oxidizing agent.

13. Derivatives of formula (I) as defined according to any one of Claims 1 to 7

14. Method of preparation of a compound of formula (I) as defined in the compositions according to any one of Claims 1 to 6 which comprises the following stages 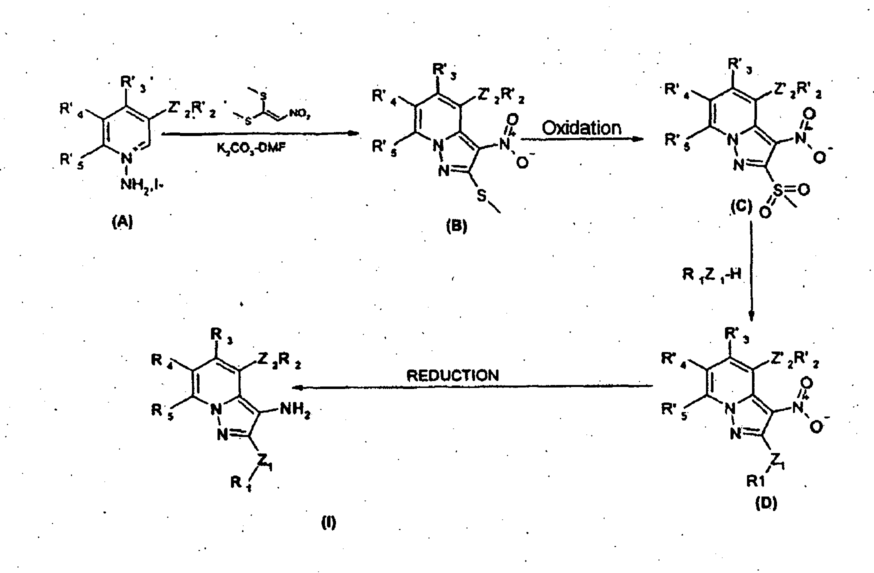 in which Z₁, R₁, Z'₂, R'₂, R'₃, R'₄ and R'₅ have the same meanings as Z₂, R₂, R₃, R₄ and R₅ or are precursors thereof.

15. Intermediates of formulae (A) and (B) as defined in Claim 14 with the exception of the compounds 1-amino-3-methylpyridinium iodide and 4-methyl-2-(methylsulphanyl)-3-nitropyrazolo[1,5-a]pyridine.

16. Intermediates of formula (C) or (D) as defined in Claim 14

## Patentansprüche

1. Zusammensetzung zum Färben von Keratinfasern, enthaltend in einem zum Färben geeigneten Medium als Oxidationsbase mindestens ein 3-Amino-pyrazolo-[1,5-a]-pyridinderivat der folgenden Formel (I) sowie seine Salze und Solvate: 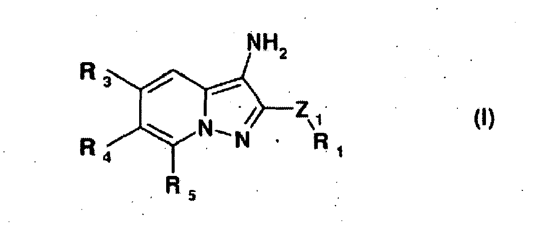 wobei in der Formel:
Z₁ bedeutet: eine Gruppe -O-, eine Gruppe -NR₆-, wobei R₆ ein Wasserstoffatom oder eine Alkylgruppe bedeutet oder R₆ bildet mit R₁ einen kationischen und/oder mit einer kationischen Gruppe substituierten Heterocyclus,
R₁ bedeutet:
- ein Wasserstoffatom,
- eine C₁₋₁₀-Alkylgruppe, die mit einer kationischen Gruppe substituiert und/oder durch eine kationische Gruppe unterbrochen ist,
R₃, R₄ und R₅, die gleich oder verschieden sind, bedeuten:
- ein Wasserstoffatom,
- eine C₁₋₄-Alkylgruppe,
- R₄ und R₅ bilden gemeinsam einen gesättigten oder ungesättigten, 5- oder 8-gliedrigen Ring,
X bedeutet ein Anion oder eine Gruppe von Anionen, die die Elektronegativität des Derivats der Formel (I) sicherstellen können,
mit der Maßgabe, dass mindestens eine der Gruppen Z₁, R₁ eine kationische Gruppe bedeutet.

2. Zusammensetzung nach Anspruch 1, bei der R₆ mit R₁ einen kationischen Heterocyclus oder einen mit einer kationischen Gruppe substituierten Heterocyclus bildet.

3. Zusammensetzung nach Anspruch 2, bei der R₆ mit R₁ Imidazole, die mit einer quartären Ammoniumgruppe substituiert sind, oder Imidazoliumverbindungen, Piperazine, die mit einer quartären Ammoniumgruppe substituiert sind, oder Piperaziniumverbindungen, Pyrrolidine, die mit einer quartären Ammoniumgruppe substituiert sind, oder Pyrrolidiniumverbindungen, Diazepane, die mit einer quartären Ammoniumgruppe substituiert sind, oder Diazepaniumverbindungen bildet.

4. Zusammensetzung nach Anspruch 1, bei der Z₁ -NH- oder NR₆ bedeutet, wobei R₆ mit R₁ einen kationischen Heterocyclus oder einen mit einer kationischen Gruppe substituierten Heterocyclus bildet.

5. Zusammensetzung nach einem der Ansprüche 1 bis 4, bei der die Verbindung der Formel (I) ausgewählt ist unter: 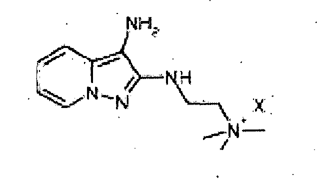
[2-(3-Amino-pyrazolo [1,5-a]pyridin-2-ylamino)-ethyl]-trimethylammonium 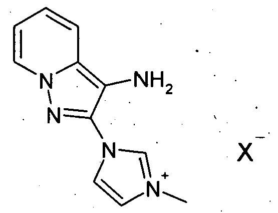
3-(3-Amino-pyrazolo[1,5-a]pyridin-2-yl)-1-methyl-3H-imidazol-1-ium 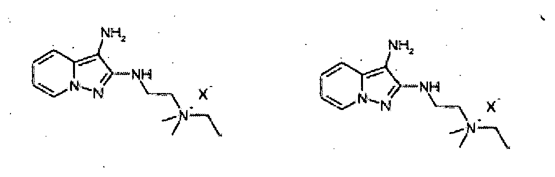
[2-(3-Amino-pyrazolo[1,5-a]pyridin-2-ylamino)-ethyl]-ethyl-dimethyl-ammonium 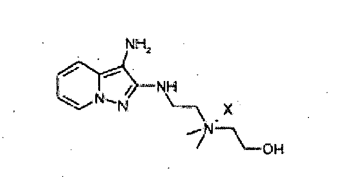
[2-(3-Amino-pyrazolo[1,5-a]pyridin-2-ylamino)-ethyl]-(2-hydroxyethyl)-dimethyl-ammonium 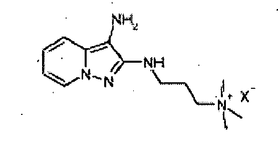
[3-(3-Amino-pyrazolo [1,5-a]pyridin-2-ylamino)-propyl]-trimethylammonium 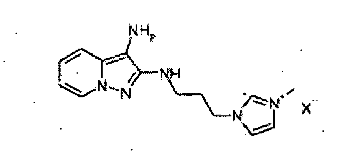
3-[3-(3-Amino-pyrazolo[1,5-a]pyridin-2-ylamino)-propyl]-1-methyl-3H-imidazol-1-ium 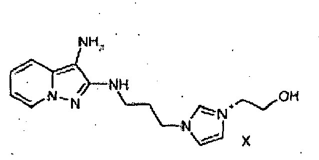
3-[3-(3-Amino-pyrazolo[1,5-a]pyridin-2-ylamino)-propyl]-1-(2-hydroxy-ethyl)-3H-imidazol-1-ium 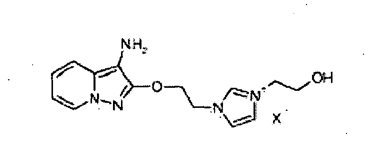
3-[2-(3-Amino-pyrazolo[1,5-a]pyridin-2-yloxy)-ethyl]-1-(2-hydroxy-ethyl)-3H-imidazol-1-ium 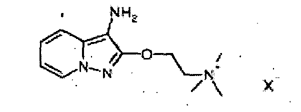
2-[(3-Aminopyrazolo[1,5-a]pyridin-2-yl)oxy]-N,N,N-trimethylethanaminium 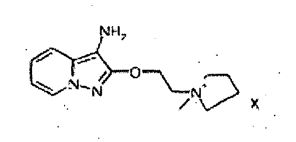
1-{2-[(3-Aminopyrazolo[1,5-a]pyridin-2-yl)oxy]ethyl}-1-methylpyrrolidinium 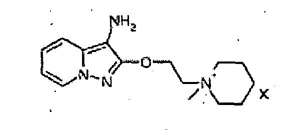
1-{2-[(3-Aminopyrazolo[1,5-a]pyridin-2-yl)oxy]ethyl}-1-methylpiperidinium 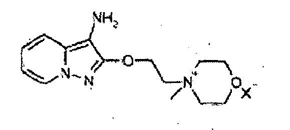
4-{2-[(3-Aminopyrazolo[1,5-a]pyridin-2-yl)oxy]ethyl}-4-methylmorpholin-4-ium 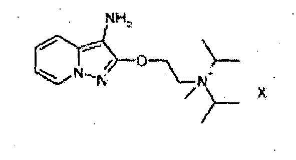
N-{2-[(3-Aminopyrazolo[1,5-a]pyridin-2-yl)oxy]ethyl}-N-isopropyl-N-methylpropan-2-aminium 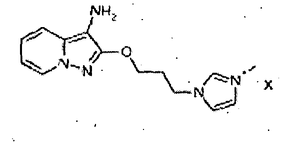
3-[3-(3-Amino-pyrazolo[1,5-a]pyridin-2-yloxy)-propyl]-1-methyl-3H-imidazol-1-ium 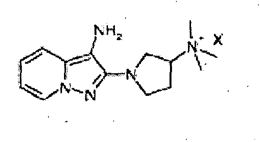
[1-(3-Amino-pyrazolo[1,5-a]pyridin-2-yl)-pyrrolidin-3-yl]-trimethyl-ammonium 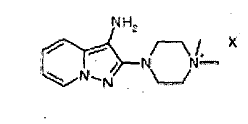
4-(3-Amino-pyrazolo[1,5-a]pyridin-2-yl)-1,1-dimethyl-piperazin-1-ium 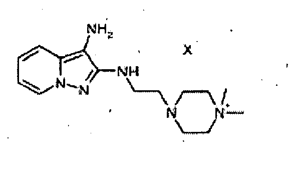
4-[2-(3-Amino-pyrazolo[1,5-a]pyridin-2-ylamino)-ethyl]-1,1-dimethyl-piperazin-1-ium 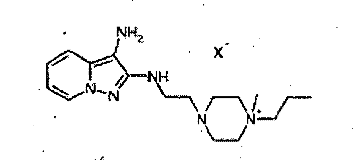
4-[2-(3-Amino-pyrazolo[1,5-a]pyridin-2-ylamino)-ethyl]-1-methyl-1-propyl-piperazin-1-ium 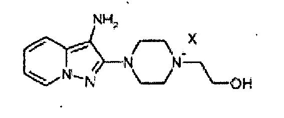
4-(3-Amino-pyrazolo[1,5-a]pyridin-2-yl)-1-(2-hydroxy-ethyl)-piperazin-1-ium 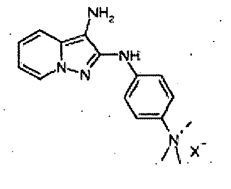
[4-(3-Amino-pyrazolo[1,5-a]pyridin-2-ylamino)-phenyl]-trimethylammonium 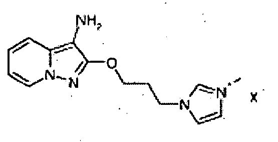
3-[3-(3-Amino-pyrazolo[1,5-a]pyridin-2-yloxy)-propyl]-1-methyl-3H-imidazol-1-ium 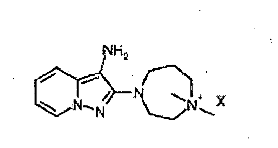
4-(3-Amino-pyrazolo[1,5-a]pyridin-2-yl)-1,1-dimethyl-[1,4]diazepan-1-ium 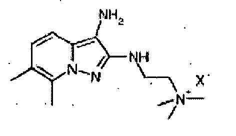
[2-(3-Amino-6,7-dimethyl-pyrazolo[1,5-a]pyridin-2-ylamino)-ethyl]-trimethyl-ammonium 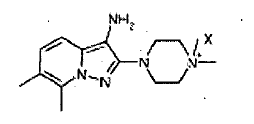
4-(3-Amino-6,7-dimethyl-pyrazolo[1,5-a]pyridin-2-yl)-1,1-dimethyl-piperazin-1-ium 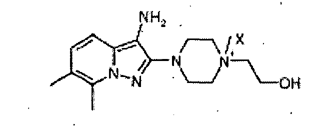
4-(3-Amino-6,7-dimethyl-pyrazolo[1,5-a]pyridin-2-yl)-1-(2-hydroxy-ethyl)-1-methyl-piperazin-1-ium 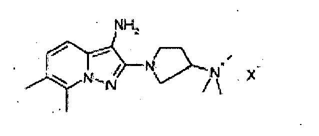
[1-(3-Amino-6,7-dimethyl-pyrazolo[1,5-a]pyridin-2-yl)-pyrrolidin-3-yl]-trimethyl-ammonium 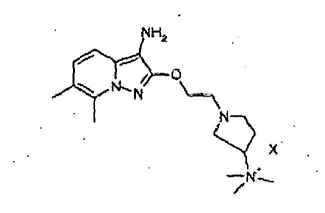
{1-[2-(3-Amino-6,7-dimethyl-pyrazolo[1,5-a]pyridin-2-yloxy)-ethyl]-pyrrolidin-3-yl}-trimethyl-ammonium 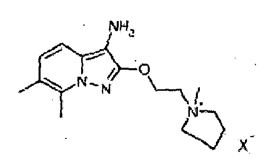
1-{2-[(3-Amino-6,7-dimethylpyrazolo[1,5-a]pyridin-2-yl)oxy]ethyl)-1-methylpyrrolidinium 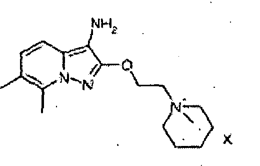
1-{2-[(3-Amino-6,7-dimethylpyrazolo[1,5-a]pyridin-2-yl)oxy]ethyl}-1-methylpiperidinium 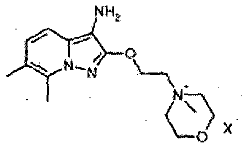
4-{2-[(3-Amino-6,7-dimethylpyrazolo[1,5-a]pyridin-2-yl)oxy]ethyl}-4-methylmorpholin-4-ium 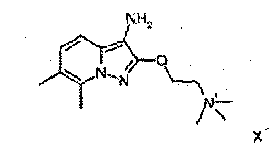
2-[(3-Amino-6,7-dimethylpyrazolo[1,5-a]pyridin-2-yl]oxy]-N,N,N-trimethylethanaminium 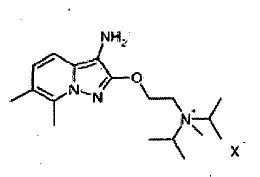
N-{2-[(3-Amino-6,7-dimethylpyrazolo[1,5-a]pyridin-2-yl)oxy]ethyl}-N-isopropyl-N-methylpropan-2-aminium 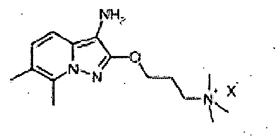
[3-(3-Amino-6,7-dimethyl-pyrazolo[1,5-a]pyridin-2-yloxy)-propyl]-trimethyl-ammonium 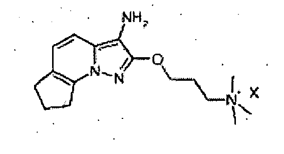
2-[(3-Amino-7,8-dihydro-6H-cyclopenta[e]pyrazolo[1,5-a]pyridin-2-yl)oxy]-N,N,N-trimethylethanaminium 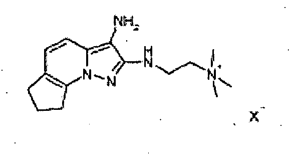
2-[(3-Amino-7,8-dihydro-6H-cyclopenta[e]pyrazolo[1,5-a]pyridin-2-yl)amino]-N,N,N-trimethylethanaminium 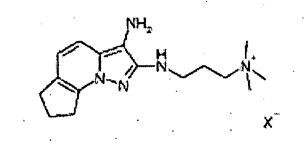
3-[(3-Amino-7,8-dihydro-6H-cyclopenta[e]pyrazolo[1,5-a]pyridin-2-yl)amino]-N,N,N-trimethylpropan-1-aminium 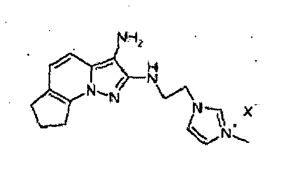
1-{2-[(3-Amino-7,8-dihydro-6H-cyclopenta[e]pyrazolo1,5-a]-pyridin-2-yl)amino]ethyl}-3-methyl-1H-imidazol-3-ium 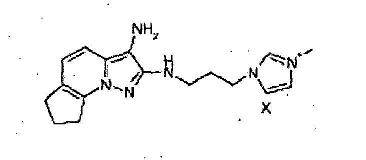
1-{3-[(3-Amino-7,8-dihydro-6H-cyclopenta[e]pyrazolo[1,5-a]-pyridin-2-yl)amino]propyl}-3-methyl-1 H-imidazol-3-ium 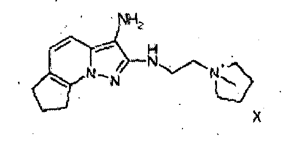
1-{2-[(3-Amino-7,8-dihydro-6H-cyclopenta[e]pyrazolo[1,5-a]pyridin-2-yl)amino]ethyl}-1-methylpyrrolidinium 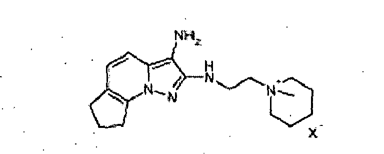
1-{2-[(3-Amino-7,8-dihydro-6H-cyclopenta[e]pyrazolo[1,5-a]-pyridin-2-yl)amino]ethyl}-1-methylpiperidinium 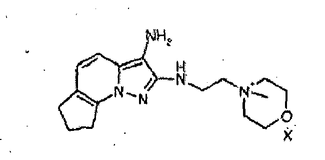
4-{2-[(3-Amino-7,8-dihydro-6H-cyclopenta[e]pyrazolo[1,5-a]-pyridin-2-yl)amino]ethyl}-4-methylmorpholin-4-ium 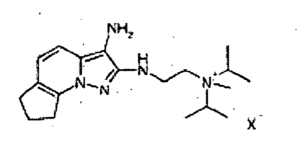
N-{2-[(3-Amino-7,8-dihydro-6H-cyclopenta[e]pyrazolo[1,5-a]-pyridin-2-yl)amino]ethyl}-N-isopropyl-N-methylpropan-2-aminium 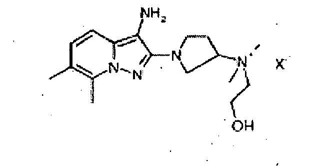
[1-(3-Amino-6,7-dimethyl-pyrazolo[1,5-a]pyridin-2-yl)-pyrrolidin-3-yl]-(2-hydroxy-ethyl)-dimethyl-ammonium 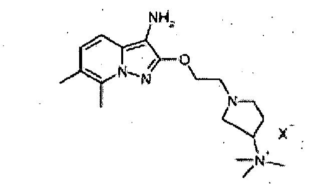
{1-[2-(3-Amino-6,7-dimethyl-pyrazolo[1,5-a]pyridin-2-yloxy)-ethyl]-pyrrolidin-3-yl}-trimethyl-ammonium 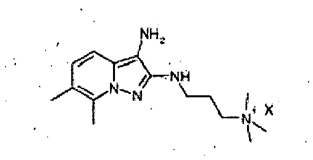
[3-(3-Amino-6,7-dimethyl-pyrazolo[1,5-a]pyridin-2-ylamino)-propyl]-trimethyl-ammonium 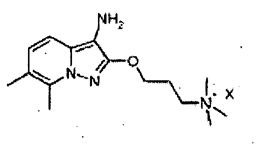
[3-(3-Amino-6,7-dimethyl-pyrazolo[1,5-a]pyridin-2-yloxy)-propyl]-trimethyl-ammonium

6. Zusammensetzung nach Anspruch 5, wobei das Derivat der Formel (I) unter den Derivaten ausgewählt ist, bei denen Z₁ -NHist und R₁ eine kationische Gruppe bedeutet oder Z₁ -O- ist und R₁ eine kationische Gruppe bedeutet.

7. Zusammensetzung nach einem der vorhergehenden Ansprüche, bei der der Mengenanteil jeder Oxidationsbase der Formel (I) im Bereich von etwa 0,001 bis 10 Gew.-%, bezogen auf das Gesamtgewicht der Farbmittelzusammensetzung, liegt.

8. Zusammensetzung nach einem der vorhergehenden Ansprüche, die ferner einen Kuppler enthält, der unter den meta-Phenylendiaminen, meta-Aminophenolen, meta-Dihydroxybenzolen, Naphthalinkupplern, heterocyclischen Kupplern und ihren Additionssalzen sowie deren Gemischen ausgewählt ist.

9. Zusammensetzung nach Anspruch 8, wobei der Mengenanteil jedes Kupplers im Bereich von 0,001 bis 10 Gew.-%, bezogen auf das Gesamtgewicht der Farbmittelzusammensetzung, liegt.

10. Verfahren zum Färben von Keratinfasern, **dadurch gekennzeichnet, dass** eine Zusammensetzung, wie sie in einem der Ansprüche 1 bis 9 definiert ist, in Gegenwart eines Oxidationsmittels während einer Zeitspanne, die zur Bildung der gewünschten Färbung ausreichend ist, auf die Keratinfasern aufgebracht wird.

11. Verfahren nach Anspruch 10, wobei das Oxidationsmittel unter Wasserstoffperoxid, Harnstoffperoxid, Alkalimetallbromaten, Salzen von Persäuren, Persäuren und Oxidasen ausgewählt ist.

12. Vorrichtung mit mehreren Abteilungen, wobei eine Abteilung eine Farbmittelzusammensetzung, wie sie in einem der Ansprüche 1 bis 9 definiert ist, und eine zweite Abteilung ein Oxidationsmittel enthält.

13. Derivate der Formel (I) nach einem der Ansprüche 1 bis 7. 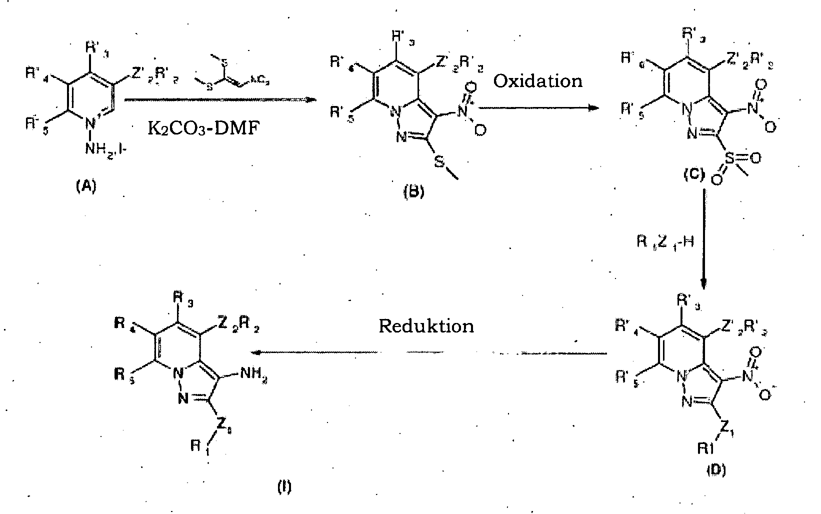 wobei Z1, R1, Z'2, R'2, R'3, R'4 und R'5 die für Z2, R2, R3, R4 und R5 angegebenen Bedeutungen aufweisen oder deren Vorläufer sind.

14. Verfahren zur Herstellung einer Verbindung der Formel (I) nach einem der Ansprüche 1 bis 6, das die folgenden Schritte umfasst:

15. Zwischenprodukte der Formeln (A) und (B), wie sie in Anspruch 14 definiert sind, wobei die Verbindungen 1-Amino-3-methylpyridiniumiodid und 4-Methyl-2-(methylsulfanyl)-3-nitropyrazolo[1,5-a]-pyridin ausgenommen sind.

16. Zwischenprodukte der Formel (C) oder (D), wie sie in Anspruch 14 definiert sind.
